(19) **Europäisches Patentamt / European Patent Office / Office européen des brevets**

(11) **EP 2 288 724 B1**

(12) # EUROPEAN PATENT SPECIFICATION

(45) Date of publication and mention of the grant of the patent:
**12.11.2014 Bulletin 2014/46**

(21) Application number: **09747015.7**

(22) Date of filing: **18.05.2009**

(51) Int Cl.:
*C12Q 1/68* (2006.01)        *G06F 19/18* (2011.01)

(86) International application number:
**PCT/US2009/003104**

(87) International publication number:
**WO 2009/139929 (19.11.2009 Gazette 2009/47)**

---

(54) **METHODS AND SYSTEMS FOR UNIVERSAL CARRIER SCREENING**

VERFAHREN UND SYSTEME FÜR UNIVERSELLES TRÄGERSCREENING

PROCÉDÉS ET SYSTÈMES POUR UN DÉPISTAGE UNIVERSEL DE PORTEUR

---

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **16.05.2008 US 53926 P**

(43) Date of publication of application:
**02.03.2011 Bulletin 2011/09**

(73) Proprietor: **Counsyl, Inc.**
**Redwood City, CA 94065 (US)**

(72) Inventors:
• **SRINIVASAN, Balaji, S.**
  **Palo Alto, CA 94304 (US)**
• **SRINIVASAN, Balaji, K.**
  **San Francisco, CA 94103 (US)**
• **EVANS, Eric**
  **Redwood City, CA 94065 (US)**
• **SRINIVASAN, Ramji**
  **San Francisco, CA 94103 (US)**
• **BALAKRISHNAN, Kumaranayagam**
  **Staten Island, NY 10301 (US)**

(74) Representative: **Kremer, Simon Mark et al**
  **Mewburn Ellis LLP**
  **33 Gutter Lane**
  **London**
  **EC2V 8AS (GB)**

(56) References cited:
  **WO-A2-2007/053752      WO-A2-2007/089145**

US-A1- 2003 040 002      US-A1- 2003 208 454
US-A1- 2004 210 400      US-A1- 2004 229 231
US-A1- 2004 265 849      US-A1- 2005 026 117
US-A1- 2006 281 120      US-A1- 2007 037 182
US-A1- 2007 050 354      US-A1- 2007 111 247
US-A1- 2008 081 331

• BICK D ET AL: "Screening semen donors for hereditary diseases. The Fairfax cryobank experience.", THE JOURNAL OF REPRODUCTIVE MEDICINE MAY 1998 LNKD- PUBMED:9610465, vol. 43, no. 5, May 1998 (1998-05), pages 423-428, XP009147397, ISSN: 0024-7758

• PICCI L ET AL: "Cystic fibrosis as a model for the carrier screening of hereditary genetic diseases", MINERVA BIOTECNOLOGICA 200006 IT, vol. 12, no. 2, June 2000 (2000-06), pages 83-89, XP009147401, ISSN: 1120-4826

• MITCHELL J J ET AL: "Twenty-year outcome analysis of genetic screening programs for Tay-Sachs and beta-thalassemia disease carriers in high schools.", AMERICAN JOURNAL OF HUMAN GENETICS OCT 1996 LNKD- PUBMED: 8808593, vol. 59, no. 4, October 1996 (1996-10), pages 793-798, XP007918319, ISSN: 0002-9297

• TSONGALIS G J ET AL: "Clinical genotyping: The need for interrogation of single nucleotide polymorphisms and mutations in the clinical laboratory", CLINICA CHIMICA ACTA, ELSEVIER BV, AMSTERDAM, NL, vol. 363, no. 1-2, 1 January 2006 (2006-01-01), pages 127-137, XP025058622, ISSN: 0009-8981, DOI: DOI:10.1016/J.CCCN. 2005.05.043 [retrieved on 2006-01-01]

---

**Description**

**BACKGROUND OF THE INVENTION**

Genetic testing of prospective parents can be used to predict the chances that offspring of a couple will have particular genetic diseases. Persons for whom such testing is attractive include those for whom genetic diseases run in the family or those from ethnic groups that have high incidence of genetic diseases. The results of such testing can provide a couple with information they can use to make decisions about becoming parents. For example, a couple may decide to make preparations for special care that might be needed to raise a child with special needs, or the couple may explore options for assisted reproduction. WO2007/053752 discloses computer-based methods and systems for facilitating assessment of results of genetic screening tests.

Many genetic diseases are rare in a population, for example, with frequencies of less than 1 in 1000. Yet, if a genetic disease is caused by a single recessive allele of a gene and both prospective parents are carriers of the recessive allele, then the probability that a child of the couple will have the disease is likely 25%.

The incidence of alleles for a certain genetic conditions is different across different populations. For example, in persons with Ashkenazi Jewish ancestry, the carrier frequency for Tay-Sachs disease is 1:30; for Canavan disease it is 1:40; for Niemann-Pick disease type A it is 1:90; for Fanconi anemia type C it is 1:89; for Bloom syndrome it is 1:100; for Gaucher disease type 1 it is 1:12. In persons of Southeast Asian ancestry, the carrier frequency of alpha-thalassemia is as high as 1:20. In Caucasians, the carrier frequency of cystic fibrosis is 1:25.

While such diseases may be individually rare, there are a sufficient number of them so the probability that any individual is a carrier for at least one of is significantly greater. For example, it has been estimated that 70% of the Ashkenazi Jewish population has at least one disease causing allele. Estimates of genetic load indicate that every human carries approximately 8 to 30 deleterious recessive alleles.

Carrier screening can decrease the incidence of genetic diseases in a population. For example, as a result of carrier screening, the incidence of Tay Sachs disease among the Ashkenazi Jewish population has decreased in recent years. Another example of a genetic Mendelian disease is cystic fibrosis (CF). Cystic fibrosis is often fatal, debilitating, incurable, and costly to treat. CF can be characterized by autosomal recessive inheritance and carried by asymptomatic individuals caused by hundreds of different mutations, each of which varies in frequency across ethnic groups. The American College of Medical Genetics (ACMG) recommends carrier screening by genetic testing for all prospective parents for a number of Mendelian diseases. Since 1998, panethnic population-wide screening for cystic fibrosis carrier status has been recommended by ACMG. The list of diseases that ACMG recommends testing is continually expanding as a function of new discoveries related to genetic diseases. Carrier screening has been adopted because the health benefits outweigh the financial costs of testing by a definitive margin.

Presently, screening of prospective parents for carrier status is selective. It is indicated for couples belonging to populations at increased risk for particular conditions. There is a need in the art for universal testing for a wide variety of genetic conditions for individuals of any ancestry.

SUMMARY OF THE INVENTION

The scope of the present invention is defined by the appended claims 1 to 19. In an aspect, a method is disclosed herein that comprises: testing a subset of biological relatives of a child or potential child to determine a presence of a plurality of causal genetic variants corresponding to at least one rare genetic disease and a presence of at least one ancestry informative marker (AIM); and predicting a probability of a phenotype of the child or potential child from the subset of biological relatives with respect to the at least one rare genetic disease based at least in part on the presence of the plurality of causal genetic variants and the presence of the at least one AIM. In some instances, predicting comprises performing a fully probabilistic analysis on data collected on said casual genetic variants. In some instances, predicting is further based on phenotypic information about at least one member of the subset of biological relatives. The method can further comprise providing genetic counseling services to at least one member of the subset of biological relatives. The method can further comprise delivering the probability of the phenotype of the child to a physician referral service.

In an aspect, a computer readable medium comprises: logic configured to predict a probability of a phenotype of a child or potential child from a subset of biological relatives with respect to at least one rare genetic disease based at least in part on the results of a test of the subset of biological relatives for the presence of a plurality of causal genetic variants and at least one ancestry informative marker (AIM). In some instances, the logic performs a fully probabilistic analysis. In some instances, the computer readable medium provides an output in the form of a report detailing the presence of the at least on AIM and the presence of any of the plurality of causal genetic variants in any member of the subset of biological relatives.

In an aspect, a computer readable medium comprises: logic configured to perform a fully probabilistic analysis on data corresponding to a plurality of causal genetic variants from a male and a female to predict a probability of a phenotype of a child, wherein the male and the female are potential parents of the child. In some instances, the causal genetic variants from each of the male and female comprise one or more ancestry informative markers (AIMs), one or more causal genetic variants corresponding to a rare genetic disease, one or more causal genetic variants corresponding to a personality trait, or both. In some instances, fully probabilistic analysis incorporates a plurality of sources of statistical uncertainty in the probability. A computer readable medium herein can further comprise logic for receiving input from a phenotype battery and assigning a weighting function to the plurality of causal genetic variants based on said input. In some instances, the input from the phenotype battery comprises: height, weight, and family disease history. In some instances, the computer readable medium provides an output in the form of a report detailing a probability distribution over child risks or phenotypes.

In an aspect, a system is described herein for predicting a child phenotype that comprises: a nucleic acid detection device configured to detect a plurality of causal genetic variants corresponding to at least one rare genetic disease and at least one ancestry informative marker (AIM), wherein the device is in contact with a sample from a biological relative of a child or potential child; a reader configured to read data from the devices; and computer readable instructions, wherein the instructions when executed utilize the data from the reader corresponding to the plurality of causal genetic variants and data from the at least one AIM to predict a probability of a phenotype of the child with respect to the at least one rare genetic disease. In some instances, the biological relative is a prospective mother or prospective father of the child or potential child. In some instances, the nucleic acid detection device comprises a plurality of nucleic acid probes that selectively bind to the plurality of causal genetic variants and the at least one AIM.

In an aspect, a system for predicting a child phenotype comprises: a nucleic acid detection device configured to detect a plurality of causal genetic variants corresponding to more than 85 rare genetic diseases, wherein the device is in contact with a sample from a biological relative of the child or potential child; a reader configured to read data from the devices; and computer readable instructions, wherein the instructions when executed utilize the data from the reader corresponding to the plurality of causal genetic variants to predict a probability of a phenotype of the child or potential child with respect to the more than 85 rare genetic diseases. In some instances, the biological relative is a prospective mother or prospective father of the child or potential child. In some instances, the nucleic acid detection device comprises a plurality of nucleic acid probes that selectively bind to the plurality of causal genetic variants corresponding to more than 85 rare genetic diseases. In some instances, the nucleic acid detection device further comprises a plurality of nucleic acid probes that selectively bind to at least one ancestry informative marker (AIM). In some instances, the computer readable instructions when executed utilize the data from the reader corresponding to the at least one AIM to predict the probability of a phenotype of the child.

In an aspect, a system for indicating if a subject is a carrier of a rare genetic disease comprises: a reader configured to read data from a nucleic acid detection device configured to detect a plurality of causal genetic variants corresponding to at least one rare genetic disease and at least one ancestry informative marker (AIM); and computer readable instructions, wherein the instructions when executed utilize the data from the reader corresponding to the plurality of causal genetic variants and the at least one ancestry informative marker to predict a plurality of probabilities of the subject being a carrier for each of the plurality of causal genetic variants.

In an aspect, a method comprises: receiving a sample from a user; testing the sample with a nucleic acid detection device configured to test for a plurality of causal genetic variants of rare genetic diseases and at least one ancestry informative marker (AIM); calculating a plurality of probabilities for the possible user genotypes at each location of the plurality of causal genetic variants based on results from the testing step relating to the plurality of causal genetic variants and the at least one AIM; and delivering to the user the plurality of probabilities corresponding to the user being a carrier. The method can further comprise: receiving a sample from a second user; testing the sample from the second user with a device configured to test for a plurality of causal genetic variants of rare genetic diseases and at least one ancestry informative marker (AIM); calculating a probability of a child phenotype corresponding to the rare genetic diseases based on results from testing the user and the second user; and delivering the probability of the child phenotype to at least one of the user and the second user. The method can further comprise providing genetic counseling service to at least one of the user and the second user. The method can be carried out as part of a child phenotype prediction service. The method can further comprise obtaining phenotypic information from the user; and using the phenotypic information from the user in the calculating steps. The method can further comprise obtaining family history from the user; and using the family history from the user in the calculating steps.

In an aspect, a nucleic acid detection device is configured to test a sample for a plurality of causal genetic variants corresponding to at least one rare genetic disease and one or more ancestry informative markers (AIMs). In some instances, the device comprises a plurality of nucleic acid probes that selectively bind to the plurality of causal genetic variants and the one or more AIMs. In some instances, the device comprises a bead array that selectively binds to the plurality of causal genetic variants and the one or more AIMs.

In another aspect, a nucleic acid detection device is configured to test as sample for a plurality of causal genetic variants corresponding more than 85 rare genetic diseases. The device can be further configured to test a sample for at least one ancestry informative marker (AIM). In some instances, the device comprises a plurality of nucleic acid probes that selectively bind to a plurality of causal genetic variants corresponding to more than 85 rare genetic diseases. The device can further comprise a plurality of nucleic acid probes that selectively bind to at least one ancestry informative marker (AIM). The device can comprise a bead array that selectively binds to a plurality of causal genetic variants corresponding to more than 85 rare genetic diseases. The device can further comprise a bead array that selectively binds to at least one ancestry informative marker (AIM). In some instances, the device comprises a resequencing assay to detect the plurality of causal genetic variants corresponding to the more than 85 rare genetic diseases. In some instances, the device further comprises a resequencing assay to detect at least one ancestry informative marker (AIM).

In some instances, the at least one AIM is not a causal genetic variant. In some instances, at least two of the rare genetic diseases occur at frequencies that differ by at least 10-fold in at least two distinct populations, wherein the at least two distinct populations are differentiated by the at least one AIM.

In an aspect, a method comprises: marketing a genetic testing service comprising predicting a probability of a child phenotype from a subset of biological relatives of the child or potential child, wherein the prediction is based at least in part on the presence of a plurality of causal genetic variants in each of the subset of biological relatives and based at least in part on the inferred ancestries of each of the subset of biological relatives; and delivering a probability of the child phenotype for a fee. The marketing can be conducted in connection with a dating or marriage service. The method can further comprise referring at least one member of the subset of biological relatives to a physician. In some instances, the inferred ancestries are inferred by a test for at least one ancestry informative marker (AIM).

In an aspect, a set of nucleic acid pools is disclosed for validating a nucleic acid sequence detection device comprising a set of causal genetic variant probes, wherein each nucleic acid pool comprises a plurality of nucleic acid segments that selectively bind a different subset of the set of causal genetic variant probes. In some instances, a first pool of the set comprises a first nucleic acid segment that interferes during detection with a second nucleic acid segment of a second pool of the set, and wherein the first pool does not comprise the second nucleic acid segment and the second pool does not comprise the first nucleic acid segment. In some instances, the nucleic acid segments of each pool are single stranded nucleic acid molecules. In some instances, the nucleic acid segments comprise one or more plasmids.

In an aspect, a method of validating a lot of manufactured nucleic acid sequence detection devices comprises: contacting each of a plurality of nucleic acid sequence detection devices from the lot with a different nucleic acid pool, wherein each nucleic acid pool comprises a plurality of nucleic acid segments that selectively bind a plurality of causal genetic variant probes on said detection devices, and wherein each nucleic acid pool binds a different set of the plurality of causal genetic variant probes; and detecting presence or absence of the plurality of causal genetic variant probes on the plurality of nucleic acid detection devices, wherein the lot of manufactured devices is validated if all of the plurality of causal genetic variant probes are present on the plurality of nucleic acid detection devices.

In some instances, the method further comprises delivering the lot of manufactured devices when the devices are validated. In some instances, the lot of manufactured devices is rejected if not all of the plurality of causal genetic variant probes are present. In some instances, the lot of manufactured devices is modified and the method is repeated if not all of the plurality of causal genetic variant probes are present.

## BRIEF DESCRIPTION OF THE DRAWINGS

Many features of the invention are set forth with particularity in the appended claims. A better understanding of the features and advantages herein will be obtained by reference to the following detailed description that sets forth illustrative embodiments, in which many principles are utilized, and the accompanying drawings of which:

Figure 1 provides a table of causal genetic variants.
Figure 2 provides a list of exemplary populations.
Figure 3 provides a number of AIMs that distinguish different populations. The entries refer to items in the dbSNP database, a database of genetic variants maintained by the US government: http://www.ncbi.nlm.nih.gov/projects/SNP/. Curated records in dbSNP contain information that describes the sequence and location of genetic variants, and where available the frequency of alleles of those variants in different populations. rs numbers (for example, rs332, rs25, etc.) are the ID numbers used to index the portion of the dbSNP database.
Figure 4 illustrates a population 1 as a group which has a hypothetical A/G SNP with the given flanking sequence.
Figure 5 illustrates population 2 has exactly the same variant with the same flanking sequence as Figure 4, but different proportions of each genotype (0.16, 0.48, 0.36 rather than 0.25, 0.50, 0.25).
Figure 6 illustrates population 3 has a different flanking sequence than Figures 4 and 5 with an extra G.
Figure 7 considers populations by gender, wherein Figures 4-6 consider populations in terms of geographic ancestry.
Figure 8 shows an intensity scatterplot for the beta thalassemia deletion allele similar to the scatterplots in Figures 4-7.
Figure 9 shows that calling of points near the boundary is aided by the use of ancestry information.
Figure 10 shows that a raw intensity measurement at a causal locus is not the only value that matters.

Figure 11 illustrates a source code example that is useful in calling.

Figure 12 shows a straightforward modification to the expression for the posterior probability of whether an individual is in a given cluster.

Figure 13 shows a measured genotype may have measurement error as represented by a false positive and false negative rate.

Figure 14 shows the inferred genotype is unphased.

Figure 15 shows how to generate a distribution over possible recombinants and hence possible gametes from the possible haplotypes.

Figure 16 shows how to repeat this process for both mother and father to obtain a probability distribution over gametic unions, corresponding to phased child genotypes (aka haplotypes).

Figure 17 shows several different kinds of genotype-to-phenotype maps that can be used with the distribution over child haplotypes to produce a distribution over possible child phenotypes.

Figure 18 depicts equations for obtaining the distribution over estimated child phenotypes given parental genotypes.

Figure 19 demonstrates examples of nucleic acid segments and pools of nucleic acids.

Figures 20-23 demonstrate exemplary processes of delivering a probability that a user is a carrier of rare genetic disease.

Figure 24 illustrates exemplary the input and output steps for report generation for two hypothetical parents: Mama Hen and Papa Hen.

## DETAILED DESCRIPTION OF THE INVENTION

Described herein are methods, computer readable instructions, software, systems, and devices that are utilized for detecting genotypes of individuals. Herein, the genotypes relate to detecting specific causal genetic variants that cause or can cause rare genetic diseases. In many instances, the rare genetic diseases are Mendelian diseases, wherein an individual is a carrier of a trait (recessive or dominant) corresponding to primarily related to single gene. The methods, computer readable instructions, software, systems, and devices can be used for family genomics, such as generating probability distributions for many family members in a family including without limitation: grandparents, parents, children, aunts, and uncles. The family members may be alive, dead, embryonic, or not yet conceived. The genotype and/or phenotype of any family member may be utilized to gain information about other family members. The methods, computer readable instructions, software, systems, and devices can be utilized as a screen for predicting the probability of the phenotype of a child or potential child from two individuals. In many instances, the child phenotype is predicted before conception based upon the genotypes (and sometimes phenotypes) of the potential father and potential mother. Child phenotype prediction can be useful in a variety of circumstances as described herein and provides an opportunity for an individual to make a decision or take action based upon his personal genotype.

## I. METHODS OF TESTING AND PREDICTION

In an aspect, a method comprises: testing each member of a set of parents or potential parents to determine presence of a plurality of causal genetic variants corresponding to at least one rare genetic disease and the presence of at least one ancestry informative marker (AIM); and predicting a probability of a phenotype of a child from the set of parents or potential parents with respect to the at least one rare genetic disease based at least in part on results of the presence of the plurality of causal genetic variants and the at least one AIM. In some instances the predicting step is further based on phenotypic information about at least one member of the set of parents or potential parents, or genotypic and/or phenotypic information on other biological relatives of the child. In some instances the method further comprises providing genetic counseling services. In some instances, a probability about a person's genotype, phenotype, or potential phenotype can be delivered to a physician referral service. In some instances the method further comprises delivering the probability of the phenotype of the child phenotype to a physician referral service.

The prediction of a probability of a phenotype herein can be a probability distribution over a variety of risks for traits. The probability distribution can be categorical or continuous for any probability prediction herein. As an example of a probability distribution over child risks, the prediction could be $P(D=1) = 0.80$ and $P(D=0) = 0.20$, representing an 80% probability that a child will be affected ($D=1$) by a Mendelian disease. As an example of a probability distribution over child phenotypes, the prediction could be a normal distribution with specified mean and variance over the possible height of the child as an adult.

A genetic disease can be any disease that is influenced by a known causal genetic variant. In some instances, a rare genetic disease is a disease that is present at a rate 1 per 100 in the human population. In other instances, a rare genetic disease is a disease that is present at a rate 1 per 1000 in the human population. In yet other instances, a rare genetic disease is a disease that is present at a rate 1 per 10,000, 1 per 100,000, 1 per 1,000,000, or 1 per 10,000,000 in the human population. In some instances, the.rare genetic disease may be present at a rate much higher than 1 per 100 in a specific ethnic or ancestral human population, for example, 1 per 50, 1 per 20, 1 per 10, or 1 per 5 in a specific population.

## A. Rare Genetic Disease

Methods, systems, software, and devices herein can test or be configured to test for phenotypic traits (also referred to simply as traits), i.e., a distinct form of a characteristic of an organism. For example, eye color is a physical characteristic; brown, green and blue are phenotypic traits. Some physical characteristics relating to health have, as associated traits, abnormal and normal, such as Huntington's disease (abnormal) and non-Huntington's disease (normal). Traits may be morphological, developmental, biochemical, physiological, or behavioral. The collection of a plurality of phenotypic traits exhibited by an individual is usually referred to as the individual's phenotype.

Mendelian traits are traits that are inherited by way of a single gene or influenced primarily by a single gene. Mendelian diseases are diseases inherited as a Mendelian trait. Typical Mendelian traits can be autosomal dominant, autosomal recessive or sex-linked (X-linked or Y-linked). Mendelian traits can also be atypical, including traits with atypical inheritance patterns (for example, cytoplasmic inheritance or incomplete penetrance) and including traits that arise as a result of spontaneous but predictable mutations (for example, repeat polymorphism expansions or mutational hot spots). Mendelian traits include both typical Mendelian traits and atypical Mendelian traits. Typically, the percentage of variance in a Mendelian trait that is explained by genotype is very high, for example, at least 99%. However, the percentage of variance in phenotype in an atypical Mendelian trait that is explained by genotype can be lower.

In certain instances, two different genes cause traits that appear similar or identical. In this case it is typical to define sub-types of the trait such that a single gene is associated with a trait single sub-type. For example, the disease Mucopolysaccharidosis is typically further described as being of a particular type, such as Mucopolysaccharidosis Type I or Mucopolysaccharidosis Type VII.

Non-Mendelian traits, also called complex traits, are inherited by way of more than one gene. Typically, for non-Mendelian traits the percentage of variance in phenotype explained by explicit genotypic markers is low, often less than 50%. A non-Mendelian trait differs from an atypical Mendelian trait in that differences between individuals in a complex trait are due to differences in more than one gene whereas differences between individuals for an atypical Mendelian trait are due primarily to differences in one gene. Not all non-Mendelian traits have genotypically-explained variance of less than 99% (Friedman, Naomi P.; Miyake, Akira; Young, Susan E.; DeFries, John C.; Corley, Robin P.; Hewitt, John K. Individual differences in executive functions are almost entirely genetic in origin.. Journal of Experimental Psychology: General. 2008 May Vol 137(2) 201-225). Examples of non-Mendelian traits are height, weight, and skin color.

An individual's genotype for a Mendelian trait is the identity of the genes (or gene in the case of sex-linked traits) responsible for the trait that the individual carries, for example, homozygous for the gene responsible for the trait, homozygous for a gene responsible for a different trait, or heterozygous.

Rare genetic diseases as described herein are a type of trait. A rare genetic disease for the purposes herein is a disease that is a trait that can be inherited genetically by a child from a set of parents. The disease or trait can then manifest itself in the phenotype of the child.

Rare genetic diseases that can be tested according to this invention include, but are not limited to:21-Hydroxylase Deficiency, ABCC8-Related Hyperinsulinism, ARSACS, Achondroplasia, Achromatopsia, Adenosine Monophosphate Deaminase 1, Agenesis of Corpus Callosum with Neuronopathy, Alkaptonuria, Alpha-1-Antitrypsin Deficiency, Alpha-Mannosidosis, Alpha-Sarcoglycanopathy, Alpha-Thalassemia, Alzheimers, Angiotensin II Receptor, Type 1, Apolipoprotein E Genotyping, Argininosuccinicaciduria, Aspartylglycosaminuria, Ataxia with Vitamin E Deficiency, Ataxia-Telangiectasia, Autoimmune Polyendocrinopathy Syndrome Type 1, BRCA1 Hereditary Breast/Ovarian Cancer, BRCA2 Hereditary Breast/Ovarian Cancer, Bardet-Biedl Syndrome, Best Vitelliform Macular Dystrophy, Beta-Sarcoglycanopathy, Beta-Thalassemia, Biotinidase Deficiency, Blau Syndrome, Bloom Syndrome, CFTR-Related Disorders, CLN3-Related Neuronal Ceroid-Lipofuscinosis, CLN5-Related Neuronal Ceroid-Lipofuscinosis, CLN8-Related Neuronal Ceroid-Lipofuscinosis, Canavan Disease, Carnitine Palmitoyltransferase IA Deficiency, Carnitine Palmitoyltransferase II Deficiency, Cartilage-Hair Hypoplasia, Cerebral Cavernous Malformation, Choroideremia, Cohen Syndrome, Congenital Cataracts, Facial Dysmorphism, and Neuropathy, Congenital Disorder of GlycosylationIa, Congenital Disorder of Glycosylation Ib, Congenital Finnish Nephrosis, Crohn Disease, Cystinosis, DFNA 9 (COCH), Diabetes and Hearing Loss, Early-Onset Primary Dystonia (DYT1), Epidermolysis Bullosa Junctional, Herlitz-Pearson Type, FANCC-Related Fanconi Anemia, FGFR1-Related Craniosynostosis, FGFR2-Related Craniosynostosis, FGFR3-Related Craniosynostosis, Factor V Leiden Thrombophilia, Factor V R2 Mutation Thrombophilia, Factor XI Deficiency, Factor XIII Deficiency, Familial Adenomatous Polyposis, Familial Dysautonomia, Familial Hypercholesterolemia Type B, Familial Mediterranean Fever, Free Sialic Acid Storage Disorders, Frontotemporal Dementia with Parkinsonism-17, Fumarase deficiency, GJB2-Related DFNA 3 Nonsyndromic Hearing Loss and Deafness, GJB2-Related DFNB 1 Nonsyndromic Hearing Loss and Deafness, GNE-Related Myopathies, Galactosemia, Gaucher Disease, Glucose-6-Phosphate Dehydrogenase Deficiency, Glutaricacidemia Type 1, Glycogen Storage Disease Type 1a, Glycogen Storage Disease Type 1b, Glycogen Storage Disease Type II, Glycogen Storage Disease Type III, Glycogen Storage Disease Type V, Gracile Syndrome, HFE-Associated Hereditary. Hemochromatosis, Halder AIMs, Hemoglobin S Beta-Thalassemia, Hereditary Fructose Intolerance, Hereditary Pancreatitis, Hereditary Thymine-Uraciluria, Hexosaminidase A Deficiency, Hidrotic Ectodermal Dysplasia 2, Homocystinuria Caused by Cystathionine Beta-Synthase Deficiency, Hyperkalemic Periodic Paralysis Type 1, Hyperornithinemia-Hyperammonemia-Homocitrullinuria Syndrome, Hyperoxaluria, Primary, Type 1, Hyperoxaluria, Primary, Type 2, Hypochondroplasia, Hypokalemic Periodic Paralysis Type 1, Hypokalemic Periodic Paralysis Type 2, Hypophosphatasia, Infantile Myopathy and Lactic Acidosis (Fatal and Non-Fatal Forms), Isovaleric Acidemias, Krabbe Disease, LGMD2I, Leber Hereditary Optic Neuropathy, Leigh Syndrome, French-Canadian Type, Long Chain 3-Hydroxyacyl-CoA Dehydrogenase Deficiency, MELAS, MERRF, MTHFR Deficiency, MTHFR Thermolabile Variant, MTRNR1-Related Hearing Loss and Deafness, MTTS1-Related Hearing Loss and Deafness, MYH-Associated Polyposis, Maple Syrup Urine Disease Type 1A, Maple Syrup Urine Disease Type 1B, McCune-Albright Syndrome, Medium Chain Acyl-Coenzyme A Dehydrogenase Deficiency, Megalencephalic Leukoencephalopathy with Subcortical Cysts, Metachromatic Leukodystrophy, Mitochondrial Cardiomyopathy, Mitochondrial DNA-Associated Leigh Syndrome and NARP, Mucolipidosis IV, Mucopolysaccharidosis Type I, Mucopolysaccharidosis Type IIIA, Mucopolysaccharidosis Type VII, Multiple Endocrine Neoplasia Type 2, Muscle-Eye-Brain Disease, Nemaline Myopathy, Neurological phenotype, Niemann-Pick Disease Due to Sphingomyelinase Deficiency, Niemann-Pick Disease Type C1, Nijmegen Breakage Syndrome, PPT1-Related Neuronal Ceroid-Lipofuscinosis, PROP1-related pituitary hormome deficiency, Pallister-Hall Syndrome, Paramyotonia Congenita, Pendred Syndrome, Peroxisomal Bifunctional Enzyme Deficiency, Pervasive Developmental Disorders, Phenylalanine Hydroxylase Deficiency, Plasminogen Activator Inhibitor I, Polycystic Kidney Disease, Autosomal Recessive, Prothrombin G20210A Thrombophilia, Pseudovitamin D Deficiency Rickets, Pycnodysostosis, Retinitis Pigmentosa, Autosomal Recessive, Bothnia Type, Rett Syndrome, Rhizomelic Chondrodysplasia Punctata Type 1, Short Chain Acyl-CoA Dehydrogenase Deficiency, Shwachman-Diamond Syndrome, Sjogren-Larsson Syndrome, Smith-Lemli-Opitz Syndrome, Spastic Paraplegia 13, Sulfate Transporter-Related Osteochondrodysplasia, TFR2-Related Hereditary Hemochromatosis, TPP1-Related Neuronal Ceroid-Lipofuscinosis, Thanatophoric Dysplasia, Transthyretin Amyloidosis, Trifunctional Protein Deficiency, Tyrosine Hydroxylase-Deficient DRD, Tyrosinemia Type I, Wilson Disease, X-Linked Juvenile Retinoschisis and Zellweger Syndrome Spectrum.

**B. Causal Genetic Variants**

Causal genetic variants are genetic variants for which there is statistical, biological, and/or functional evidence of association with a disease or trait such that the variant is likely to play a role in etiology. A single causal genetic variant can be associated with more than one trait. A causal genetic variant can be associated with a Mendelian trait or a non-Mendelian trait or both.

Although it is typical to refer to genetic variants that occur at a frequency of greater than or equal to 1% as polymorphisms and refer to a less common variants as mutations, these terms are used interchangeably and without intending to refer to a particular frequency, unless specified. For example, a SNP is a single nucleotide polymorphism, and it can occur at any frequency. An example of a causal genetic variant that is a SNP is the Hb S variant of hemoglobin that causes sickle cell anemia. A DIP is a deletion/insertion polymorphism, and it can occur at any frequency. DIPs can also be referred to as indels. An example of a causal genetic variant that is a DIP is the delta508 mutation of the CFTR gene which causes cystic fibrosis. A CNV is a copy number variant, and it can occur at any frequency. An example of a causal genetic variant that is a CNV is trisomy 21, which causes Downs syndrome. A STR is a short tandem repeat variation, and it can occur at any frequency. STRs are also called repeat polymorphisms. An example of a causal genetic variant that is an STR is tandem repeat that causes Huntington's disease.

Causal genetic variants can manifest as variations in a DNA polynucleotide, such as results from a SNP, DIP, CNV, STR, or heritable epigenetic modification (for example, DNA methylation). Multiple instantiations of a causal genetic variant can be recognized as such because they are identical-by-state and/or identical-by-descent. A causal genetic variant may also be a set of closely related causal genetic variants. Some causal genetic variants may exert influence as sequence variations in RNA polynucleotides. At this level, some causal genetic variants are also indicated by the presence or absence of a species of RNA polynucleotides. Also, some causal genetic variants result in sequence variations in protein polypeptides. At this level, some causal genetic variants are also indicated by the presence or absence of a species of protein polypeptides.

The nomenclature for cataloging and describing causal genetic variants was developed on an ad hoc basis over many years. Causal genetic variants can be described by more than one name. For example, causal genetic variants can be labeled with both a common name and a systematic name. Systematic names are based on an evolving notation developed by the Human Genome Variation Society.

Causal genetic variants are distinguished from indirectly associated genetic variants, which are associated with a trait or disease as a result of correlated coinheritance with a causal genetic variant.

Causal genetic variants can be the cause of Mendelian and non-Mendelian traits. In many instances, causal genetic variants that determine deleterious Mendelian traits and Mendelian diseases can be rare and occur at a frequency less than 1% of a population. However, some causal genetic variants that determine deleterious Mendelian traits and Mendelian diseases occur at frequencies in the range of 1% to 10%. In some cases, causal genetic variants that determine deleterious Mendelian traits and Mendelian diseases occur at frequencies greater than 10%.

Causal genetic variants can be originally discovered by statistical and molecular genetic analyses of the genotypes and phenotypes of individuals, families, and populations. The causal genetic variants for Mendelian traits are typically identified in a two-stage process. In the first stage, families in which multiple individuals who possess the trait are examined for genotype and phenotype. Genotype and phenotype data from these families is used to establish the statistical association between the presence of the Mendelian trait and the presence of a number of genetic markers, which typically are indirectly associated genetic variants that are physically proximal to a causal genetic variant on the chromosome. This association establishes a candidate region in which the causal genetic variant is likely to map. In a second stage, the causal genetic variant itself is identified. The second step typically entails sequencing the candidate region. More sophisticated, one-stage processes are possible with more advanced technologies which permit the direct identification of a causal genetic variant or the identification of smaller candidate regions. After one causal genetic variant for a trait is discovered, additional variants for the same trait can be discovered by simple methods. For example, the gene associated with the trait can be sequenced in individuals who possess the trait or their relatives. The invention of new methods for discovering causal genetic variants is an active area of research. The application of existing methods and the incorporation of new methods is expected to continue to result in the discovery of additional causal genetic variants which can be used or tested for by the devices, systems, and methods herein. Many causal genetic variants are cataloged in databases including the Online Mendelian Inheritance in Man (OMIM) and the Human Gene Mutation Database (HGMD). Causal genetic variants are also reported in the scholarly literature, at conferences, and in personal communications between scholars.

In diploid organisms, including humans and other mammals, a causal genetic variant can be present in zero, one, or two copies. In some cases, the causal genetic variant can be present in more than two copies. Methods for detecting the presence of a causal genetic variant typically also determine the number of copies of the variant that are present.

In an embodiment at least one of the causal genetic variants causes a trait having an incidence of no more than 1% in a first of the populations and at least one other of the causal genetic variants causes a trait having an incidence of no more than 1% in a second of the populations. In another embodiment at least one of the causal genetic variants causes a trait having an incidence of no more than 1/10,000 in a first of the populations and at least one other of the causal genetic variants causes a trait having an incidence of no more than 1/10,000 in a second of the populations.

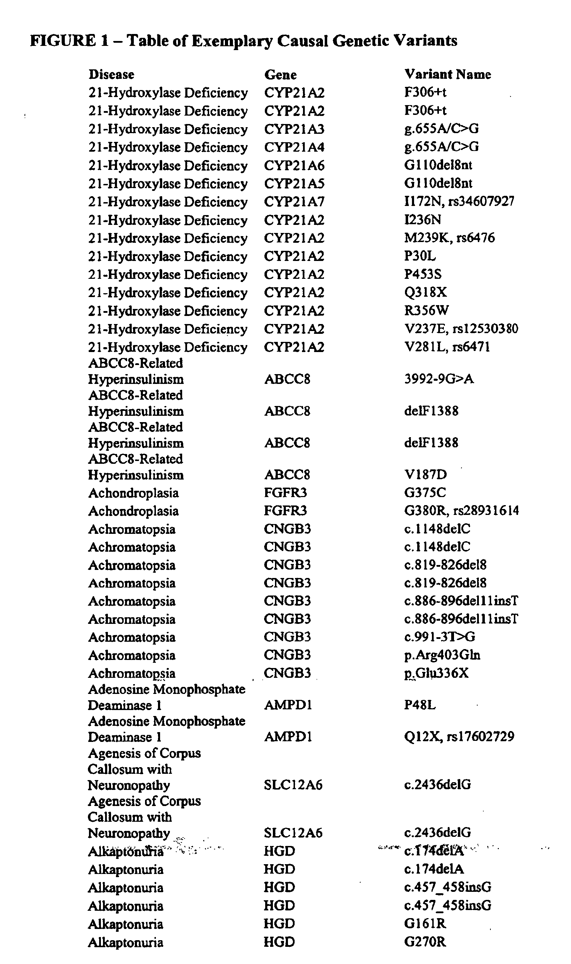

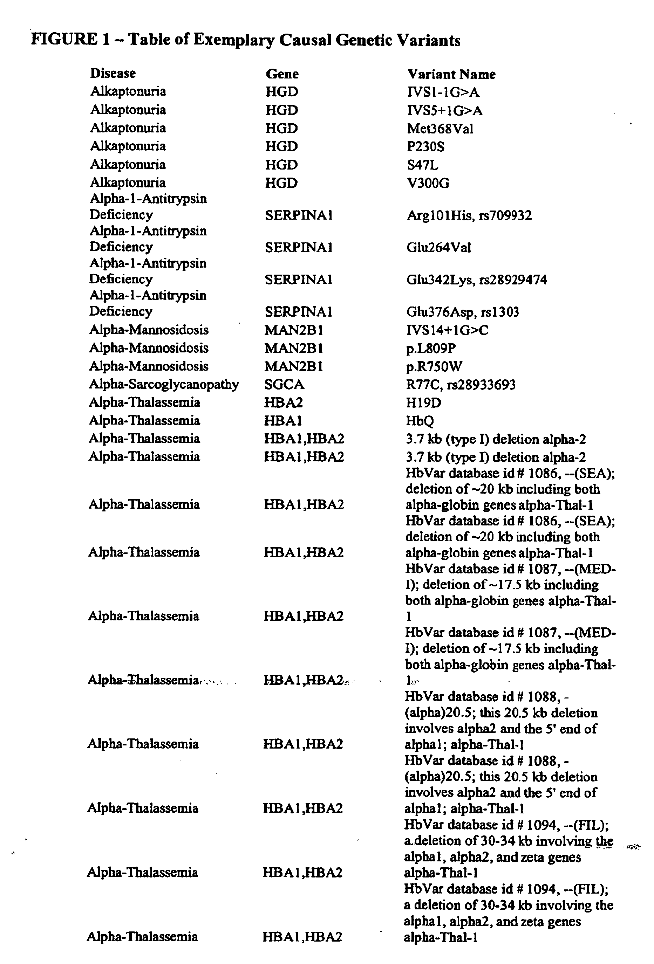

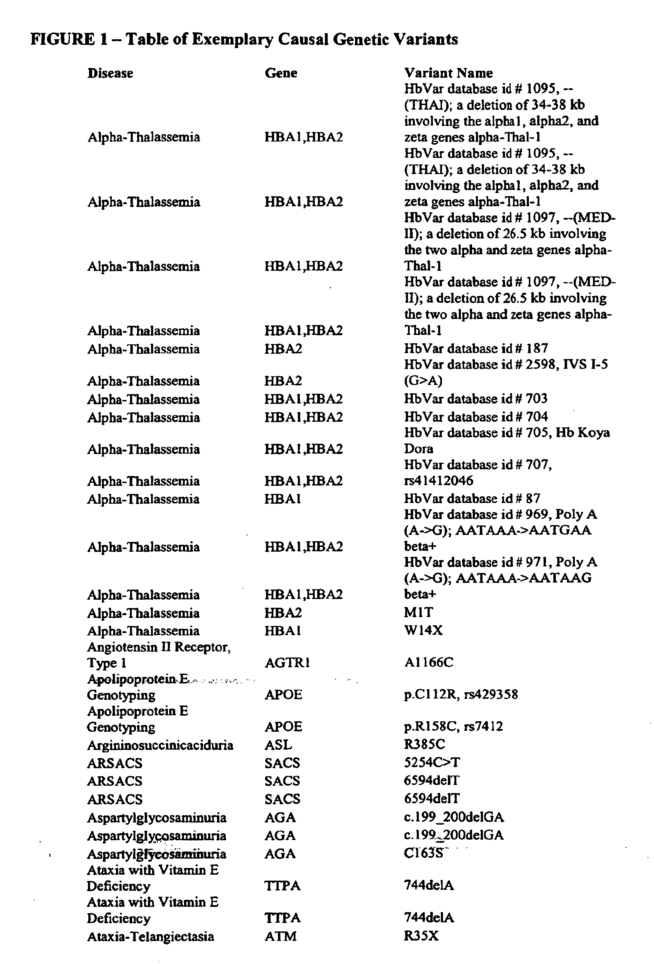

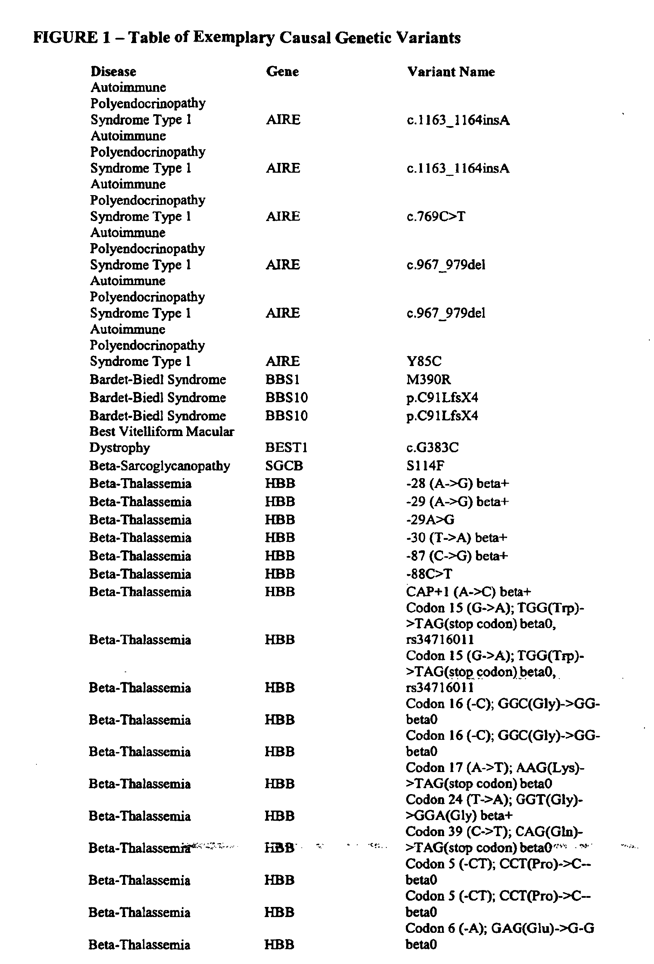

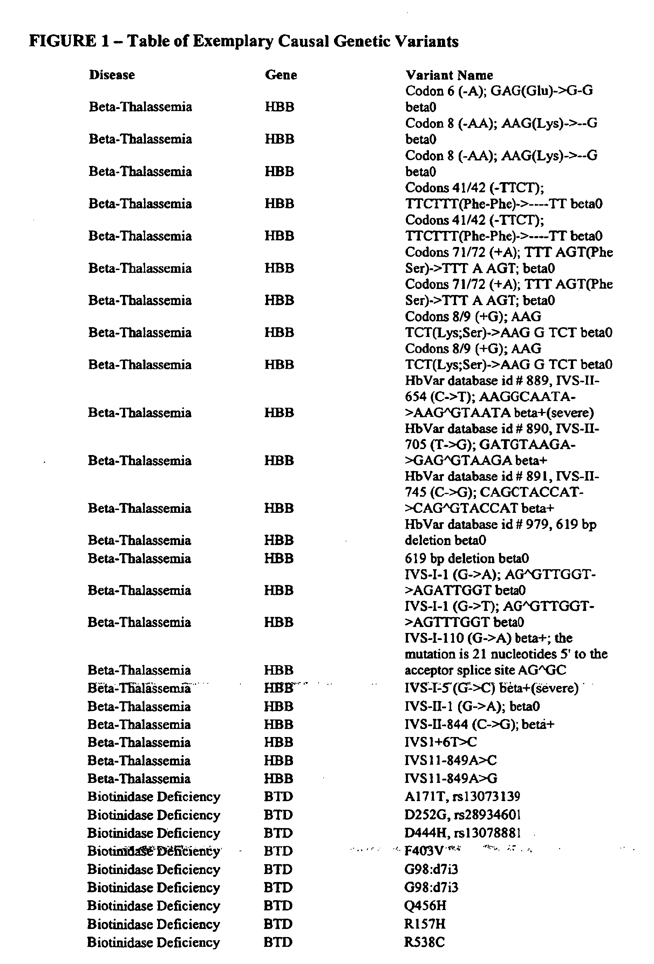

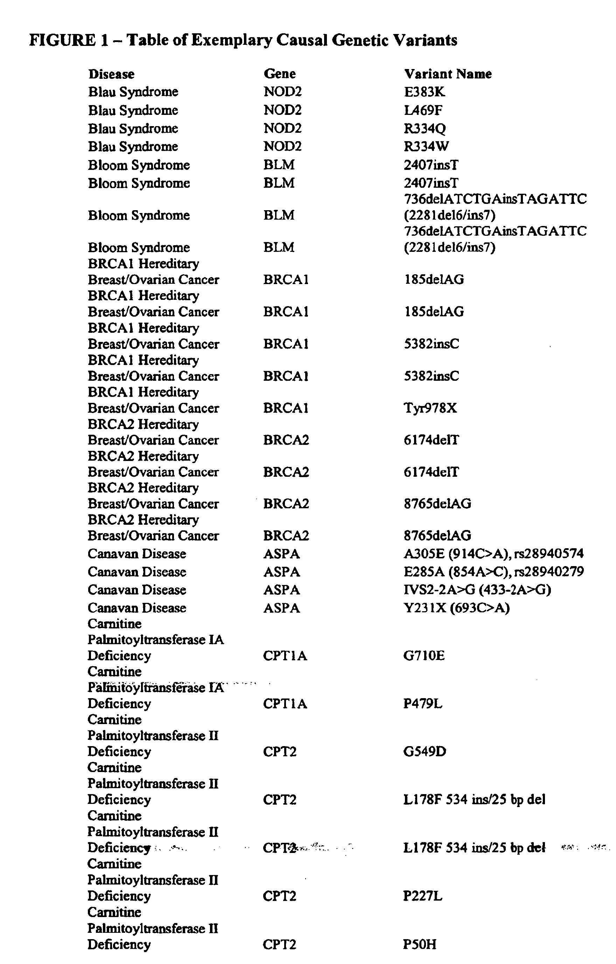

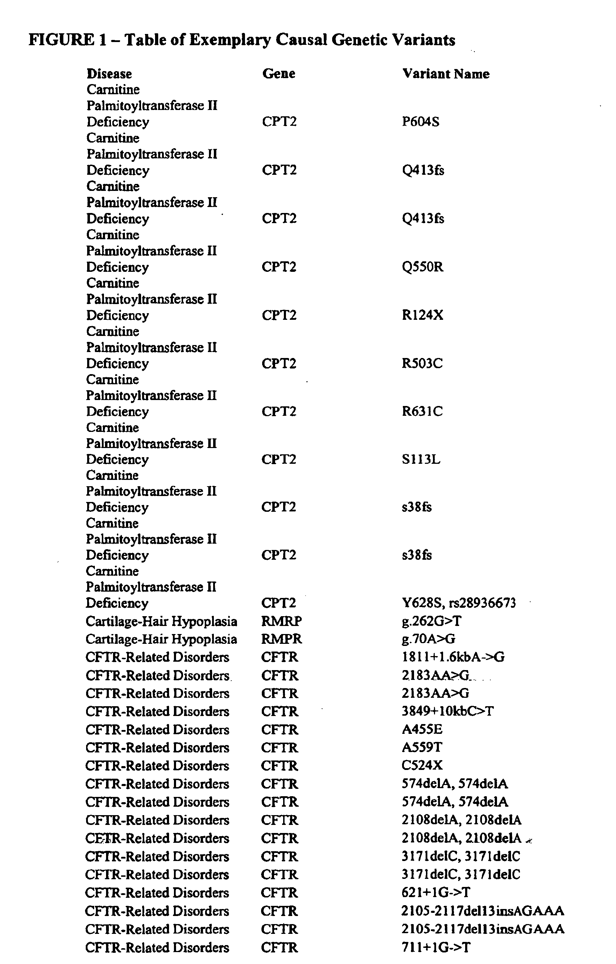

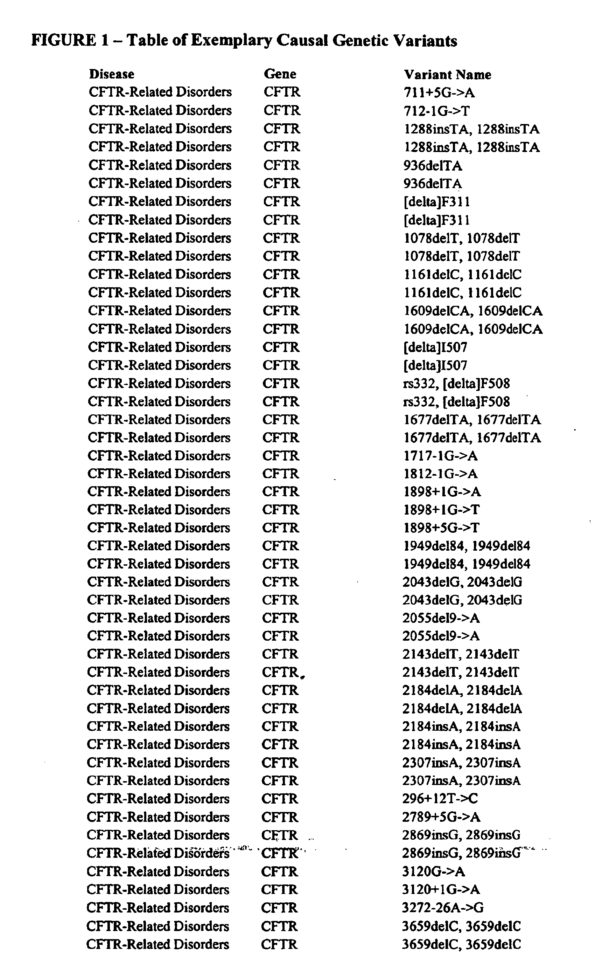

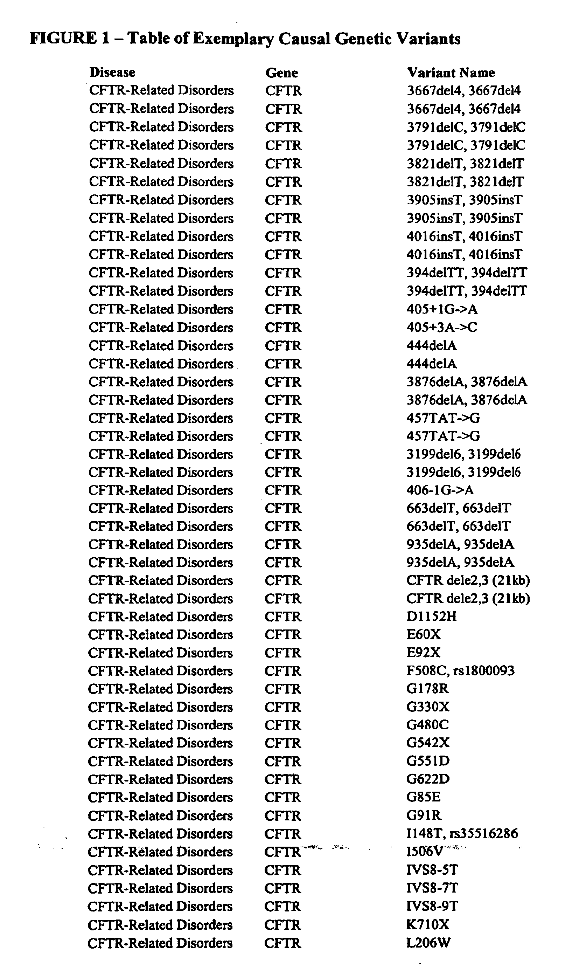

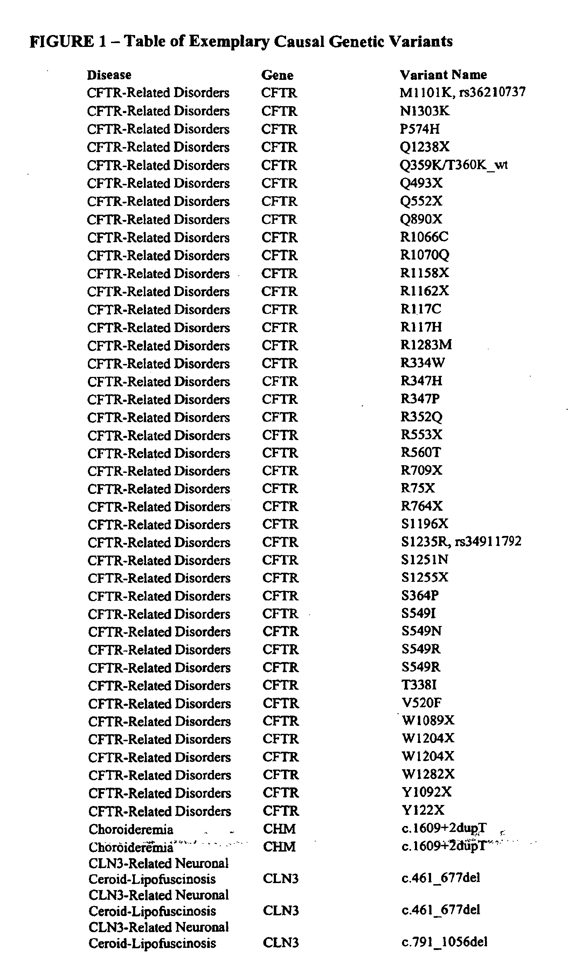

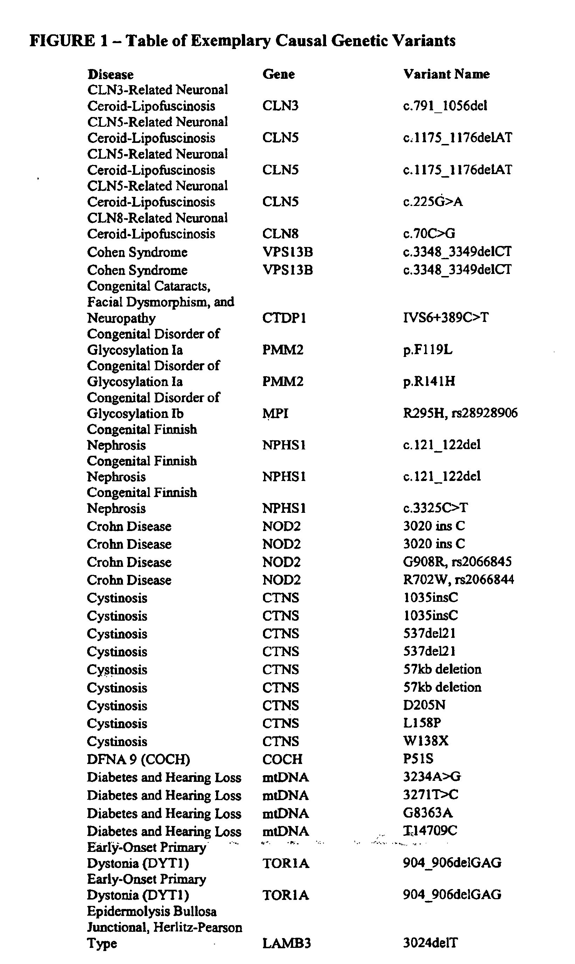

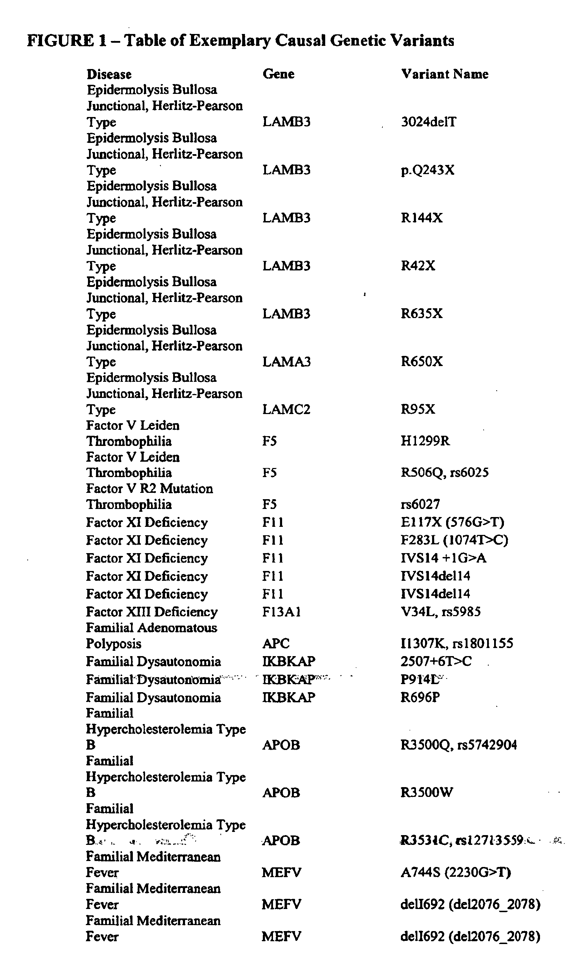

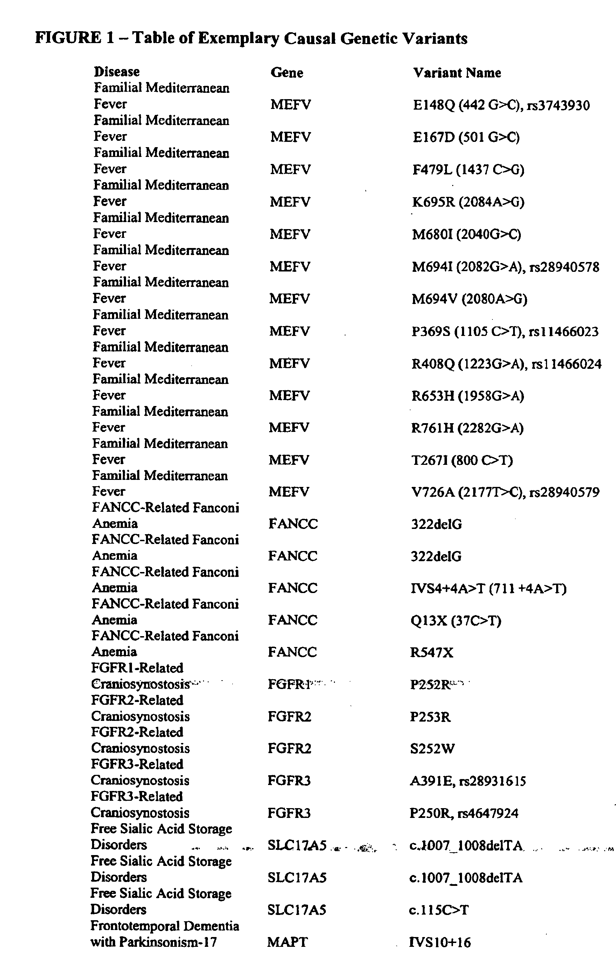

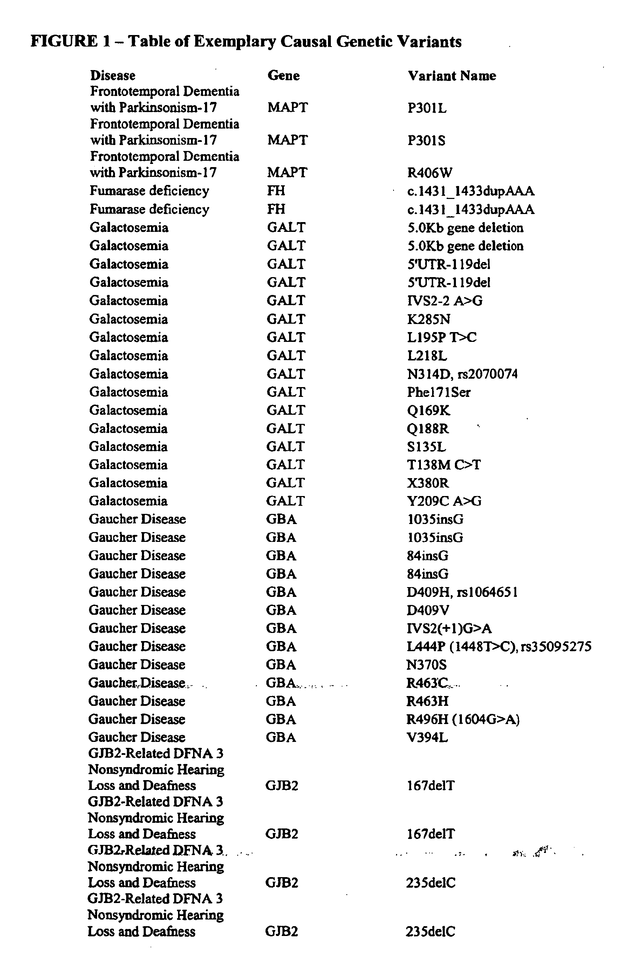

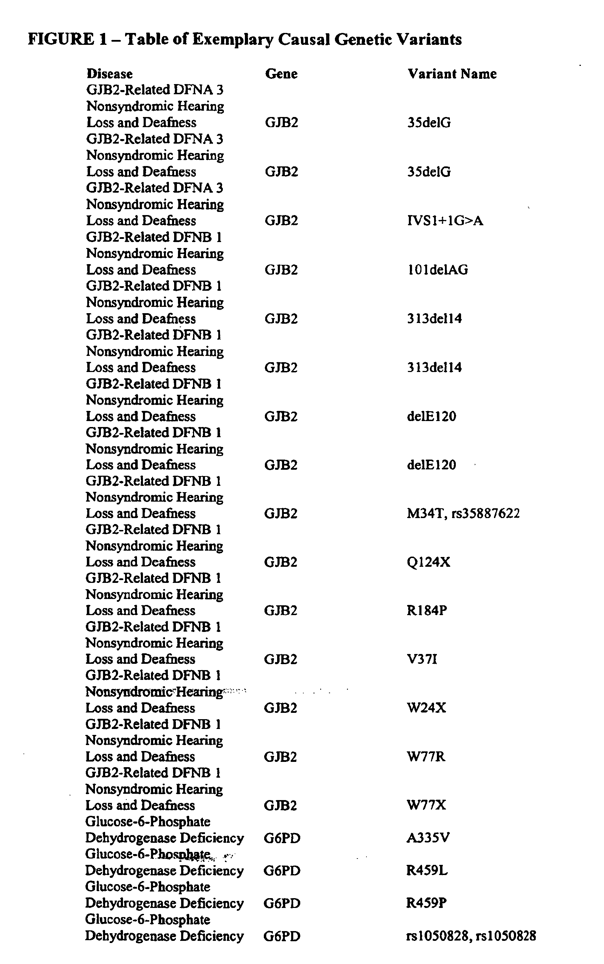

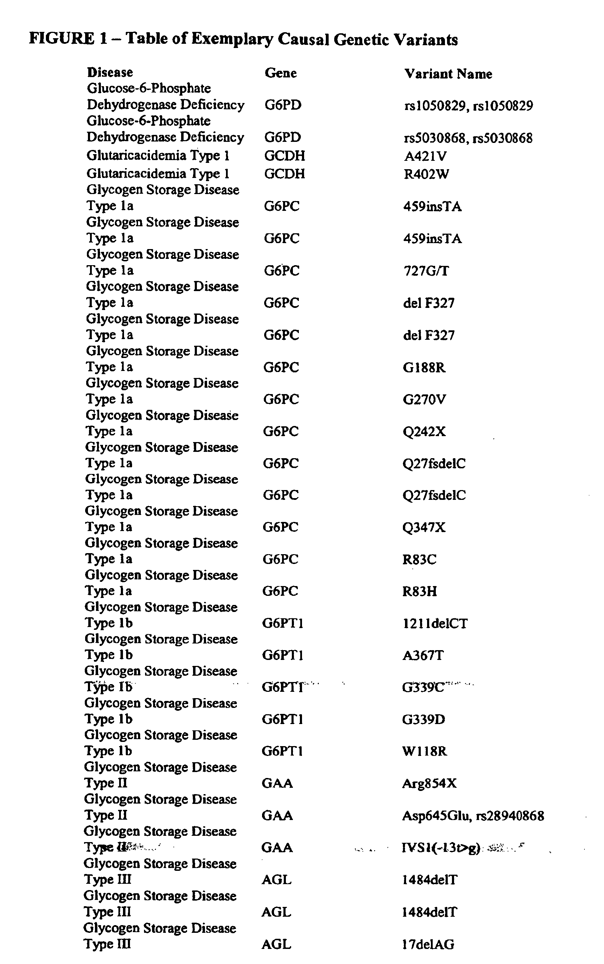

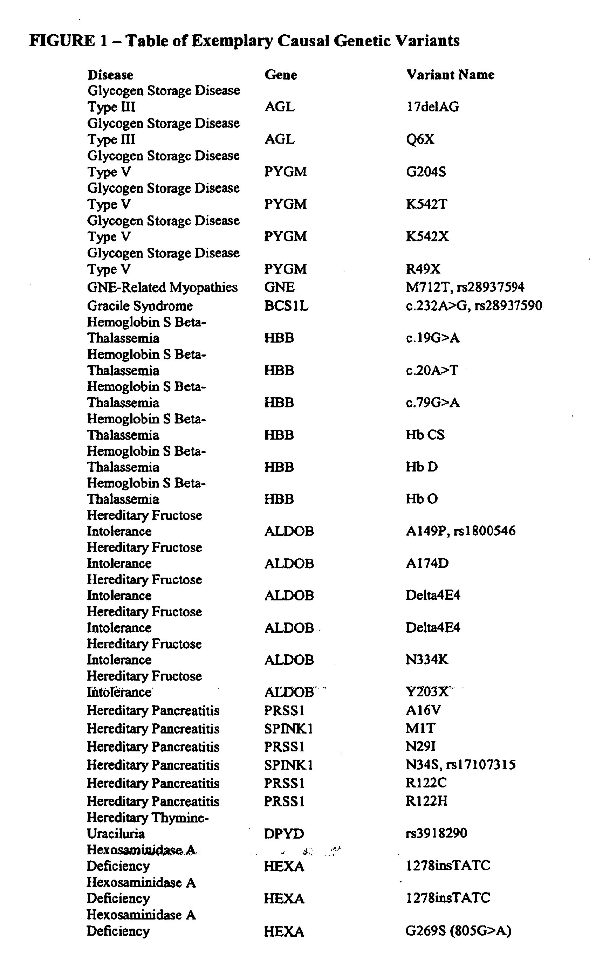

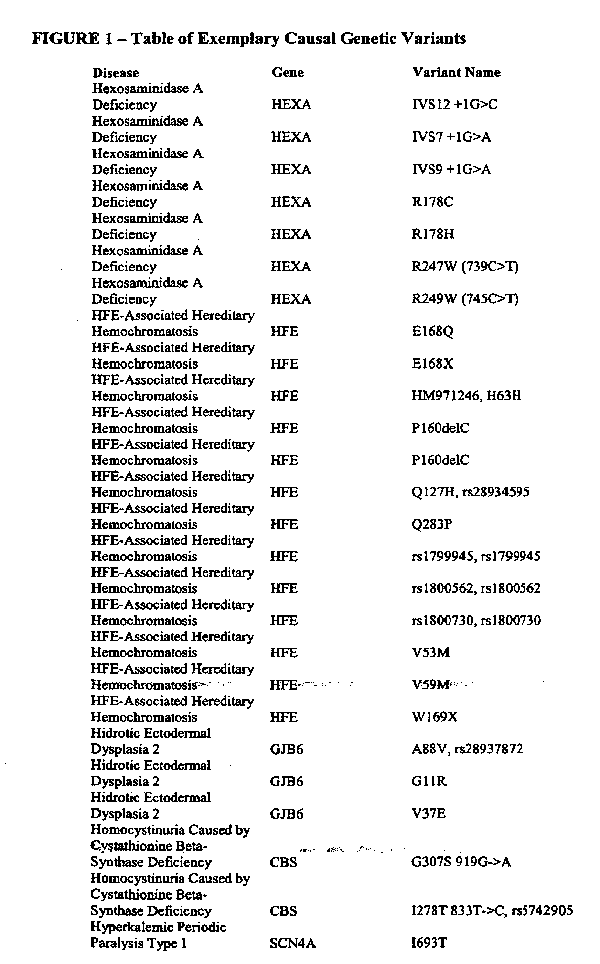

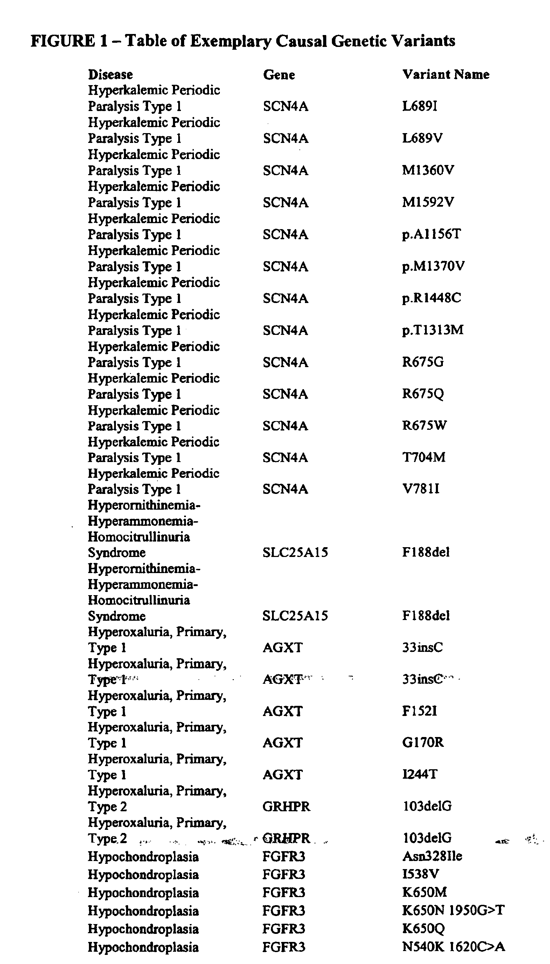

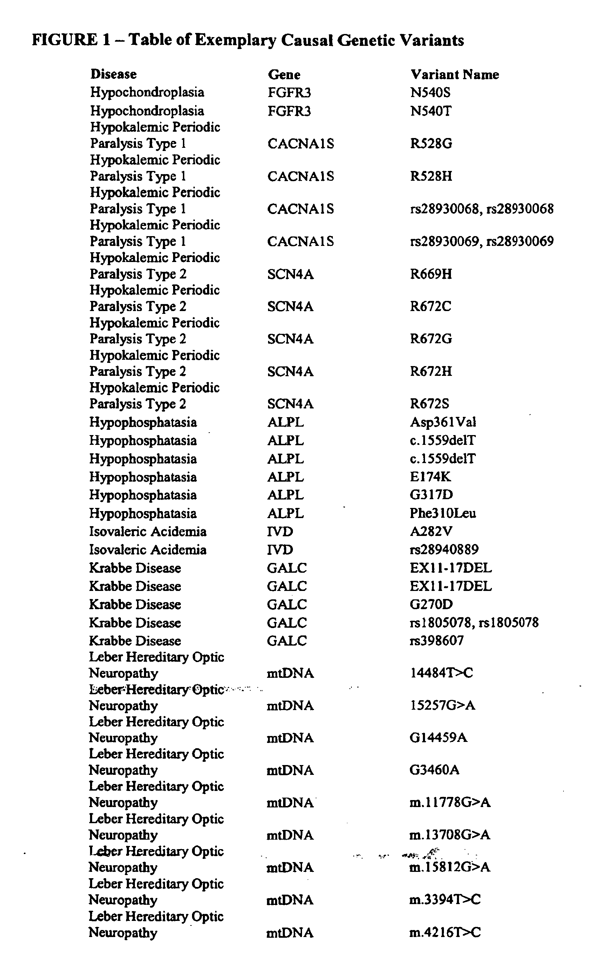

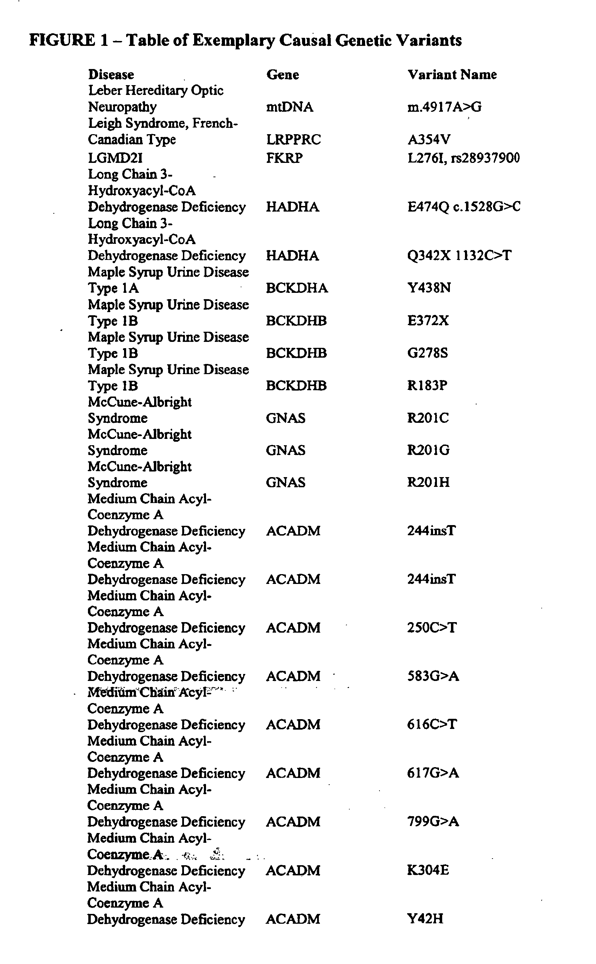

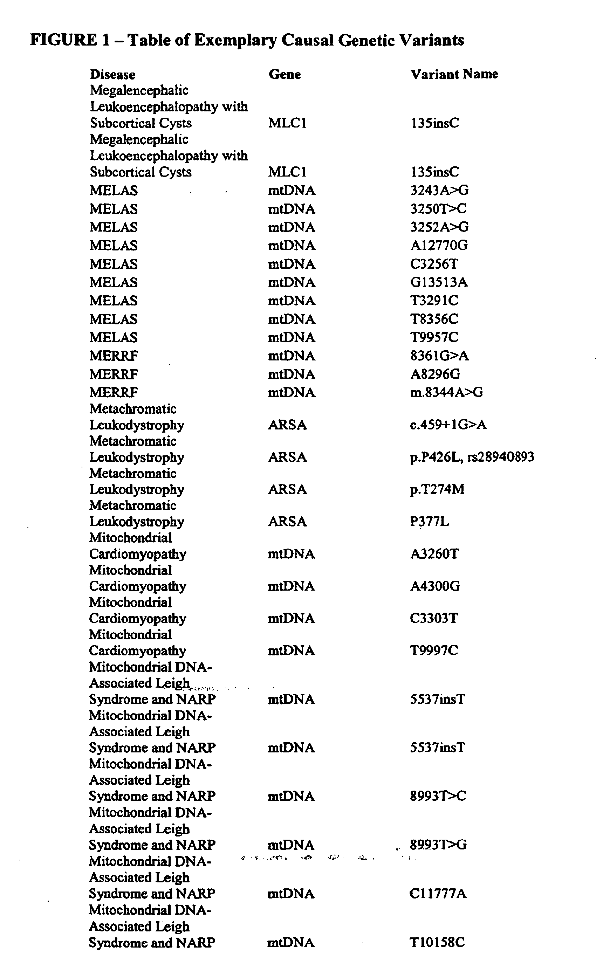

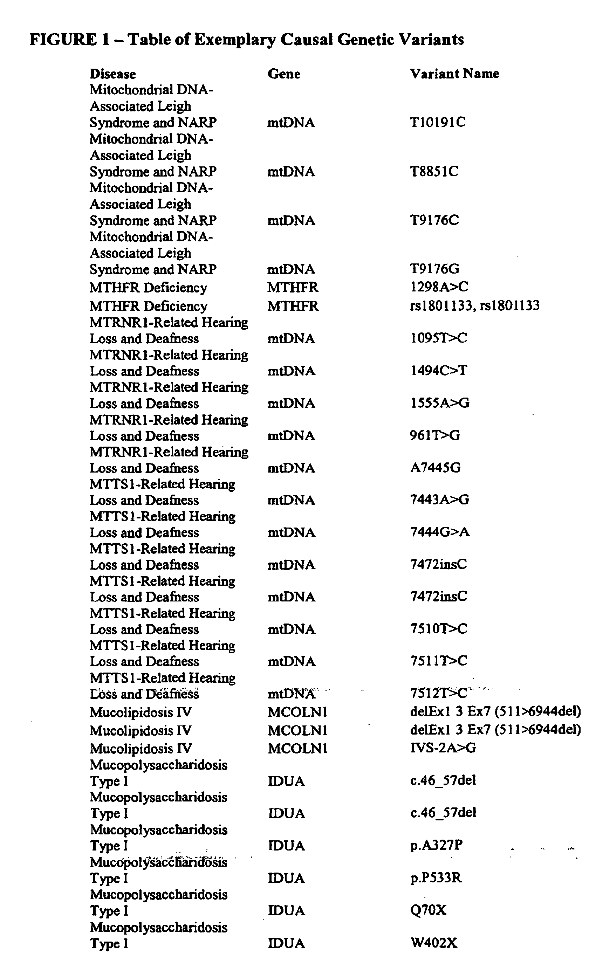

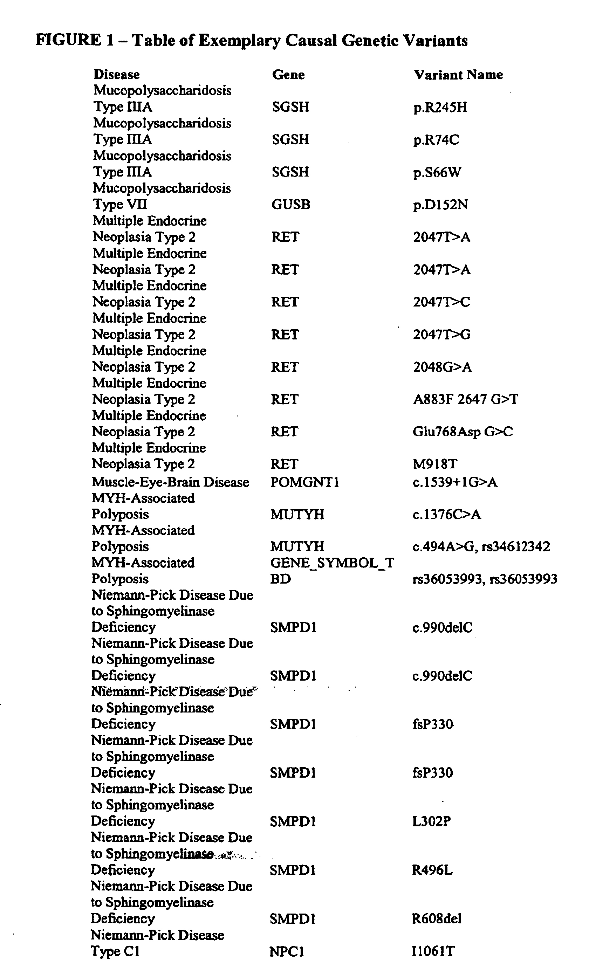

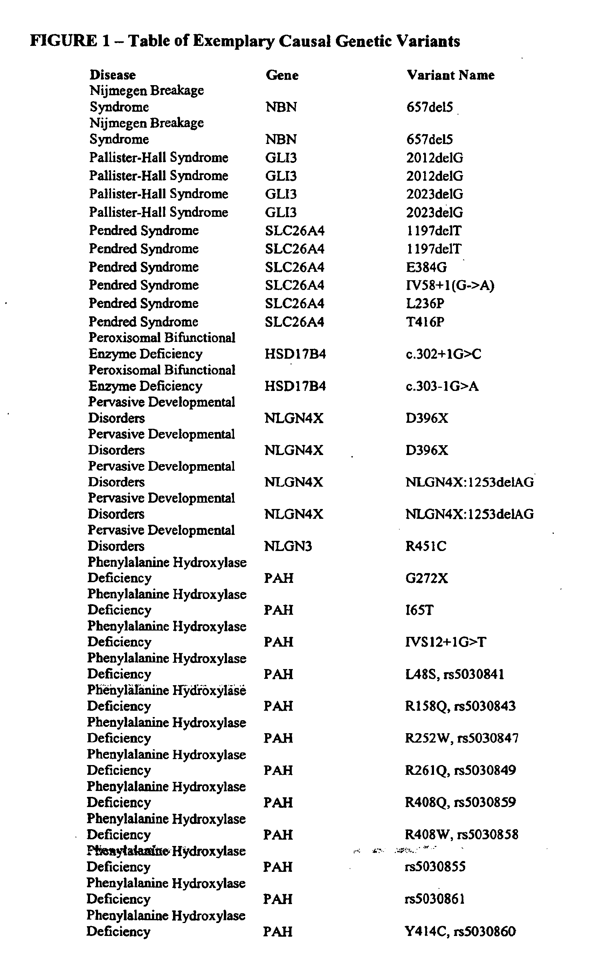

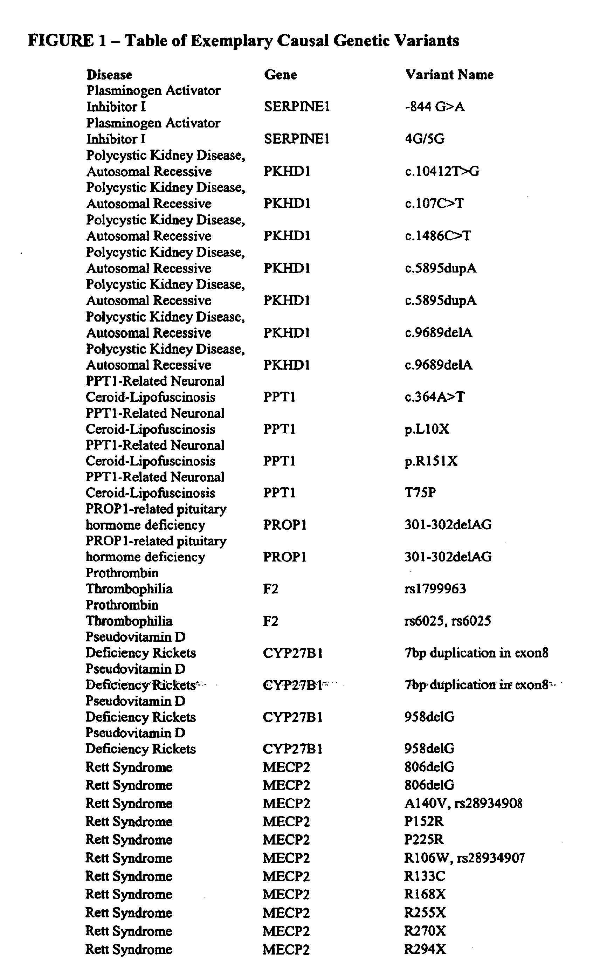

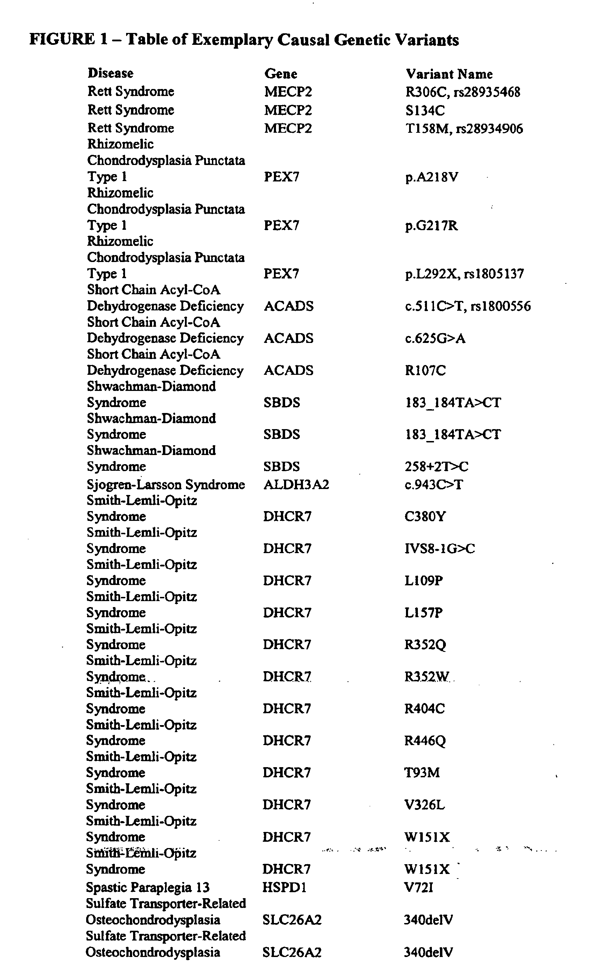

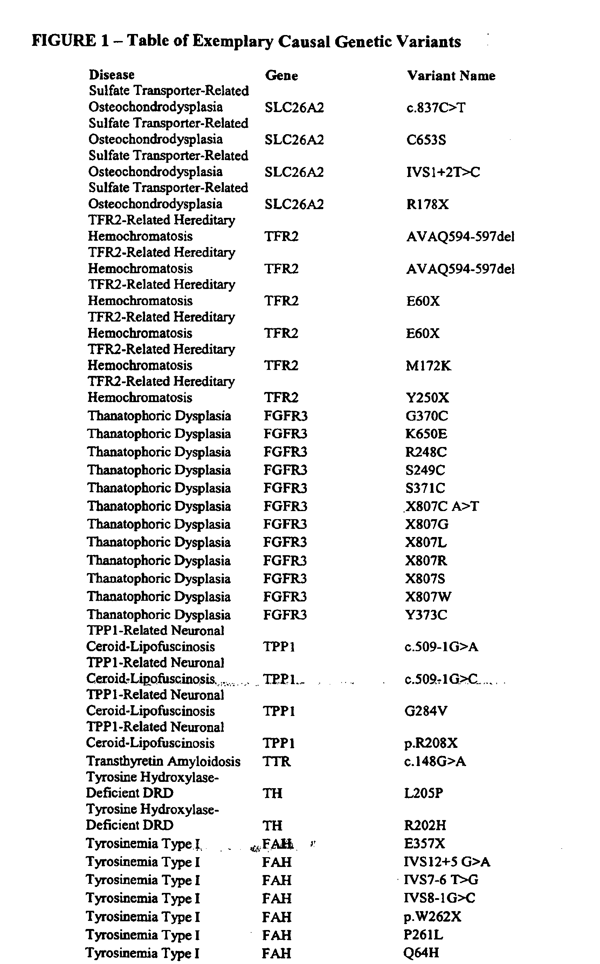

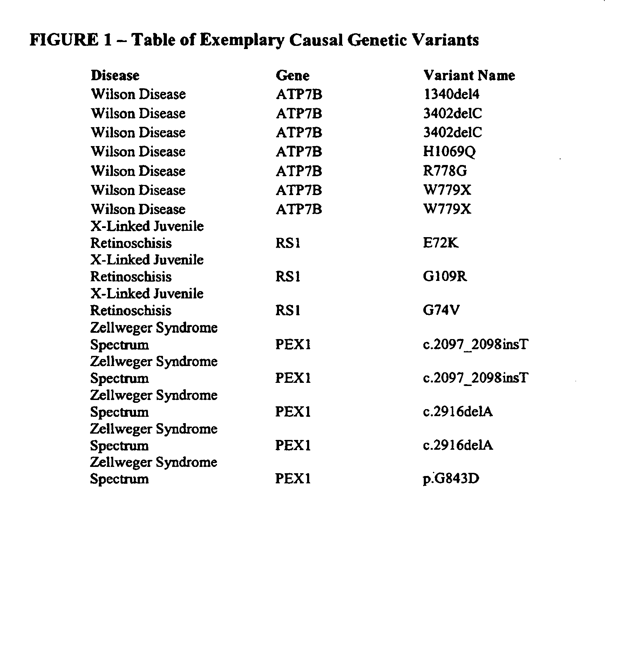

Figure 1 provides a table of exemplary causal genetic variants for rare genetic diseases.

## C. Populations

In some instances, devices, methods, and systems herein are configured to test for causal genetic variants for traits, wherein the traits differ in frequency between populations. Populations are groups of individuals of the same species, such as humans or other mammals. Human populations are often given particular names which can form the basis for social identities.

A human population can be a group of people sharing a common genetic inheritance, such as an ethnic group (for example, Caucasian). A human population can be a haplotype population or group of haplotype populations (for example, haplotype H1, M52). A human population can be a national group (for example, Americans, English, Irish). A human population can be a demographic population such as those delineated by age, sex, and socioeconomic factors. Human populations can be historical populations.

A population can consist of individuals distributed over a large geographic area such that individuals at extremes of the distribution may never meet one another. The individuals of a population can be geographically dispersed into discontinuous areas. Populations can be informative about biogeographical ancestry. The labels used to identify populations do not need to contain an explicit geographic or ancestry reference for the populations to be informative about ancestry, for example, a personal identification of race.

Populations can also be defined by ancestry. Genetic studies can define populations. As defined by ancestry and genetics, the major human populations correspond to continental scale groupings, which include Western Eurasian, sub-Saharan African, East Asian, and Native American. Most humans can be assigned to at least one of these populations on the basis of ancestry. A number of smaller populations are also distinguished as continental groups, including Indigenous Australian, Oceanian, and Bushmen.

Very often, populations can be further decomposed into sub-populations. The relationship between populations and subpopulations can be hierarchical. For example, the Oceanian population can be further sub-divided into sub-populations including Polynesians, Melanesians and Micronesians. The Western Eurasian population can be further sub-divided into sub-populations including European, Western/Central Asian, South Asian, and North African. The European population can be further sub-divided into sub-populations including North-Western European, Southern European, and Ashkenazi Jewish populations. The North-Western European population can be further sub-divided into national populations including English, Irish, German, Finnish, and the like. The East Asian population can be further sub-divided into Chinese, Japanese, and Korean subpopulations. The South Asian population can be further sub-divided into Indian and Pakistani populations. The Indian population can be further sub-divided into Dravidian people, Brahui people, Kannadigas, Malayalis, Tamils, Telugus, Tuluvas, and Gonds.

An individual can self-identify to one or more reference populations (for example, to an ethnic group) on the basis of their ancestry. A person may identify their ancestry based on the identity of their four biological grandparents. The ancestry of some individuals (sometimes called their individual ancestry or individual biogeographical ancestry) can be described as coming from more than one distinct population. Admixture is defined relative to a set of reference populations. The ancestry of an individual can be classified in terms of admixture of a set of reference populations. These reference populations can vary depending on the application. It is common to refer to admixture in term of the four major continental groups because these groups can be readily distinguishable genetically and phenotypically.

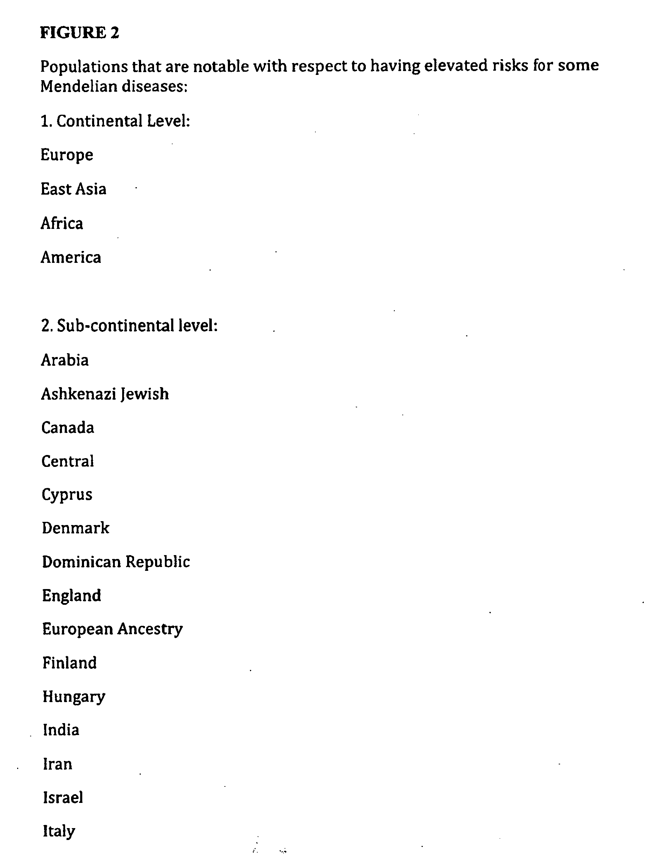

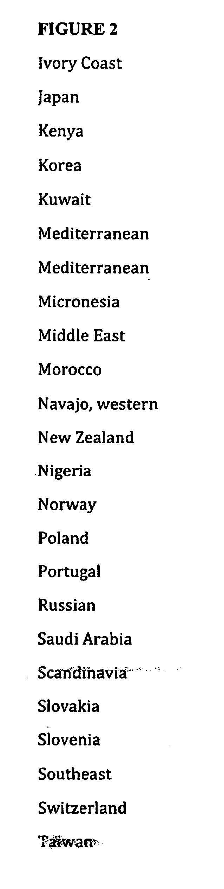

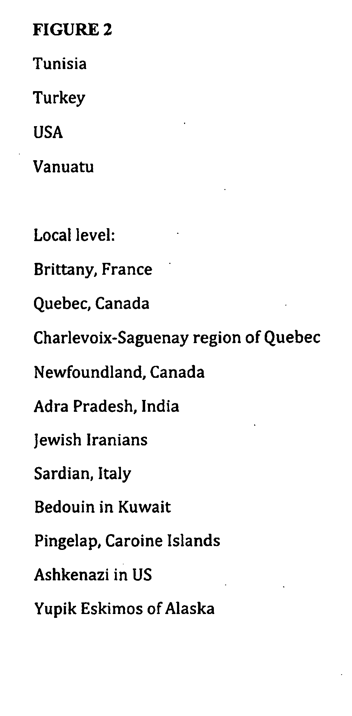

Figure 2 provides a list of exemplary populations.

## D. Ancestry Informative Markers

Ancestry informative markers (AIMs) are genetic variants that differ in frequency between populations. Almost all common genetic variants differ both within and between populations. However, individual variants are found across a spectrum of variance distributions. In an instance, variants that differ within but not between populations are typically not AIMs. In another instance, variants that differ between but not within populations are especially informative.

A variety of kinds of genetic variants can be AIMs, including SNPs, DIPs, CNVs, and STRs. AIMs can also be sequence variations in RNA polynucleotides. Some AIMs can also be indicated by the presence or the concentration of a species of RNA polynucleotides. Some AIMs can also be sequence variations in protein polypeptides. Some AIMs can also be indicated by the presence or absence of a species of protein polypeptides.

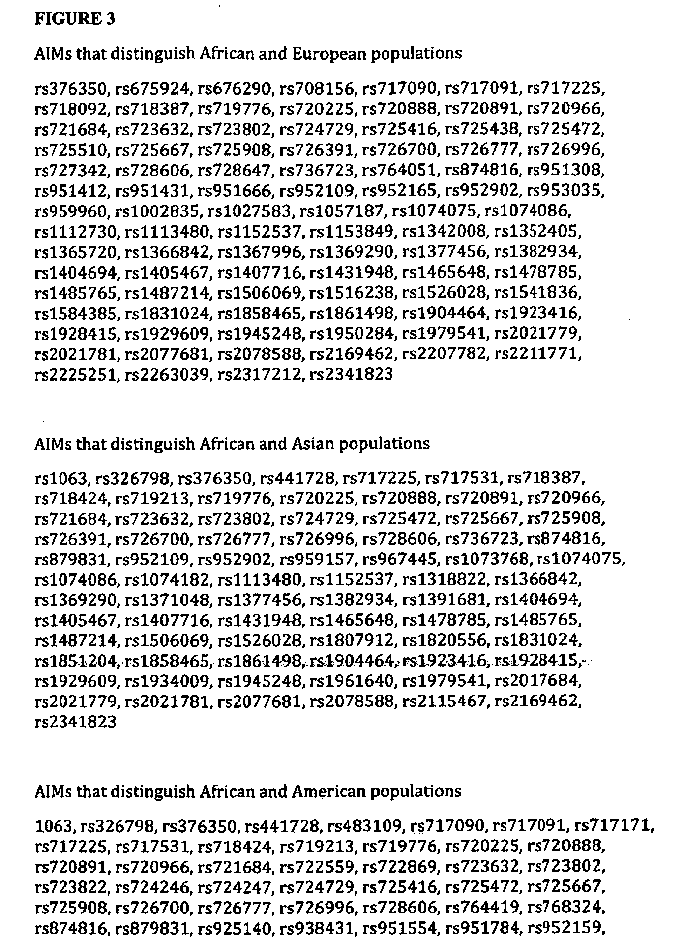

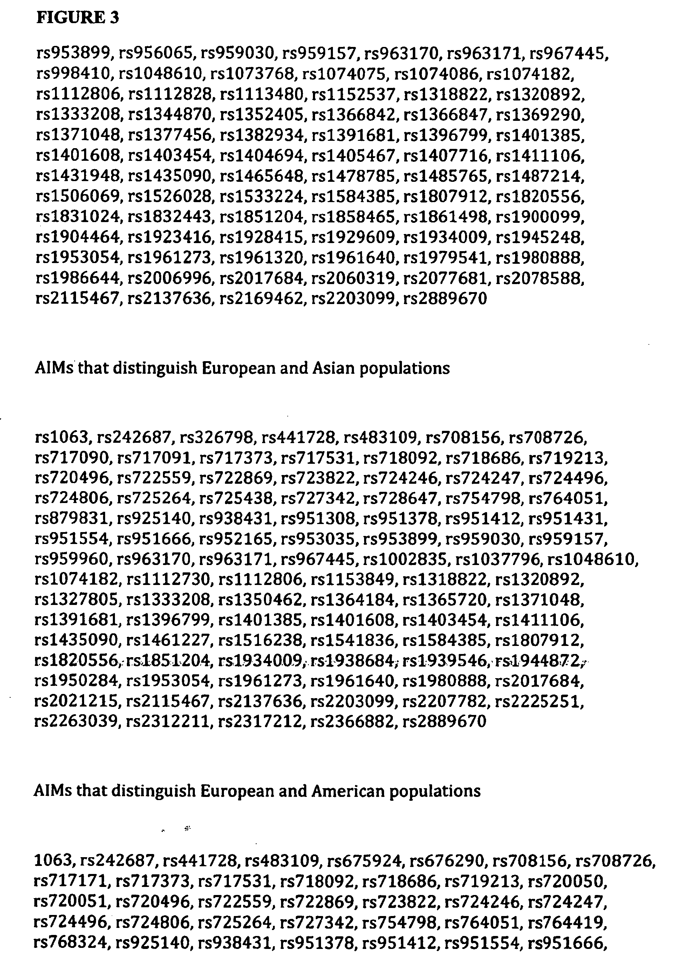

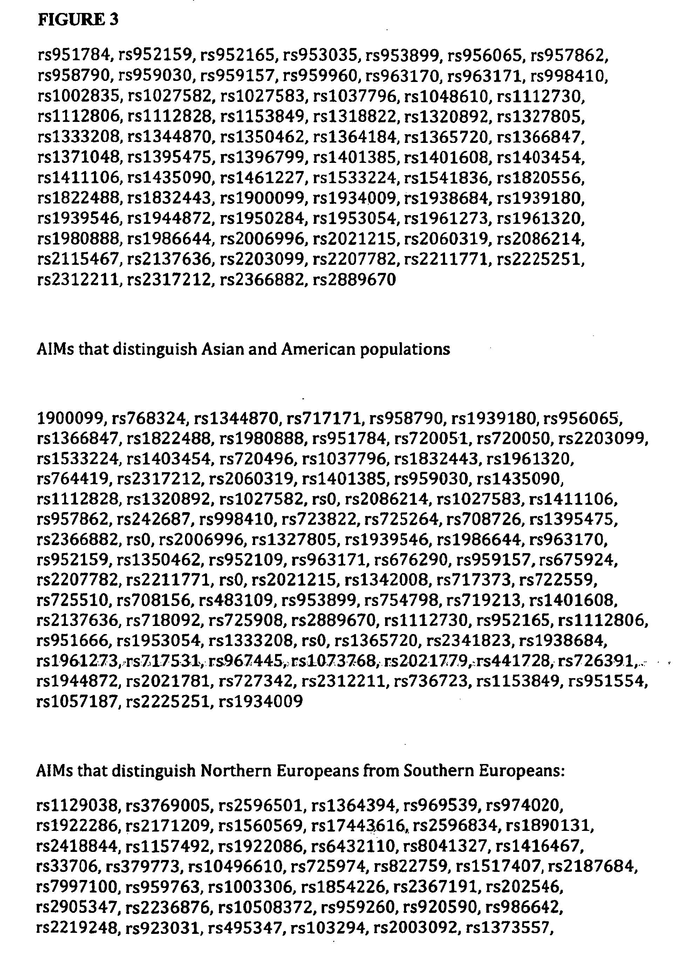

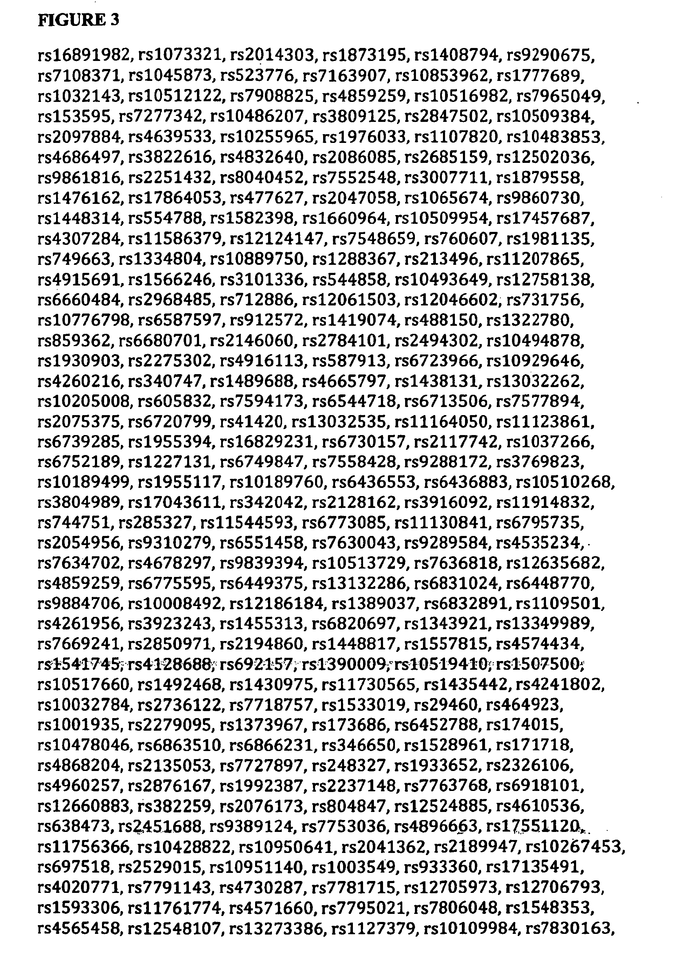

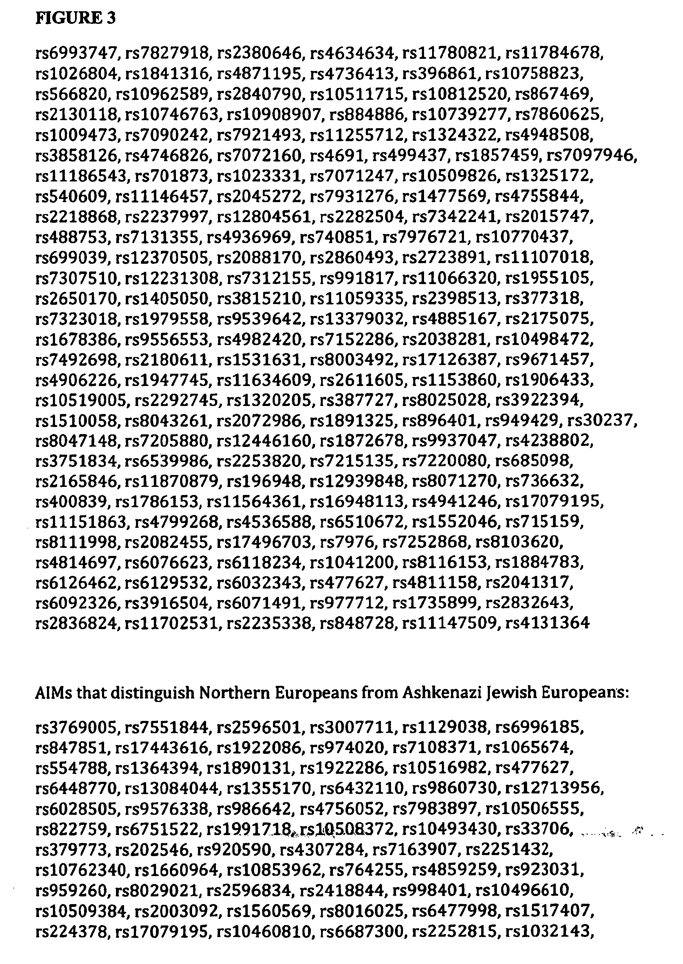

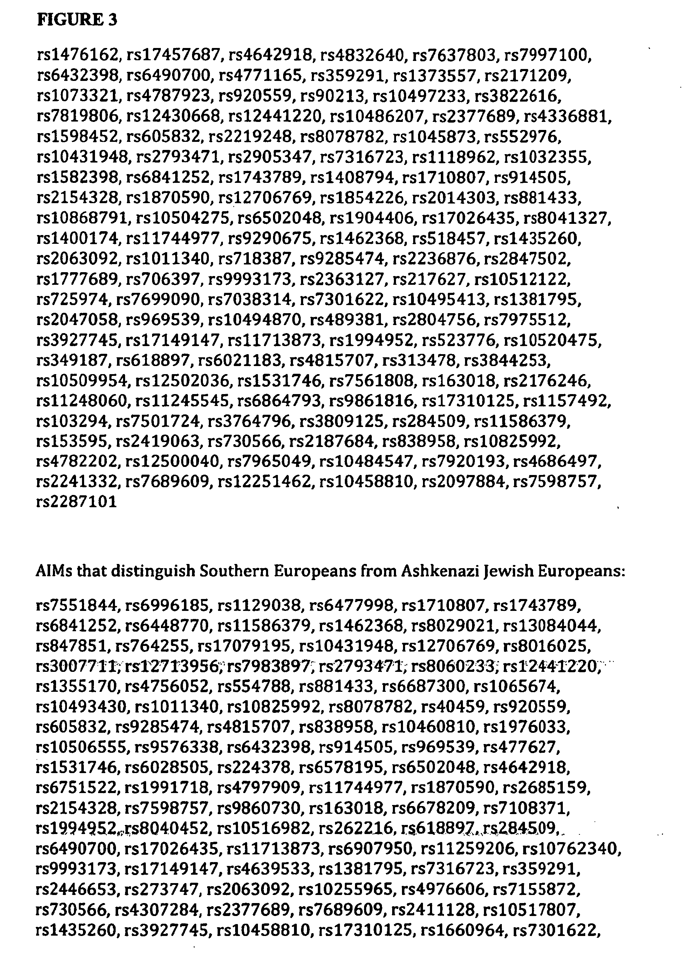

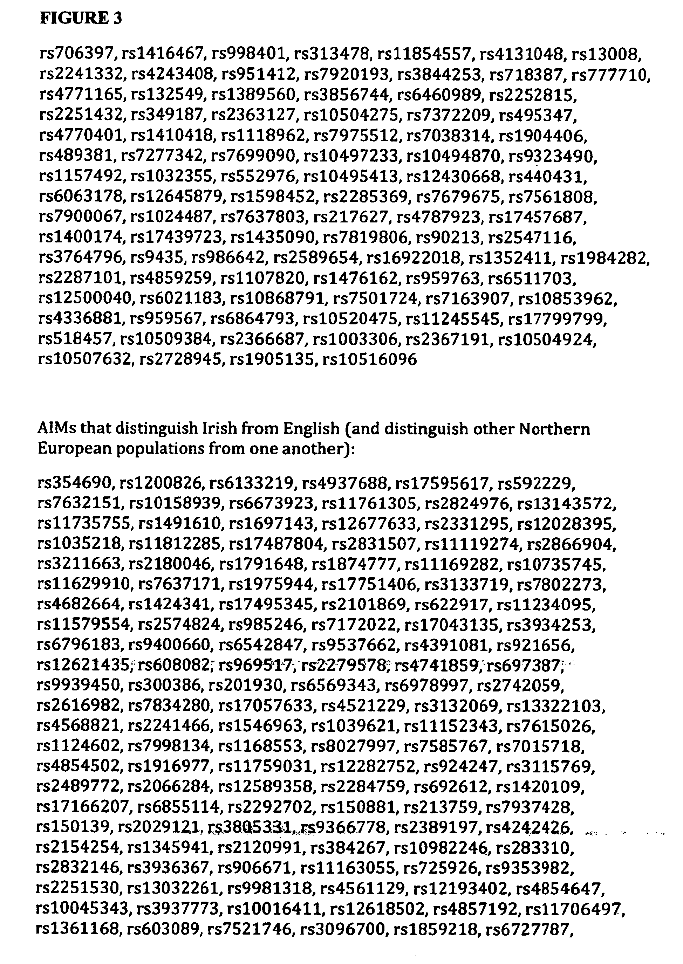

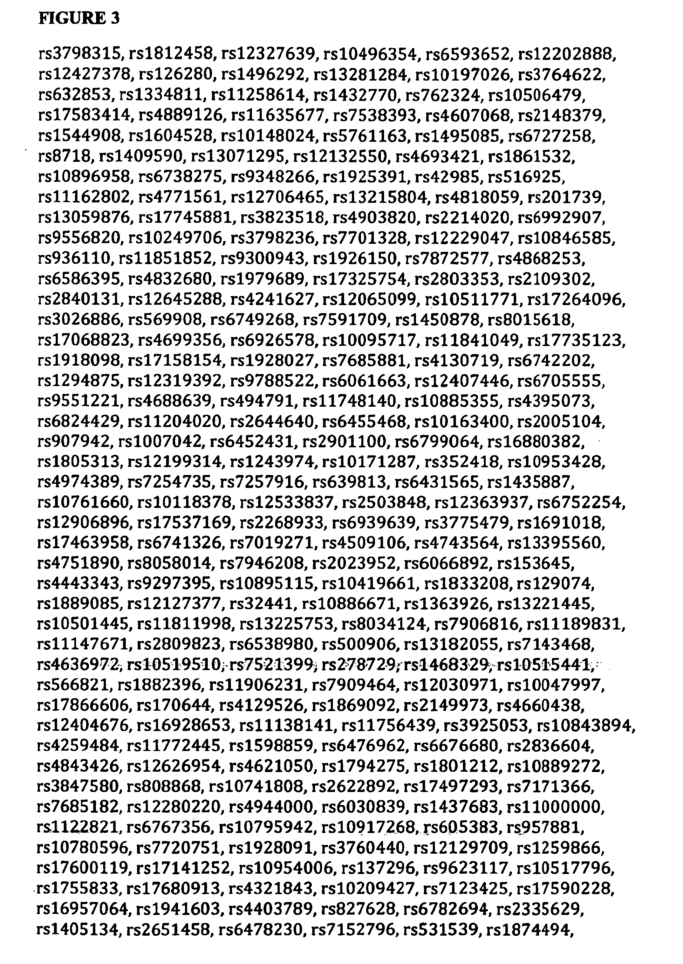

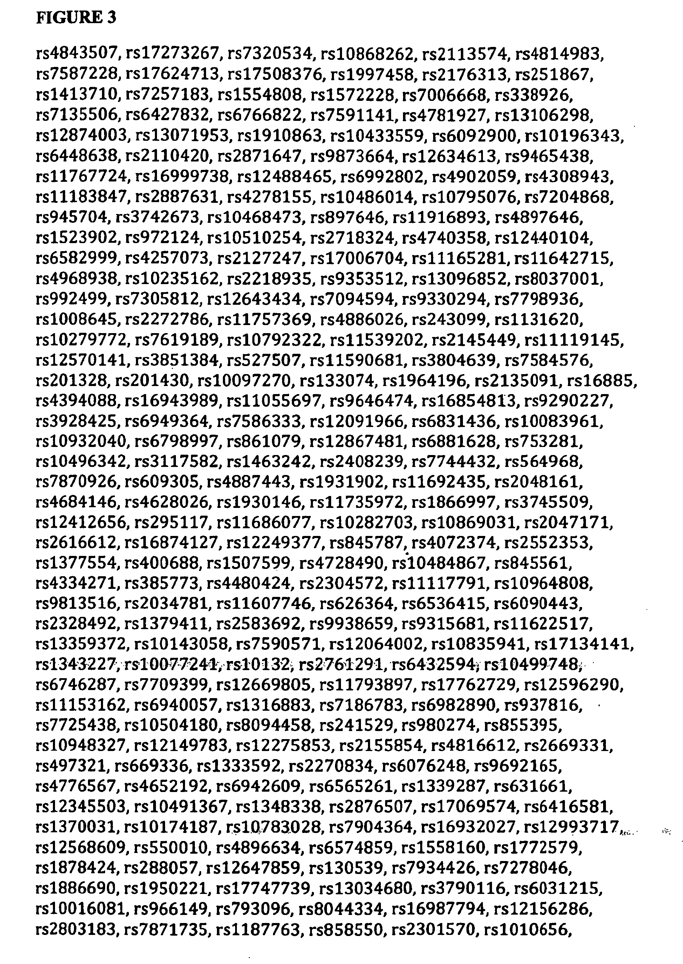

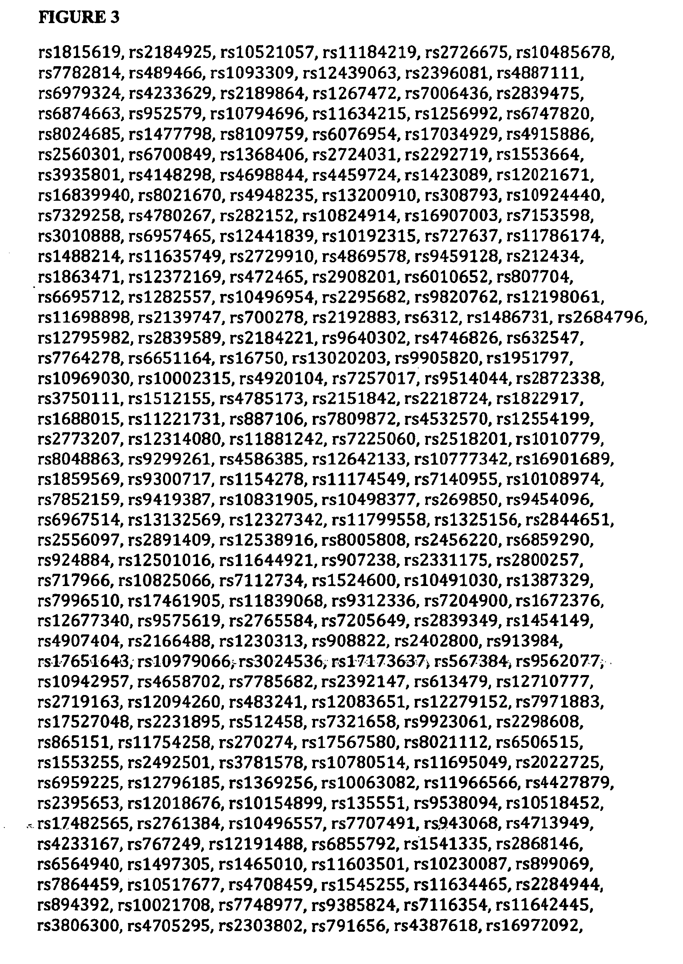

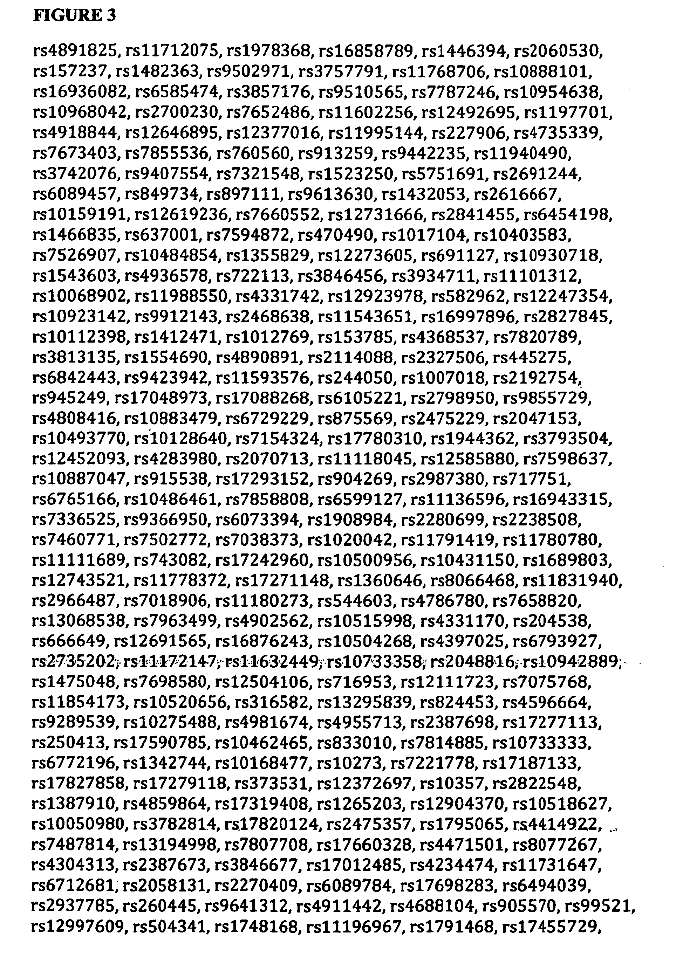

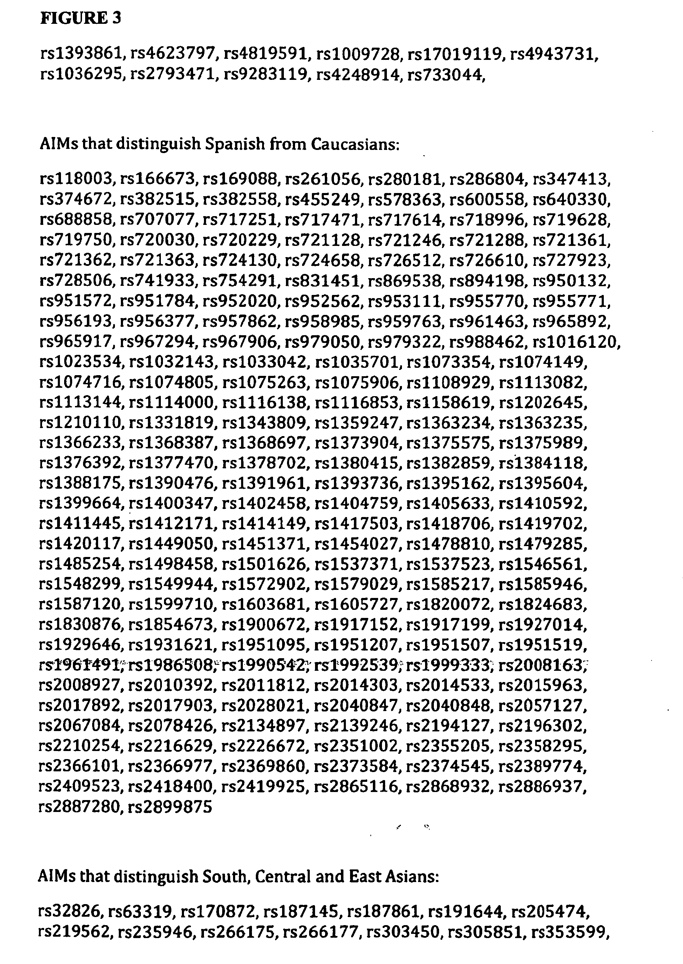

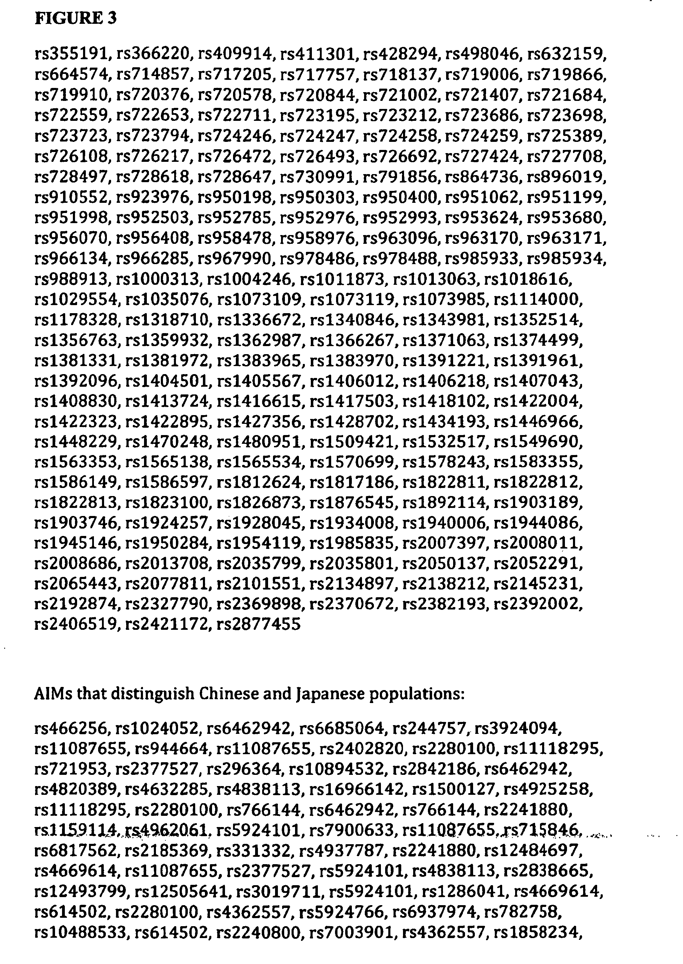

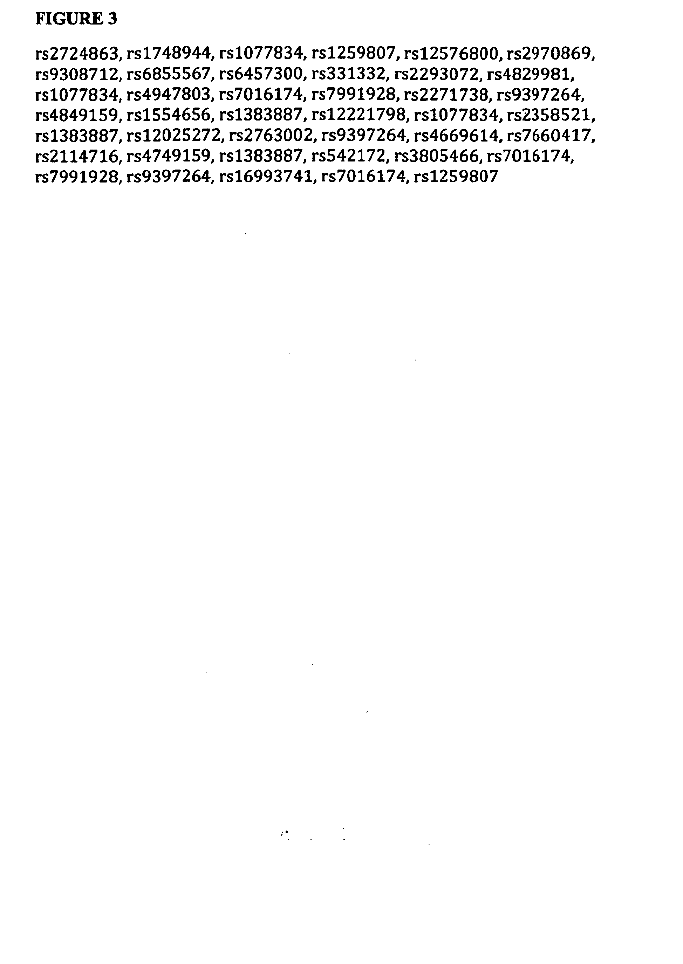

A number of ancestry informative markers are identified in Figure 3. Others also are described in US 2007/0037182 (Gaskin et al.).

Ancestry informative markers can be discovered by determining the frequency of genetic variants in a plurality of populations. This may be achieved by determining the frequency of already known variants in individuals from various populations. It may also be achieved intrinsically during the process of variant discovery. Both tasks were undertaken by the International HapMap project, which catalogued SNP polymorphisms.

Ancestry informative markers can be ranked by a variety of measurements which judge their predictive power. One measurement is Wright's F-statistic, called Fst or FST. This variable is known by other names, including Fixation index. Another metric for ranking AIMs is informativeness. Another method of ranking AIMs is the PCA-correlated SNPs method of Paschou et al. (Paschou et al. PCA-correlated SNPs for structure identification in worldwide human populations. PLoS Genet (2007) vol. 3 (9) pp. 1672-86).

To achieve a pre-selected degree of confidence in ancestry inference on the basis of ancestry informative markers, and to achieve ancestry inference for a plurality of populations, it is typically necessary to examine more than one ancestry informative marker. A sufficiently large panel of randomly selected genetic variants can be used to infer ancestry. A targeted set of especially appropriate AIMs can be constructed. Many researchers have published lists of suggested ancestry informative markers (for example: Seldin et al. Application of ancestry informative markers to association studies in European Americans. PLoS Genet (2008) vol. 4 (1) pp. e5; Halder et al. A panel of ancestry informative markers for estimating individual biogeographical ancestry and admixture from four continents: utility and applications. Hum Mutat (2008) vol. 29 (5) pp. 648-58; Tian et al. Analysis and application of European genetic substructure using 300 K SNP information. PLoS Genet (2008) vol. 4 (1) pp. e4; Price et al. Discerning the ancestry of European Americans in genetic association studies. PLoS Genet (2008) vol. 4 (1) pp. e236; Paschou et al. PCA-correlated SNPs for structure identification in worldwide human populations. PLoS Genet (2007) vol. 3 (9) pp. 1672-86; and Bauchet et al. Measuring European population stratification with microarray genotype data. Am J Hum Genet (2007) vol. 80 (5) pp. 948-56.). These and similar lists can be used to build a panel of AIMs for which a device or method herein can be configured to test for.

Some AIMs are also causal genetic variants. For example, the Duffy Null (FY*0) genetic variant causes an absence of a blood antigen. This variant is at nearly 100% frequency in sub-Saharan African populations and at nearly 0% frequency in populations outside of sub-Saharan Africa. Many causal genetic variants associated with pigmentation are also AIMs. Conversely, AIMs that are not causal genetic variants can be indirectly associated with traits caused by other AIMs. When selecting a set of causal genetic variants and ancestry informative markers, it is useful for the AIMs to be different than the causal genetic variants.

## II. SYSTEMS

In an aspect, a computer readable medium comprises logic that predicts a probability of a phenotype of a child from a set of parents or potential parents with respect to at least one rare genetic disease based at least in part on the presence or absence of a plurality of causal genetic variants in a first parent, the presence or absence of a plurality of causal genetic variants in a second parent, at least one AIM from the first parent and at least one AIM from the second parent. In another aspect, a computer readable medium comprises: logic for performing a fully probabilistic analysis on data corresponding to a plurality of causal genetic variants from a male and a female to predict a probability of a phenotype of a child, wherein the male and the female are potential parents of the child. In some instances, performing a fully probabilistic analysis comprises carrying statistical noise throughout the entire process. In some instances, the causal genetic variants from each of the male and female comprise one or more ancestry informative markers (AIMs), one or more causal genetic variants corresponding to a rare genetic disease, one or more causal genetic variants corresponding to a personality trait, or both. In some instances, the computer readable medium further comprises logic for receiving input from a questionnaire completed by said male and/or female and assigning a weighting function to the plurality of causal genetic variants based on said input. In yet another instance, the information from the questionnaire comprises one or more of the following types of information regarding the male or female: height, weight, and family disease history. The computer readable medium can provide an output in the form of a report detailing the probability of the phenotype of the child. Logic can be computer readable instructions as described herein.

In an aspect, computer readable instructions for predicting a phenotype of a child are provided that when executed: perform a fully probabilistic analysis on data from a male and a female corresponding to a plurality of causal genetic variants to predict a probability distribution over child phenotypes, wherein the male and the female are potential parents of the child. In some instances, a fully probabilistic analysis is an analysis that does not make a determination based on a probability at one step, but rather carries the probability throughout the entire analysis. For example, if genotype AA has a probability of 91 % and genotype AT has a probability of 9% then when calculating child risk in subsequent steps, the probabilities of 91% and 9% are carried through to that calculation step, instead of using the most probably genotype AA. In some instances, this can be an important model for alleles that have low probability but very high relative risk. The variants from each of the male and female can comprise one or more ancestry informative markers (AIMs), one or more causal genetic variants corresponding to a rare genetic disease, or both. The instructions can receive input from information from a phenotype battery from said male and female and assign a weighting function to the plurality of causal genetic variants to predict the probability of the phenotype of the child. The phenotype battery can be collected by a variety of items or means, including without limitation: questionnaires, surveys, tests (such as IQ tests), medical records, and games. The information from the phenotype battery used when executed by the instructions can be height, weight, and family disease history. Elements of the phenotype battery can include, but is not limited to: age at first menopause, agreeableness, airway histamine responsiveness, alcohol dependence, birth weight, blood pressure, body mass index, cannabis dependence, chronic pelvic pain, coffee consumption, complete blood count, conscientiousness, DSM-IV major depressive disorder, depth of sleep, endometriosis, exercise participation, extraversion, fainting, family history of disease, fatigue, finger ridge count, fitness, forced expiratory volume, harm avoidance, hay fever, height, Immunoglobulin E levels, incisor geometry, individual alpha frequency (EEG), inspection time, insulin concentration, IQ, liability to appendectomy, liability to asthma, liability to tonsillectomy, male baldness, mole size and count, mouth ulcers, neuroticism, novelty seeking, openness, osteoarthritis in women, parietal P300 latency, parietal slow wave amplitude, performance IQ, persistence, prefrontal P300 latency, prefrontal slow wave amplitude, premature parturition for any birth, presence of eating disorder, psychosis proneness, quality of sleep, reward dependence, smoking initiation, stuttering, susceptibility to migraine, tea consumption, total cholesterol, triglycerides, weight, verbal IQ, voting behavior, and VO2max. The instructions can provide an output in the form of a report detailing the probability of the phenotype of the child.

In an aspect, a system for predicting a child phenotype comprises: at least two a nucleic acid detection devices configured to detect a plurality of causal genetic variants corresponding to at least one rare genetic disease and at least one ancestry informative marker (AIM), wherein a first device is in contact with a sample from a female and wherein a second device is in contact with a sample from a male, wherein the male and the female are parents or potential parents of a child; a reader configured to read data from the devices; and computer readable instructions, wherein the instructions when executed utilize the data from the reader corresponding to the plurality of causal genetic variants and the at least one ancestry informative marker to predict a probability of a phenotype of the child with respect to the at least one rare genetic disease. The at least two nucleic acid detection devices can comprise a plurality of nucleic acid probes that selectively bind to the plurality of causal genetic variants and the at least one AIM.

In an aspect, a system for predicting a child phenotype comprises: at least two a nucleic acid detection devices configured to detect a plurality of causal genetic variants corresponding to more than 85 rare genetic diseases, wherein a first device is in contact with a sample from a female and wherein a second device is in contact with a sample from a male, wherein the male and the female are parents or potential parents of a child; a reader configured to read data from the devices; and computer readable instructions, wherein the instructions when executed utilize the data from the reader corresponding to the plurality of causal genetic variants to predict a probability of a phenotype of the child with respect to the more than 85 rare genetic diseases. The at least two nucleic acid detection devices can comprise a plurality of nucleic acid probes that selectively bind to the plurality of causal genetic variants corresponding to more than 85 rare genetic diseases. The at least two nucleic acid detection devices can further comprise a plurality of nucleic acid probes that selectively bind to at least one ancestry informative marker (AIM). The computer readable instructions when executed can utilize the data from the reader corresponding to the at least one AIM to predict the probability of a phenotype of the child.

In an aspect, a system for indicating if a subject is a carrier of a rare genetic disease comprises: a reader configured to read data from a nucleic acid detection device configured to detect a plurality of causal genetic variants corresponding to at least one rare genetic disease and at least one ancestry informative marker (AIM); and computer readable instructions, wherein the instructions when executed utilize the data from the reader corresponding to the plurality of causal genetic variants and the at least one ancestry informative marker to predict a plurality of probabilities of the subject being a carrier for each of the plurality of causal genetic variants.

## A. Genotyping Individuals

There are many different ways of acquiring information about an individual's genetic sequence. Discussed herein are exemplary methods for analyzing genotype data produced by SNP chips. Some of the methods relate directly to solving some of the issues with identifying causal genetic variants for rare genetic diseases, for example: minimizing the false negative rate (for example, the probability that the test comes back negative when the couple is at risk for having a child with the disease) while keeping the false positive rate below a specified threshold. However, the same concepts can be generalized to other methods for producing sequence data (such as resequencing by sequence capture and sequencing-by-synthesis) or for ascertaining sequence information indirectly (for example by making measurements on RNA or protein proxies).

Genotype calling for biallelic SNPs is traditionally done by clustering. Given alleles X and Y, a scatterplot of the normalized signals for the X and Y allele can be created. The XX, XY, and YY genotypes manifest themselves as distinct clusters of points in this scatterplot. Other types of variants such as insertions, deletions, and triallelic SNPs can be accommodated by using multiple biallelic probes. Genotype calling can proceed by clustering in a higher dimensional space, in which more than two axes are present.

A false negative call can correspond to informing a couple that they are not at risk when in reality they are. This is a failure of a screening test and may result in a child with a disease. In cost terms, the standard measure for the price of treating a child with a rare Mendelian disorder is approximately $1 million.

Suppose that the wild type allele is X and the disease allele is Y at a given locus. At this locus, most couples will be XX (normal) or XY (carriers), though incomplete penetrance/expressivity may lead to a few YY individuals. The determination of an individual's ancestral group from AIMs and self-reported ethnicity (as described in claim B below) provides prior probabilities for the XX, XY, and YY genotype frequencies at the disease locus. In many embodiments, ancestry determination is important because many Mendelian disease loci are much more frequent in one population than another.

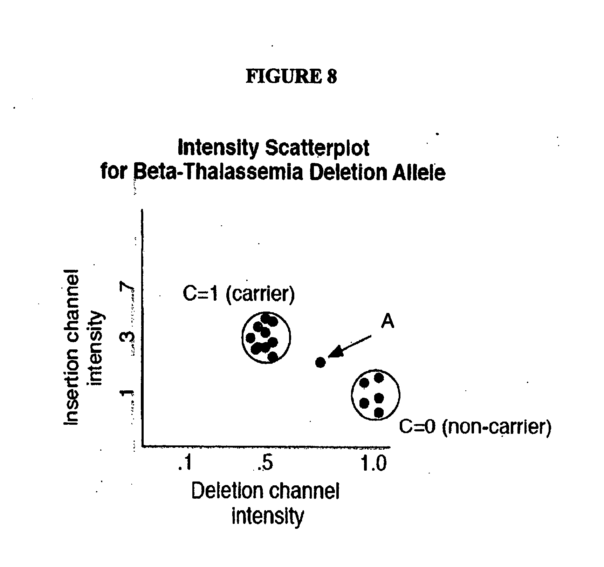

Most genotype calling software uses the prior probabilities for genotype frequencies as the cluster priors, as the calling algorithm is optimized to minimize misclassification probability. For example, for a biallelic SNP, if A is the ancestral group which is being assayed, MX is the measured normalized signal in the X channel and MY is the measured normalized signal in the Y channel, the clustering procedure for genotyping calling is equivalent to estimating $P(G|MX,MY,A)$. The optimal way to do this is with Bayes' rule as shown in Figures 8-12.

In many instances, the devices and systems herein can be designed err on the side of identifying people as carriers or no call if there is any doubt about them being wild type. Geometrically, what this corresponds to are looser cluster boundaries where we are more aggressive in calling XY than baseline carrier probabilities would suggest. This may be desirable because false positives may only require followup tests, while false negatives may represent a failure of a screening test.

Mathematically, the exact geometry is determined by the choosing the boundary which minimizes a measure of cost (for example expected or median cost) rather than the one which minimizes misclassification probability. Computationally, this may require specialized software (such as the computer readable instructions provided herein) to process the raw chip readout.

In order to attain a desired false positive rate of 1/100 or less, and false negative rate of 1/10000 or less for a multiplexed assay, the following protocol can be carried out: a) infer ancestry to obtain population specific cluster parameters as shown in Figure s4-7, b) assay each variant several times on the same chip, and c) use a control sample with each batch of chips to confirm manufacturing validity.

The use of repeated measurements allows these false positive and negative rates to be achieved. As an example, consider an assay for a variant with an average accuracy of 99%, where the accuracy is the probability that the actual genotype is the same as the called genotype. With symmetric error (for example, with default genotype calling) this corresponds to a false positive and false negative rate of 1%. If C is the called genotype and G the actual genotype, then formally we have: the false positive rate: P(C=XX|=XY) = 0.01, and the false negative rate: P(C=XY|G=XX) = 0.01.

If three of these assays are used in parallel and have independent failure rates, a combined best-of-three vote for genotype calling can achieve much better false positive and negative rates. Suppose that C1, C2, C3 are the genotype calls of these three assays. Then the probability that all three assays produce false negatives at the same time is:

$$P(C1=XX,C2=XX,C3=XX|G=XY) = P(C1=XX|G=XY)P(C2=XX|G=XY)P(C3=XX|G=XY) =$$

$$(.01)^3 = 1 * 10^{-6}.$$

This is now one in one million, which is much less than one in 100, and achieves the false negative rates necessary for a feasible multiplexed assay.

It is possible to achieve approximately independent failure rates by locating probes in different regions of the chip or by using multiple chips or assay types. However, the assumption of independent failure can be compromised if there is systematic error, either in the design of unreliable probes or the presence of samples with extremely low DNA levels.

In practice, probe reliability can be guaranteed by using a funnel approach, by starting with many redundant probes and triaging those probes which perform poorly in a representative training set. Specifically, a combination of random samples, engineered plasmids, and samples from DNA repositories like Coriell may be used to empirically determine sensitivity and specificity for each variant of the assay. Variants with poor calling statistics are not used for risk calculation. A control sample or samples may also be prepared and run alongside customer DNA to ensure that each batch of assays/chips has been manufactured properly.

## B. Inferring Ancestry

Given a set of measurements of N ancestrally informative markers $X\_1$ through $X\_N$, we can infer membership in a given ancestry group through standard classification techniques. For example, we can use expectation-maximization based clustering or similar algorithms to achieve probabilistic classification into categories, using assayed variants as features and self-reported ancestry as training data. Often individuals who have four grandparents hailing from the same geographic area or ethnic group are preferred as training points. Those whose grandparents hail from different regions or ethnic groups are then considered admixed. This analysis can be naturally extended to the case where the ancestral categories are hierarchical and/or overlapping. For example, it is possible to define multiple types of ancestry: continental scale (European, Asian, African), national (English, French, German), linguistic (French Canadian), and so on. Different classifiers employing different combinations of ancestrally informative markers can be used to distinguish between virtually any such collection of categories.

For the purposes herein, the categories which are most relevant are those that correspond to populations with significantly increased rates of endogamy or frequencies of particular disease causing alleles. AIMs to identify all of these populations can be included on the chip.

AIMs in general are useful because ancestral self-report, while a useful starting point, can be misleading or incomplete. In North America, for example, many Caucasians do not know their admixture proportions. Given that populations like Italians and English differ in their frequencies for disease alleles like the Mediterranean variant for beta thalassemia (higher in Italians: http://www.genome.gov/10001221), AIMs can provide significant and useful information beyond that provided by self report. In particular, for admixed individuals it is also possible to assign ancestry on a more granular basis to particular regions of DNA (http://www.genome.org/cgi/content/abstract/gr.072850.107).

Ultimately, AIMs are useful for the muliplexed chip for uncommon Mendelian traits because knowledge of ancestry provides a prior distribution over genotype frequencies, thereby reducing the misclassification probability. For example, someone who is not Ashkenazi Jewish is unlikely to be a carrier for familial dysautonomia (http://en.wikipedia.org/wiki/Familial_dysautonomia), and the extra information on ancestry provides an independent check in addition to the direct results of the assay.

## C. Determining Genetic Risk

Risk calculation in the context of an assay for rare genetic diseases can be that the false positive and false negative rates be held to within acceptable parameters. The discussion on genotyping above is the primary means of achieving this. Given reliable genotyping strategies with false positive rates on the order of 1% or less and false negative rates on the order of 0.001% or less, standard risk calculation methods can be applied to determine the risk that a couple will have a child with a given genetic disease. Application of such methods requires at a minimum knowledge of the mode of inheritance of the disease. For many traits, risk calculations may involve more complex assessments involving the possibility of incomplete penetrance or expressivity, de novo or recurrent mutations, genetic anticipation, and similar complicating factors.

Survey questions, including an online family history, may be important for augmenting risk calculations in the event that an assay result alone is not dispositive. However, for many of the Mendelian diseases assayed on the chip, a traditional consultation may not provide much extra information because carriers are often asymptomatic and most carriers do not have immediate family members with a rare Mendelian disease. Any positive result from a device herein may be subject to a specialized followup test in the event that the false positive rate is non-negligible.

## D. Predicting Phenotype from Parental Genotype and Phenotype

The devices and systems herein provide genotype data that can be used to forecast the risk of having a child with a given disease. Conceptually, this forecasting is possible because of the predictable mode of inheritance of a Mendelian trait and the generally high correlation between having a variant and having the condition, notwithstanding the issues involved with variable expressivity and penetrance. For complex traits, it is still difficult to predict their values from genotype measurements alone. It may be possible to do this in the future by measuring CNVs, from resequencing data, or by combining multiple assay modalities (for example methylation, expression, etcetera), but the results from SNP chips alone have not explained much of the variance. For example, a recent whole genome association study of IQ found that only 1% of the variance in IQ could be predicted by a measurement of 500000 SNPs (http://www.blackwell-synergy.com/doi/abs/10.1111/j.1601-183X.2007.00368.x). Similarly low values have been found for other traits like height, where large studies involving 30000 plus individuals have found SNPs explaining only 3.7% of the variance in height.

Importantly, while it is difficult to predict the values of an individual's complex traits from genotype information, it is often easy to predict the values of a child's complex traits from phenotype information on the parents. That is, for phenotypes with high heritability, a measurement of mother and father trait values is sufficient to provide excellent predictive power for child traits. For example, the narrow sense heritability of height is on the order of 0.70 to 0.80, depending on the population and environmental background (http://www.sciam.com/article.cfm?id=how-much-of-human-height). This means that the proportion of child variance explained by two measurements (the mother height and father height) ranges from 49% to 64%, a number which is far in excess of what has been achieved with SNP based predictions of individual height as of May 2008. It is thus possible to exploit this phenomenon for the purposes of child prediction. For traits with nonzero heritabilities, child trait values can be forecast from parental measurements with low error rates.

A survey of parental and child risks or phenotypes from a random sample of 1000 people from a target population may be used to provide empirical training data for regression equations relating parental and child characteristics. AIMs may also be collected to facilitate identification of members of this population. It is also possible to include data from other family members, such as grandparents, uncles, and aunts, as additional predictive features for predicting child traits. These regression equations may be more sophisticated than the simple linear models employed in quantitative genetics. Any kind of regression algorithm may be employed to predict child phenotype from the measurements of parental phenotypes. For example, this is of demonstrable utility in the case of longevity, which has a highly nonlinear inheritance pattern (http://longevity-science.org/Genetics-Australia.html). Importantly, the nonlinearity of longevity inheritance results in a deceptively low heritability coefficient of 0.10.

Standard heritability estimates may thus underestimate the ability to predict offspring traits from parental values, indicating that it may be useful to assay any trait of potential commercial relevance on a large number of parent/child trios from a given population and determine empirically whether prediction is feasible or infeasible. As before, it may be useful to assay other relatives besides immediate parents. It may also be useful to return confidence intervals or quantiles in addition to point estimates. The regression equations for predicting complex child risks or phenotypes from parental phenotype and the risk calculation algorithms for predicting child disease risk from parental genotype form a unified suite of algorithms for child prediction. From an input/output perspective, parents provide survey data and saliva samples, and algorithms return child predictions of both Mendelian disease risks and complex traits.

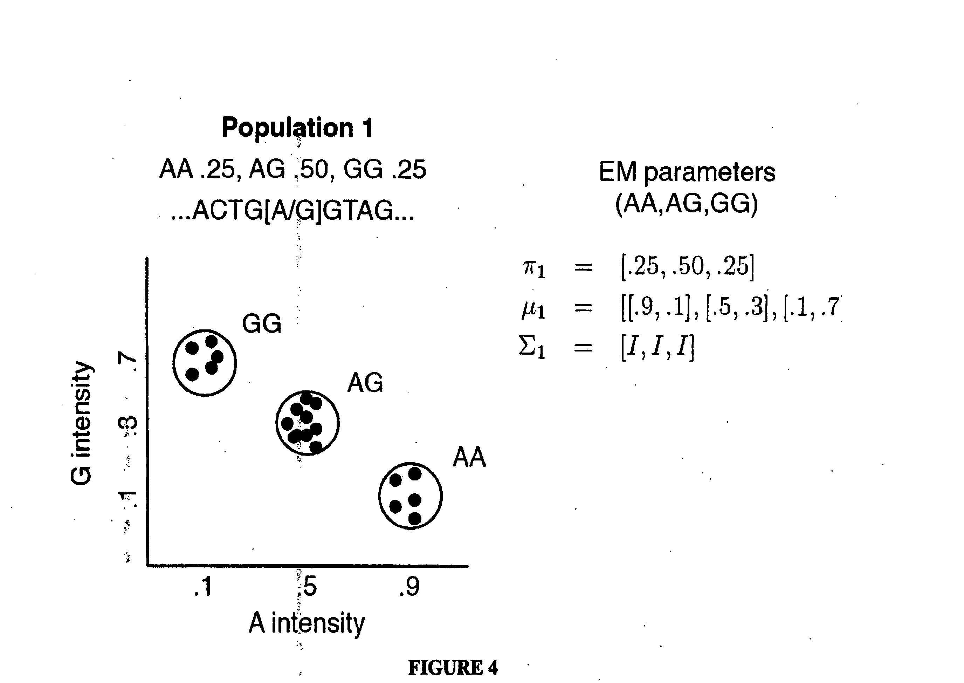

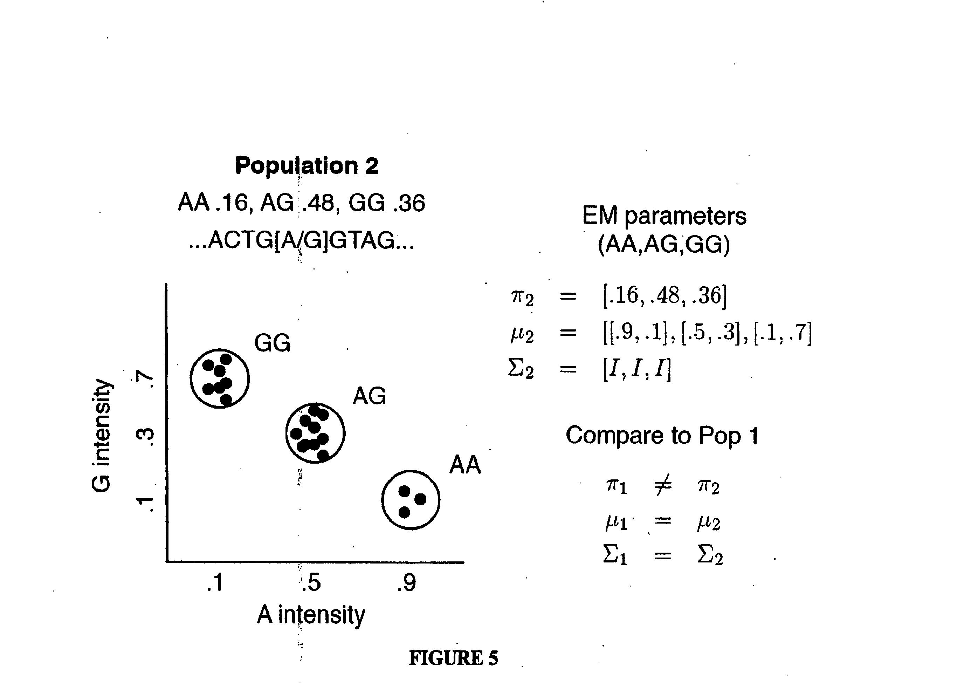

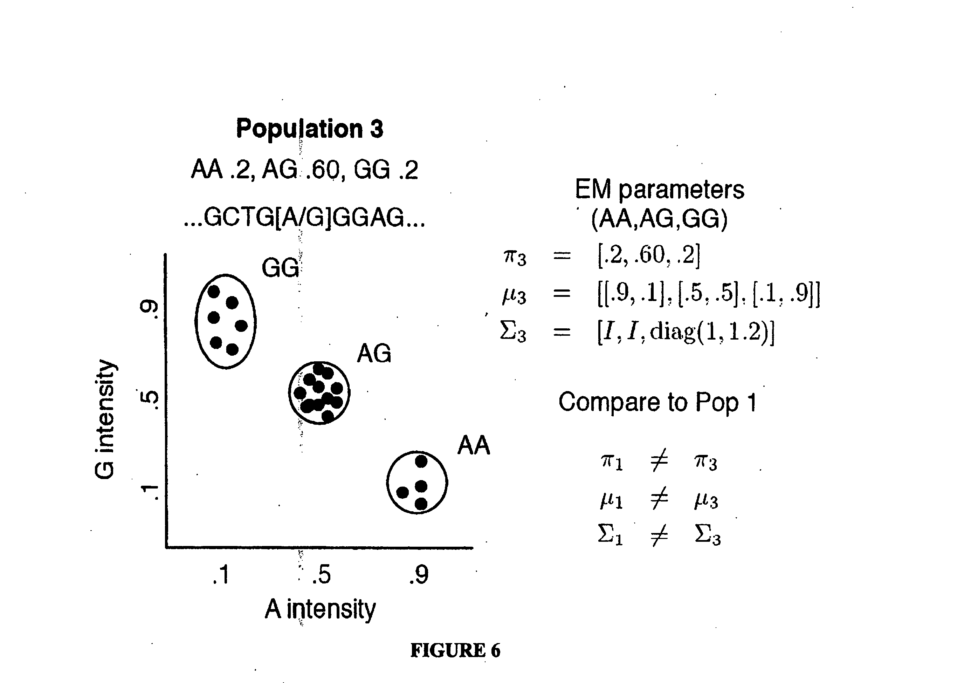

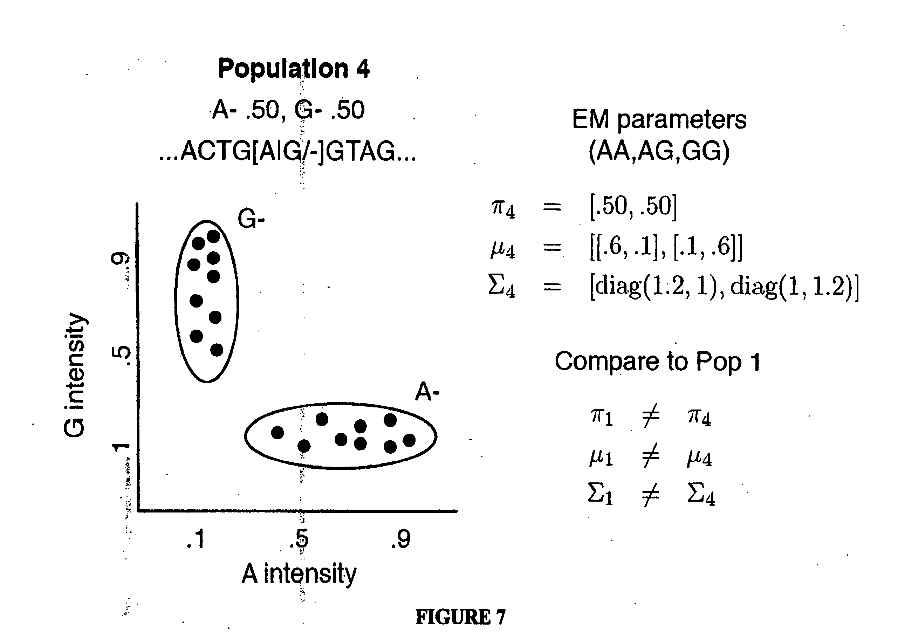

As described herein, population information can determine cluster parameters. Figures 4-7 illustrate a theoretical basis by which population information (as revealed by AIMs and/or self-report) can inform cluster parameters and genotype calling, and thereby influence the accuracy of carrier testing and child phenotype prediction. Population 1 is a group which has a hypothetical A/G SNP with the given flanking sequence in Figure 4. If 1000 individuals from this population are genotyped, 1000 measurements are obtained of the normalized intensities of the A and G channel probes at that SNP. These intensity values can be depicted in a scatterplot as shown, where each point represents an individual and the X and Y axes correspond to the A and G channels. In this example, three clusters arise, corresponding to the AA, AG, and GG genotypes. Parametric models of various kinds can be fit to these clusters. One of the most popular is the expectation maximization (EM) model, in which a multivariate gaussian distribution with parameters mu (the mean vector) and sigma (the covariance matrix) is fit to each cluster. In this example, there are three mu vectors of dimension 2, and three sigma matrices of dimension 2 x 2 (in this case the identity matrices). The relative area under each multivariate gaussian is given by the pi parameter, corresponding to the proportion of points which lie in that cluster. In Figure 5, population 2 has exactly the same variant witch the same flanking sequence, but different proportions of each genotype (0.16, 0.48, 0.36 rather than 0.25, 0.50, 0.25). Because the molecular biology of the variant and the flanking sequence is the same, the clusters are in the same position and only pi differs. In Figure 6, population 3 has a different flanking sequence with an extra G. The molecular biology differences in the flanking sequence can cause shifts in the positions and orientations of the clusters, for example by translating clusters upwards on the G-axis or introducing more variance along the G axis. In such a case, the mu and sigma parameters may also differ along with the pi parameters. The previous Figures 4-6 have been considering populations in terms of geographic ancestry, however Figure 7 considers populations by gender. Specifically, suppose that population 4 is composed of males, that the locus under consideration is on the X-chromosome, and that all members of previous populations were females. In this case the intensity plot for males would look quite different. In particular, only two clusters are possible (A/- and G-/) rather than three (AA, AG, GG). Thus the cluster parameters mu, sigma, and pi are different from that of population 1. These examples illustrate the importance of taking population information (for example as informed by gender or ethnicity) into account when training calling models.

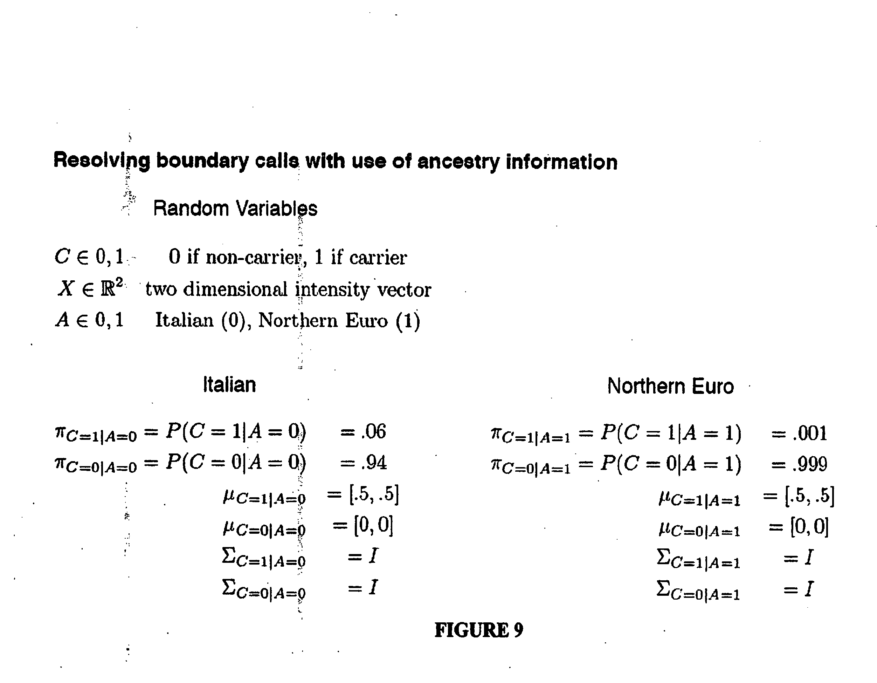

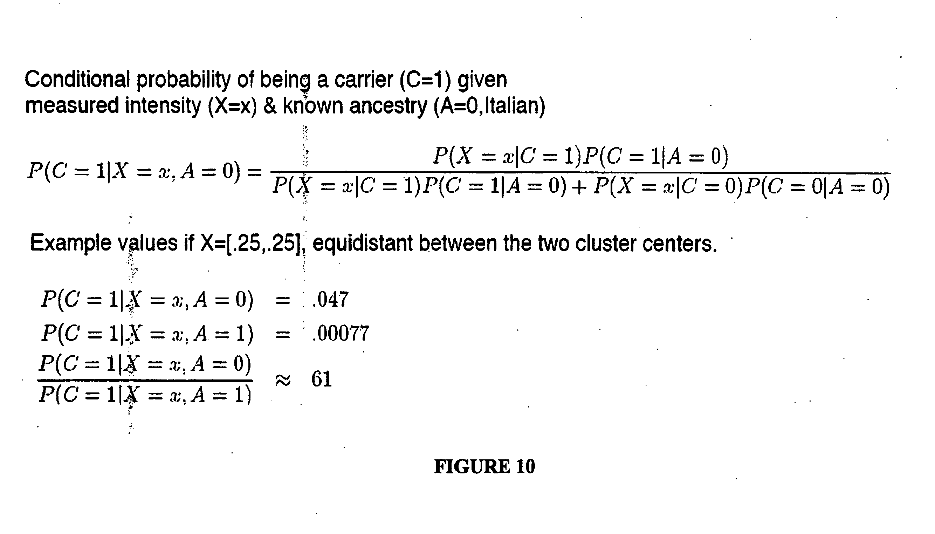

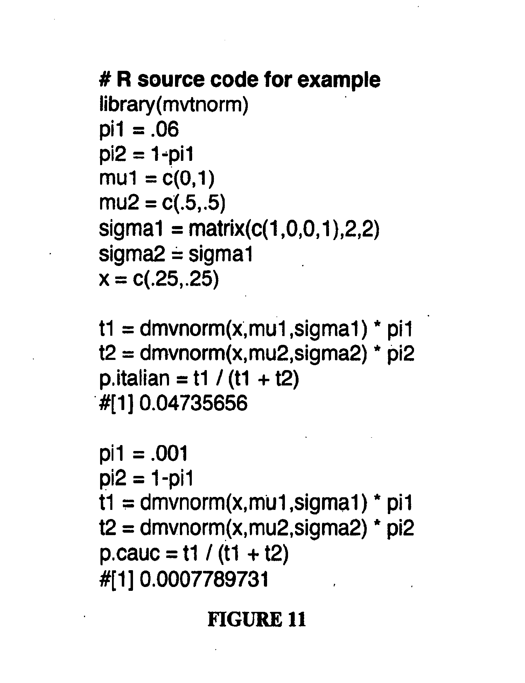

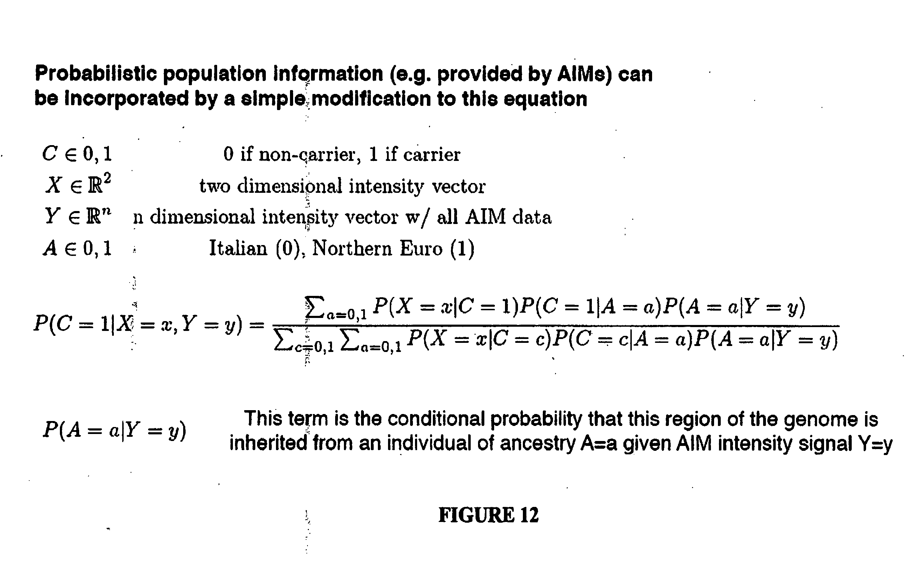

In many instances, population information aids genotype calling. Figures 8-12 demonstrate an example of how population information influences genotype calling and risk calculation in the context of beta-thalassemia, which sharply differs in frequency between two phenotypically similar populations: Italians and Caucasians from the general population. The carrier frequency for the deleterious beta-thalassemia variant differs starkly between Northern European and Italian populations, who are otherwise phenotypically similar and highly admixed in countries like the United States. For example, the carrier frequency in Northern Europeans is less than 1 per 1000, whereas the carrier frequency in Italians is greater than 1 per 100. The plot in Figure 8 shows an intensity scatterplot for the beta thalassemia deletion allele similar to the scatterplots in Figures 4-7. For simplicity only two clusters are shown, corresponding to the wildtype and carrier genotypes and excluding the homozygote. A key question is how to classify points with intensities that are not extremely close to the means of their respective clusters, such as 'A' in Figure 8. In Figure 9, it is shown that calling of points near the boundary is aided by the use of ancestry information. The numerical example in Figure 10 shows that a raw intensity measurement at a causal locus is not the only value that matters. Coupling this measurement (X) with knowledge of ancestry (A) can change the probability of being a carrier by almost 60 fold. A source code example is provided in Figure 11. In practice one will not have complete knowledge of ancestry. AIMs help in this situation. One may use signal from AIMs to produce a distribution over populations, obtaining the probability that a given locus is inherited from an ancestor of a given population group. As shown in Figure 12, this requires a straightforward modification to the expression for the posterior probability of whether an individual is in a given cluster. Instead of assuming that the value of the ancestry parameter is known with probability 1, a distribution can be used over ancestries. This technique of using AIMs to improve calling at causal loci provides a substantial gain in classification power and is particularly useful for assays such as those described herein, as it is indicated for many different ancestry groups.

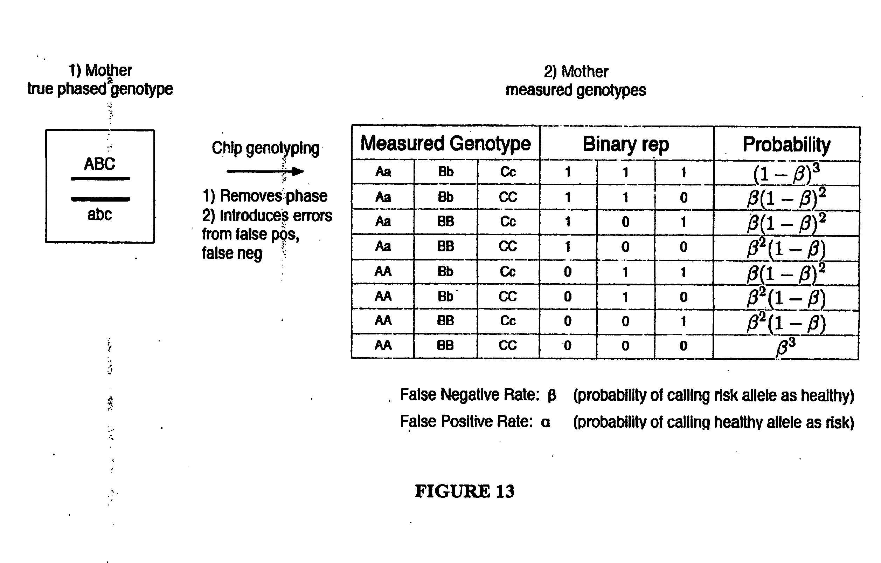

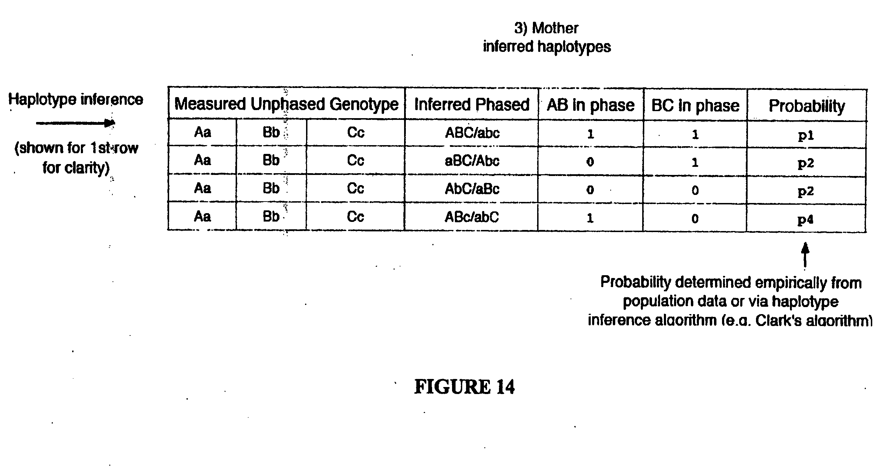

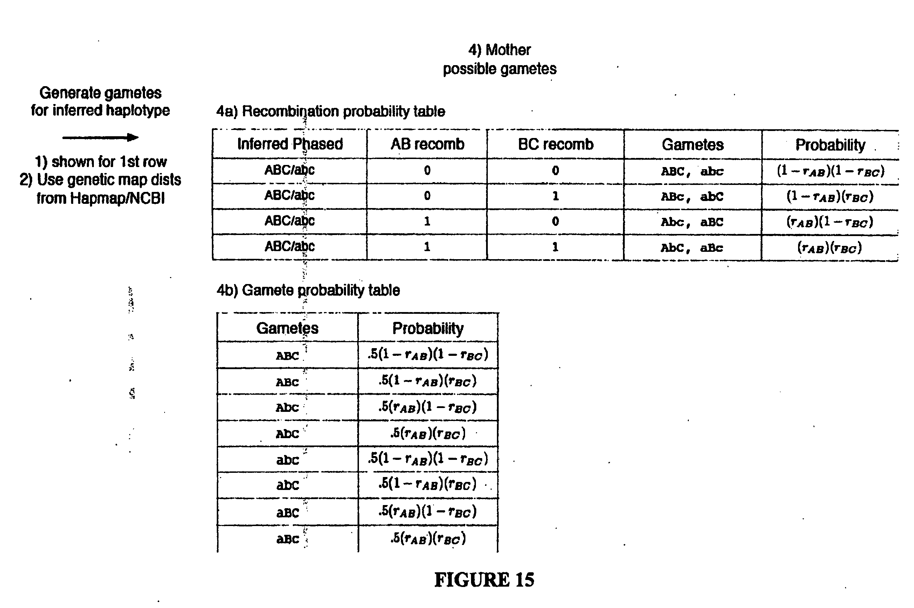

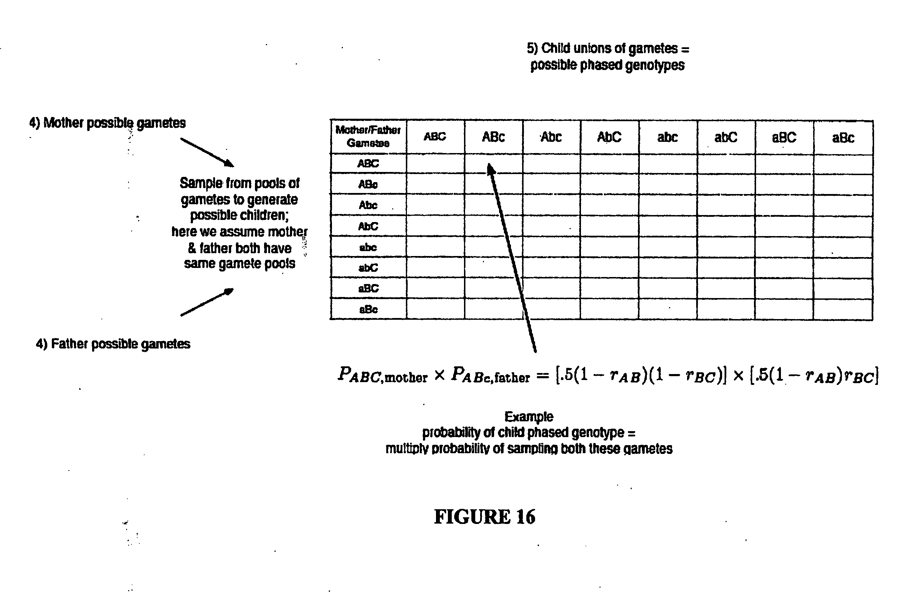

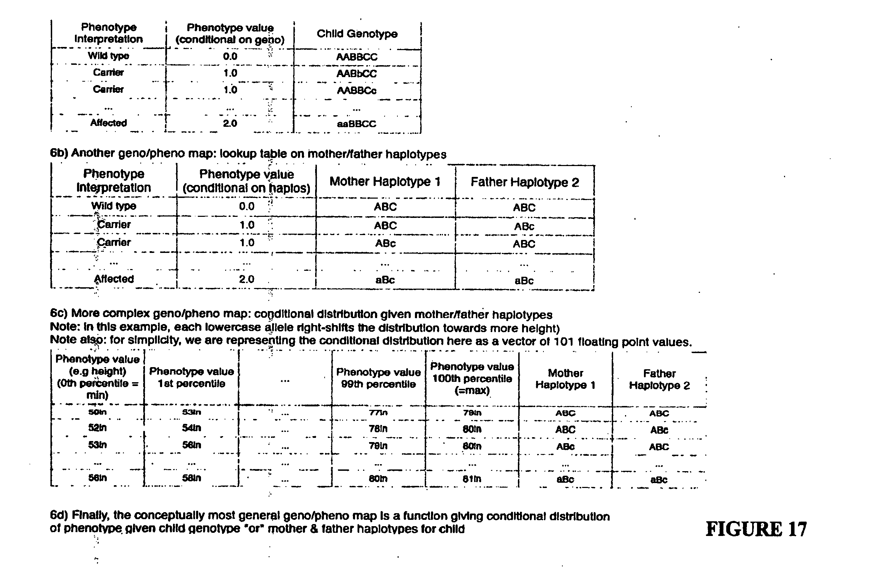

There are several possible sources of noise that should be accounted for when performing child phenotype predictions using genotype information. Figures 13-18 illustrate the process of fully probabilistic prediction of a distribution over child risks and phenotypes. The process accounts for several possible sources of uncertainty, including genotype calling error, phasing uncertainty, recombination, gametic union, and a noisy genotype-to-phenotype map. First, as shown in Figure 13, a measured genotype may have measurement error as represented by a false positive and false negative rate, but more generally one would specify a confusion matrix that gave the probability of predicting genotype i given a true genotype j. Given a measured genotype, the result is a distribution over possible true genotypes. Next, as shown in Figure 14, the inferred genotype is unphased. That is, adjacent heterozygote loci may be in either cis or trans and this is unknown. One can model phase uncertainty by either: using an appropriate database of population haplotype frequencies at the given locus; or using one of several haplotype inference algorithms, such as Clark's algorithm or model based haplotype inference. Figure 15 shows how to generate a distribution over possible recombinants and hence possible gametes from the possible haplotypes. Figure 16 then shows how to repeat this process for both mother and father to obtain a probability distribution over gametic unions, corresponding to phased child genotypes (aka haplotypes). This results in a distribution over child haplotypes. Finally, Figure 17 reviews several different kinds of genotype-to-phenotype maps that can be used with the distribution over child haplotypes to produce the desired quantity: a distribution over possible child risks or phenotypes.

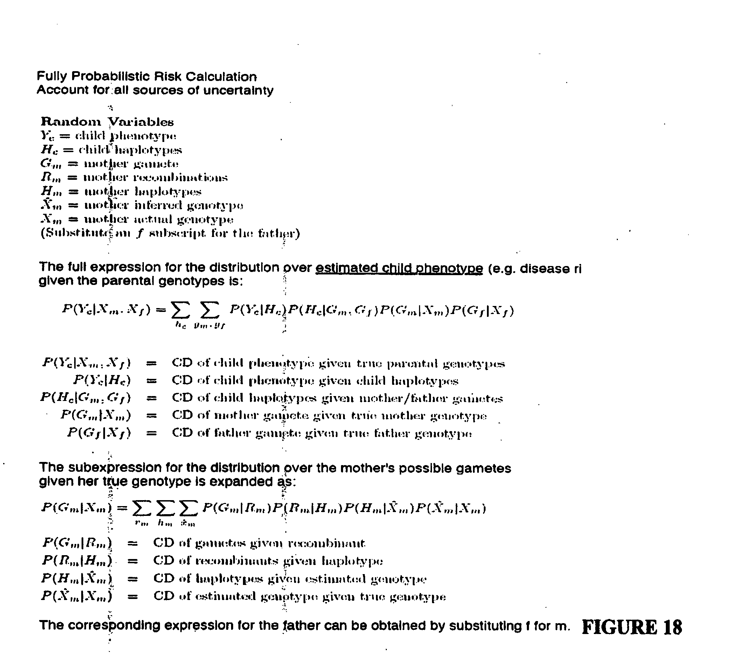

Figure 18 depicts equations for obtaining the distribution over estimated child risks or phenotypes given parental genotypes, formalizing the flow diagrams of Figures 13-17. This expression is useful in that it is exact and can be approximated for any given locus. This can be done via simulation or by noting that certain steps collapse; for example, predicting child phenotype based on a single locus trait need not deal with recombination. It is notable that each conditional distribution can be expressed as a matrix and the series of sums can be expressed in terms of repeated matrix multiplications, a formulation which allows rapid computer implementation.

In the future, as resequencing costs drop, it is important to note that parent phenotypes and genotypes determined in this manner can then be regressed upon each other. That is, the voluntary large scale provision of parental genotype and phenotype information for the purposes of predicting child traits can be used as a source of data for extremely large scale association studies.

### E. Biological Relatives Genomics

The methods herein can be utilized for family genomics. In some aspects, the methods can be utilized to determine probabilities of phenotypes or probabilities that family member has or had a rare genetic disease based upon genetic screening of other family members. For example, a subset of biological relatives can be screened as carriers for a plurality of causal genetic variants of at least one rare genetic disease. The data from the subset can then be utilized to predict the probability of phenotype of another biological relative that was not a member of the subset tested. For example, three children can be tested and the probability that a parent, living or deceased, has a particular phenotype or rare genetic disease as described herein can be calculated. These methods may be useful for postmortem determination of relative that may have had an unclear diagnosis of a disease at the time of death or did not have the tests available to him to determine if he had a rare genetic disease.

In some instances, the devices and systems as described herein that can be used to predict child phenotype probabilities can be utilized to predict probabilities of living relatives having certain traits or diseases or phenotypes. Relatives can include grandparents, great grandparents, aunts, uncles, mothers, fathers, siblings, children, and any other biological family member.

In some instances, a relative who was not included in the subset of biological relatives that were genotyped with regards to causal genetic variants can be referred to a genetic counselor and/or physician. In an instance, a relative who is a member or not member of the subset of biological relatives can pursue a course of medical action based on the results of the tests. For example, aspirin can be prescribed for heart disease to a family member based on risk calculated by a method or system herein. Information can also be collected from a phenotype battery, such as that discussed herein, for some or all of the subset of biological relatives and utilized in the calculations of the probabilities for other relatives. The risk of developing a disease for any biological relatives can be calculated and adjusted based upon family genomics, such as described herein. The results can be delivered to the relative and actionable methods can be carried out. For example, a genomic family tree can be created and utilized to factor into a probability of risk of disease for one, some, or all members of their family tree.

### III. DEVICES

Described herein is a universal carrier screen device and methods of utilizing the device. Also disclosed is computer software and code for translating data from the devices and methods to probabilities that a user is a carrier or that a set of parents have a probability of having a child with a genetic disease. A device herein can be configured to test for a plurality of rare genetic diseases or at least 5, 10, 85, 100, 200, 500, or 1000 of rare genetic diseases.

In an aspect, a nucleic acid detection device is configured to test a sample for a plurality of causal genetic variants corresponding more than 85 rare genetic diseases. The device can be further configured to test a sample for at least one ancestry informative marker (AIM). In some instances, the device comprises a plurality of nucleic acid probes that selectively bind to a plurality of causal genetic variants corresponding to more than 85 rare genetic diseases. The device can further comprise a plurality of nucleic acid probes that selectively bind to at least one ancestry informative marker (AIM). The device can comprise a bead array that selectively binds to a plurality of causal genetic variants corresponding to more than 85 rare genetic diseases. The device can further comprise a bead array that selectively binds to at least one ancestry informative marker (AIM). In some instances, the at least one AIM is not a causal genetic variant. In some instances, at least two of the rare genetic diseases occur at frequencies that differ by at least 10-fold in at least two distinct populations, wherein the at least two distinct populations are differentiated by the at least one AIM. In some instances, the device comprises a sequence capture assay to detect a plurality of causal genetic variants corresponding to more than 85 rare genetic diseases.

In some instances, the device simultaneously detects 85, 100, 150, 200, 500, 1000 or more rare causal genetic variants. In many instances, the causal genetic variants indicate a Mendelian disease in which a single gene is responsible for a disease. Often times, Mendelian diseases have a recessive inheritance pattern as demonstrated in Figures 4-7 and a person has two recessive alleles to develop the disease. Individuals with one mutant and one non-mutant allele can be described as carriers because they carry the causal genetic variant but do not have the disease. If parents are both carriers for the same disease, their children are at a risk for inheriting two copies of the mutant gene and thus developing the disease. For an exemplary Mendelian disease, one quarter of the children of two carriers will be expected to inherit the disease. Because the risk of having an affected child is a deterministic function of the genes of the parent, genetic tests are commonly used to preemptively identify parents who are carriers. These tests are considered screening tests because they are given to otherwise healthy individuals. If a couple is found to be at high risk for having a child with a Mendelian disease, in an example a genetic counselor or physician can help them consider their reproductive choices. Many Mendelian diseases that are understood at a molecular level are severe, resulting in early death or serious debilitation

A device herein can be a single test intended for panethnic population screening for a panel of Mendelian diseases.

Described herein is a device configured to test for causal genetic variants, for example, genetic variants that cause a particular phenotype. The device also can be configured to test for the presence of ancestry informative markers (AIMs), that is, genetic markers that correlate with populations, in particular human populations, coming from specific geographic locations. Causal genetic variants contrast with genetic markers in that certain markers may be highly correlated with the expression of a trait without actually causing the trait. In some instances, causal genetic variants can be selected wherein some populations (for example, populations that can be distinguished by AIMs) have different frequencies for traits or diseases caused by the causal genetic variants. Herein, a device can be configured to test for one of more AIMs and the AIMs can provide prior knowledge about the user of the device and to which population(s) to which he belongs. The prior knowledge of the population background of a user can then be utilized by the software and methods described herein to adjust the probability that the user is a carrier for a specific trait or a plurality of traits. For example, if a person tests positive for an allele that causes Tay Sachs disease and also tests positive for an AIM associated with Ashkenazi Jews, then the probability that the person is a carrier for the Tay Sachs gene can be calculated. On the other hand, if a person tests positive for an allele that causes Tay Sachs disease and tests negative for an AIM associated with Azhenazi Jews, then there may be reason to believe that the Tay Sachs results is a false positive and that can be reflected in the probability calculation as described herein.

In an example, two users (one male, one female) receive their probability of being a carrier for a plurality of causal genetic variants as described herein and the probability of the child of the two users developing traits from the causal genetic variants can be calculated using the methods and systems herein.

In some instances, a device comprises tests for causal genetic variants for traits for which different populations are at increased risk, as well as tests for AIMs for these populations. The traits can be Mendelian traits having low incidence in a population.

Devices herein are configured to test for genetic variants and/or ancestry informative markers. Many such devices for these purposes are known. They include, without limitation, nucleic acid arrays (including oligonucleotide arrays, spotted arrays, photolithographically generated arrays, random arrays and complete genomic hybridization arrays) and nucleic acid sequencers. These devices detect genetic variants or ancestry informative markers in a variety of ways. Such ways include sequencing by hybridization, sequencing by ligation, sequencing by extension, Sanger sequencing, Maxam Gilbert sequencing and pyrosequencing. These devices and methodologies are all well known in the art. Knowledge of the specific sequence or variant one wishes to detect can provide the information necessary to configure the device.

A device herein can be created from, for example, nucleic acid arrays (for example, Affymetrix DNA microarrays or Illumina bead arrays), single molecule sequence by synthesis methods (for example, Helicos BioSciences Corporation), amplification of nucleic acid molecules on a bead (for example, 454 Lifesciences), clonal single molecule arrays technology (for example, Solexa, Inc.), single base polymerization using enhanced nucleotide fluorescence (for example, Genovoxx GmbH) and single molecule nucleic acid arrays (for example, Drmanac, U.S. Patent Application No. 2007/0099208).

Methods and devices are described in many patents including for example, United States Patent 7368265 (Brenner et al. Selective genome amplification), United States Patent 7361468 (Liu et al. Methods for genotyping polymorphisms), United States Patent 7332277 (Dhallan, Methods for detection of genetic disorders), United States Patent 6576424 (Fodor, nucleic acid arrays) and United States Patent 7323305 (Leamon et al., Methods of amplifying and sequencing nucleic acids).

## A. Device Types and Methodology

With the exception of stable epigenetic modifications, genetic variants and markers at the level of DNA are typically detected as variations in the sequence in DNA nucleotides. The processing of detecting variations in the sequence in DNA nucleotides is also called genotyping. Genotyping of DNA in a sample is performed by contacting the sample with a device or devices.

It is possible to detect variations in the sequence in DNA nucleotides by randomly sequencing until such a point that sufficient coverage is achieved. Random sequencing is usually not truly random but pseudorandom, and may be biased. A number of technologies permit random sequencing. It is preferable for the sequencing technology to excel on a number of performance standards, including accuracy. A commercial sequencing technology can be used to facilitate meeting high performance standards. However, a non-commercial platform can be used as well.

In another embodiment the variants are tested by SNP analysis, complementary genomic hybridization, gene sequencing, sequence capture, DIP analysis, STR analysis, and CNV analysis. In another embodiment the sub-devices comprise a nucleic acid array, a bead arrays, a CGH array, a collection of molecular inversion probes, a collection of allele specific oligonucleotide (ASO) probes, pyrosequencing, Sanger sequencing, sequencing by synthesis and sequencing by hybridization. In another embodiment the causal genetic variant is a SNP (for example, missense, nonsense, splicing, or regulatory), DIP (for example, small deletion, small insertion, gross deletion, or gross insertion), CNV, repeat polymorphism, or complex rearrangements.

Sequencing can be performed by pyrosequencing. This technique is based on the detection of released pyrophosphate (Hyman, 1988. A new method of sequencing DNA. Anal Biochem. 174:423-36; and Ronaghi, 2001. Pyrosequencing sheds light on DNA sequencing. Genome Res. 11:3-11). Sequencing can be performed by sequencing by synthesis. Sequencing by synthesis is also called sequencing by incorporation (United States Patent 7344865). Sequencing can be performed by sequencing by ligation. The Applied Biosystems SOLiD sequencing system performs sequencing by ligation. Sequencing can be performed by Sanger sequencing. Sanger sequencing is also called dideoxy chain termination sequencing. Sequencing can be performed by single-molecule sequencing, which is a broad class of sequencing technologies in various stages of development.

Random sequencing can be an inefficient method of detecting particular variations in DNA sequence. To increase efficient coverage of particular variants, random sequencing can be augmented by a selective amplification of the regions of DNA that contain the variants of interest. A number of technologies permit selective amplification. It is preferable for the selective amplification technology to excel on a number of performance standards, including fidelity. A commercial selective amplification technology can be used to facilitate meeting high performance standards. With appropriate validation and optimization, a non-commercial technology can be used. The aim of selective amplification is to enrich for particular sequences within a complex mixture.

Selective amplification can be performed by polymerase chain reaction (PCR). PCR can be multiplexed. Multiples reactions can be performed in parallel. PCR requires optimization of reaction conditions, a standard laboratory practice.

Selective amplification can be performed by sequence capture. Sequence capture involves the use of hybridization to enrich for sequences of interest. Sequence capture can be performed using a microarray (Albert TJ, et al. Direct selection of human genomic loci by microarray hybridization. Nature Methods. 2007 Nov; 4(11):903-5; Okou DT, et al. Microarray-based genomic selection for high-throughput resequencing. Nature Methods 2007 Nov; 4(11):907-9; and Hodges E, et al. Genome-wide in situ exon capture for selective resequencing. Nature Genetics 2007 Dec; 39(12): 1522-7). For example, the NimbleGen Sequence Capture technology can be used for selective amplification. Selective amplification can be performed by rolling circle amplification. Selective amplification can be performed by cloning. Cloning can involve the use of a restriction endonuclease to cleave a complex mixture of DNA into fragments of predictable lengths.

It is also possible to detect particular variations in the sequence of DNA nucleotides by targeted genotyping. A number of technologies permit targeted genotyping. A number of technologies permit selective amplification. It is preferable for the selective amplification technology to excel on a number of performance standards, including fidelity. A commercial selective amplification technology can be used to facilitate meeting high performance standards. With appropriate validation and optimization, a non-commercial technology can be used. The efficiency of targeted genotype techniques can also be augmented selective amplification of the regions of DNA that contain the variants of interest, including PCR, sequence capture, rolling circle amplification, and cloning.

Targeted genotyping can be performed by sequencing by hybridization. Sequencing by hybridization can be performed using a microarray. Targeted genotyping can be performed by microarrays. An example of a technology that allows targeted genotyping by microarray is the Affymetrix Genome-Wide Human SNP Array 6.0. Targeted genotyping can be performed by bead arrays. An example of a technology that allows targeted genotyping by bead arrays is the Illumina Human1M BeadChip. Targeted genotyping can be performed by comparative genome hybridization (CGH) arrays. CGH arrays involve the co-hybridization of a reference sample and a target sample to the same array. Targeted genotyping can be performed by molecular inversion probes. An example of a technology that allows targeted genotyping by molecular inversion probes is the Affymetrix GeneChip Targeted Genotyping platform. Targeted genotyping can be performed by allele specific oligonucleotide (ASO) probes. The design of ASO probes permits a number of large number of permutations. ASO probes can be 15 to 21 nucleotides in length. ASO probes can contain zero, one, two, three, or more mismatches compared to the variants being detected. ASO probes can contain continuous stretches nucleotides at one end with zero or one mismatches to the variants being detected. Targeted genotyping can be performed by allele specific PCR. Targeted genotyping can be performed by TaqMan assays. Targeted genotyping can be performed by single base extension. Targeted genotyping can be performed by restriction length fragment polymorphism (RFLP) typing. Targeted genotyping can be performed by diagnostic PCR. A variety of diagnostic PCR techniques have been developed. In one example, PCR amplification of a region flanking a DIP will produce fragments of different sizes according to presence or absence of the DIP. In another example, PCR amplification of a region flanking a STR will produce fragments of different sizes according to which alleles of the STR are present.

To increase efficiency, a multiplex genotyping technique can be used. Most genotyping technologies intrinsically permit multiplex genotyping. Those technologies which do not intrinsically multiplex can be made to perform like multiplex genotyping by performing multiple singular genotyping procedures in parallel or serial. This fashion of genotyping may also be described as high-throughput.

The nucleotide occurrence of a SNP in a sample can be determined by a variety of technologies, including a number not explicitly listed here. CGH arrays are generally not suitable for SNP genotyping. The nucleotide occurrence of a DIP in a sample can be determined by a variety of technologies, depending on the size of the deletion/insertion. In general, small deletions/insertions, for example those less than 20 nucleotides in size, can be genotyped with technologies used to genotype SNPs. Larger DIPs may be treated as CNVs. The nucleotide occurrence of a CNV in a sample can be determined by a number of technologies. SNP genotyping technologies have been adapted for use in CNV genotyping. CGH arrays are also often suitable for CNV genotyping. The nucleotide occurrence of a STR in a sample can be determined by a number of technologies. STRs are most commonly genotyping by diagnostic PCR.

Stable epigenetic modifications, such as methylation, can be detected be detected by a number of technologies for example, as disclosed in United States Patent 7364855. For example, the Illumina GoldenGate Methylation Panel and the Illumina HumanMethylation27 BeadChip can be used to detect methylation differences. Bisulfite sequencing can be used to determine the presence of methylation. Methylation can be detected by pyrosequencing, methylation-sensitive single-strand conformation analysis (MS-SSCA), high-resolution melting analysis (HRM), methylation-sensitive single nucleotide primer extension (MS-SnuPE), base-specific cleavage/MADLI-TOF, methylation-specific PCR (MSP), and others.

A panel of genetic variants and markers could be genotyped by combining several different technologies into a single system.

Some genetic variants and markers can be detected at the level ofRNA polynucleotide. Reverse transcription polymerase chain reaction (RT-PCR) can be used in lieu of PCR or in addition to PCR to detect genetic variants at the level of RNA polynucleotides. Genetic variants can alter the sequence of an RNA polynucleotide. Detecting genetic variants that affect the sequence of an RNA polynucleotide is essentially equivalent to detecting genetic variants that affect the sequence of a DNA polynucleotide. Genetic variants can be detected as the presence of absence of an RNA polynucleotide, or more generally as the concentration of an RNA polynucleotide. A number of techniques can be used to determine the presence, absence, or concentration of an RNA polynucleotide. RT-PCR can be used to determine the presence, absence, or concentration of an RNA polynucleotide. Spotted microarrays, oligo microarrays, and bead arrays can be used to determine the presence, absence, or concentration of an RNA polynucleotide.

A device can be constructed that detects genetic variants and markers at both the level of DNA and the level of RNA.

Some genetic variants and markers can be detected at the level of protein polypeptides. Genetic variants can alter the sequence of a protein polypeptide. Genetic variants can be detected as the presence of absence of a protein polypeptide, or more generally as the concentration of a protein polypeptide.

### B. Causal Genetic Variants

The devices herein are configured to test for causal genetic variants and/or for ancestry informative markers. Detecting the presence of a variant or marker includes, by implication, detecting its absence. In some instances, the devices are configured to perform genotyping on the trait, not merely the presence of one polymorphic form of the variant or gene. The tests selected can include variants for a plurality of different at-risk populations; for example, at least 2 different populations, at least 5 different populations, at least 10 different populations, at least 25 different populations, at least 50 different populations or at least 100 different populations. For example, the device could be configured to test for one or more causal genetic variants for which the Western Eurasian population is at increased risk, one or more causal genetic variants for which the sub-Saharan African population is at increased risk, one or more causal genetic variants for which the East Asian population is at increased risk and one or more causal genetic variants for which the Native American population is at increased risk.

In an aspect, a device is disclosed that tests for a plurality (for example, at least 100, at least 500 at least 1000) of traits. Diseases occur in populations with different prevalence. Traits or diseases can be ranked according to prevalence, many will be grouped among rare traits, for example, traits having prevalence of 1/1000 or less or 1/10,000 or less. Such traits are said to inhabit the long tail, of such a ranking. The concept of a long tail is easily visualized by a bar chart in which the Y axis indicates prevalence and the traits are ordered along the X axis from most to least prevalent. In certain embodiments, the number and traits and the incidence of each trait can be chosen so that the collective incidence of the rare diseases tested for amount to at least 1%, at least 2%, at least 4%, or at least 5%.

In certain devices the causal genetic variants tested for have different incidence in different populations or sub-populations. The difference in incidence is at least statistically significant ($p < 0.05$) and may be increased by at least 10%, at least 100%, at least 5 times, at least 10 times, at least 100 times at least 1000 times or at least 10,000 in two different populations. A variant may cause a trait having the same incidence in two different populations, but it typically will have different incidence in at least two different populations represented on the device.

## C. Ancestry Informative Markers

Ancestry informative markers that a device tests for typically are selected with reference to the variants being tested for. In certain embodiments, the device tests for causal genetic variants that have different incidence in different populations or sub-populations. AIMs can distinguish between two populations for which one of the causal genetic variants exhibits a difference in incidence. The AIM also can classify a person as belonging to or not belonging to the population that is at increased risk for one of the causal genetic variants, for example, the AIM can be diagnostic for the population in which the trait is at increased prevalence. In certain instances the AIM may distinguish between populations with finer granularity, for example, between sub-continental groups or related ethnic groups. In this example, a device comprises a test for a causal genetic variant having different incidence between these sub-populations.

Because causal genetic variants can have different frequencies in different populations they have some level of predictive value about the population from which the individual comes. In certain embodiments the AIMs used on the device are not, themselves, causal genetic variants. In other embodiments the AIM or collection of AIMs used to predict the source population is/are selected to have a predictive value of at least 80%, 90%, 95%, 98%, or 99%.

In another embodiment the AIMs are selected to classify at least three different populations. In another embodiment the AIMs are selected to classify at least four different continental scale groupings. The AIMs can be selected to classify at least three different populations from the same continent. In another embodiment the AIMs are selected to classify at least ten sub-continental groupings. In another embodiment the AIMs are selected to classify three populations selected from Europeans, East Asians, South Asians, Sub-Saharan Africans, and Native Americans. In another embodiment the AIMs are located on at least 11 different human chromosomes.

## D. Device Validation

In an aspect herein, methods and sets of nucleic acid pools are provided to validate the devices described herein. Genomic DNA samples are not publicly available for many rare genetic diseases. Therefore it can be difficult to validate or quality control a device configured to test for more than 85 rare genetic diseases simultaneously.

In an aspect, a set of nucleic acid pools is disclosed for validating a nucleic acid sequence detection device, wherein each nucleic acid pool comprises a plurality of nucleic acid segments that selectively bind a plurality of causal genetic variant probes, and wherein each pool binds a different plurality of said probes. In some instances, a first pool of the set comprises a first nucleic acid segment that interferes during detection with a second nucleic acid segment of a second pool of the set, and wherein the first pool does not comprise the second nucleic acid segment and the second pool does not comprise the first nucleic acid segment. In some instances, the nucleic acid segments of each pool are a single nucleic acid molecule. In some instances, the nucleic acid segments comprise one or more plasmids.

In an aspect, a method of validating a lot of manufactured nucleic acid sequence detection devices comprises: contacting a plurality of nucleic acid sequence detection devices from the lot with a set of nucleic acid pools for validating a nucleic acid sequence detection device, wherein each nucleic acid pool comprises a plurality of nucleic acid segments that selectively bind a plurality of causal genetic variant probes, and wherein each pool binds a different plurality of said probes; and detecting a presence of the plurality of causal genetic variant probes of the nucleic acid detection devices, wherein the lot of manufactured devices is validated if all of the plurality of causal genetic variant probes are present. In some instances, the method further comprises delivering the lot of manufactured devices when the devices are validated. In some instances, the lot of manufactured devices is rejected if not all of the plurality of causal genetic variant probes are present. In some instances, the lot of manufactured devices is modified and the method is repeated if not all of the plurality of causal genetic variant probes are present.

As described herein, DNA samples representing these rare genetic diseases can be generated by synthesis. Double stranded DNA can be synthesized de novo and cloned into standard high-copy plasmid vectors. This approach has also been recognized by the FDA guidance document "Pharmacogenetic Tests and Genetic Tests for Heritable Markers". The plasmids provide a renewable resource of DNA for use in validation and for use as controls for patient genotyping. Plasmids will be pooled, creating an artificial patient sample. The mutant alleles for each causal genetic variant herein can be synthesized.

In an instance, all of the plasmids comprising all of the causal genetic variants to be tested for by a device herein can be placed into a single large pool or sample. In many cases, many of the causal genetic variants are closely spaced together. In these cases, plasmids generated to test one variant are also templates for other nearby variants and the templates will generate a wildtype signal. This can make it difficult to appropriately simulate the causal genetic variant signal in the off-target probes they interact with. In an instance, overlapping sequences are split into different pools of plasmids that make up a set of nucleic acid segments. For example, variants in the same block are placed in different pools. In another example, the total number of pools needed in a set of pools is equal to the size of the largest block.

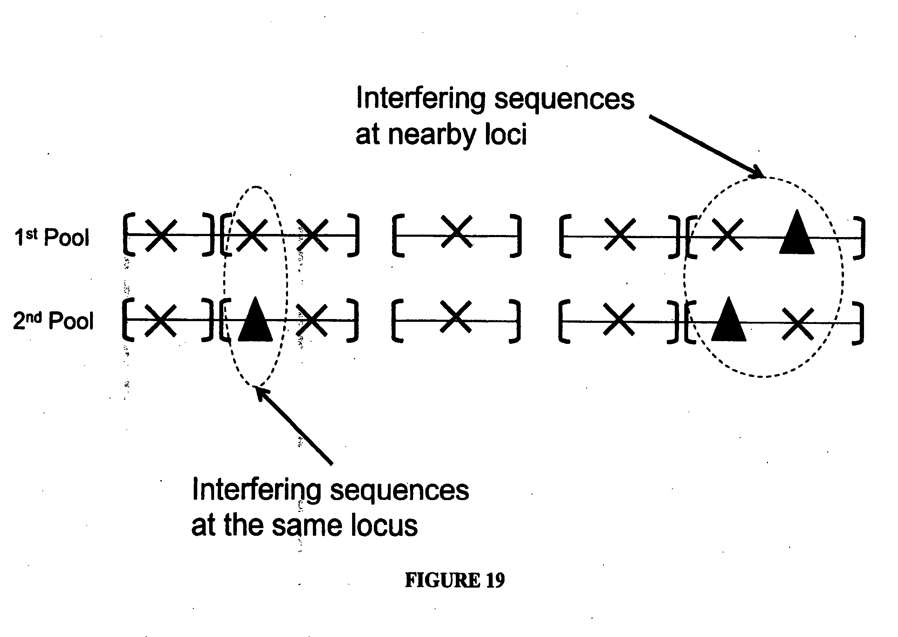

Examples of nucleic acid segments and pools of nucleic acids are demonstrated in Figure 19. Figure 19 illustrates the sequence architecture of the validation sample pools. The basic issue is that validation of a multiplex nucleic acid assay benefits from the use of non-interfering templates to train genotyping call boundaries.

For detecting causal genetic variants to validate a device described herein, plasmid pools are generated by combining one group from each block size (for example, one group each of size 1, 2, 3, 4, 5, 6, 7, 8, 10, 12, and 34) in the appropriate concentration. Thus, each pool contains 455 synthetic variants. For example:

- Pool 1 comprises: Size 1 Group 0 + Size 2 Group 0 + Size 3 Group 0 + Size 4 Group 0 + Size 5 Group 0 + Size 6 Group 0 + Size 7 Group 0 + Size 8 Group 0 + Size 10 Group 0 + Size 12 Group 0 + Size 34 Group 0.
- Pool 2 comprises: Size 1 Group 0 + Size 2 Group 1 + Size 3 Group 1 + Size 4 Group 1 + Size 5 Group 1 + Size 6 Group 1 + Size 7 Group 1 + Size 8 Group 1 + Size 10 Group 1 + Size 12 Group 1 + Size 34 Group 1.
- Pool 3 comprises: Size 1 Group 0 + Size 2 Group 0 + Size 3 Group 2 + Size 4 Group 2 + Size 5 Group 2 + Size 6 Group 2 + Size 7 Group 2 + Size 8 Group 2 + Size 10 Group 2 + Size 12 Group 2 + Size 34 Group 2.

## IV. BUSINESS METHODS

In an aspect, a method comprises: receiving a sample from a user; testing the sample with a nucleic acid detection device configured to test for a plurality of causal genetic variants of rare genetic diseases and at least one ancestry informative marker (AIM); calculating a plurality of probabilities corresponding to the user being a carrier for each of the plurality of causal genetic variants based on results from the testing step relating to the plurality of causal genetic variants and the at least one AIM; and delivering to the user the plurality of probabilities corresponding to the user being a carrier. The method can further comprise: receiving a sample from a second user; testing the sample from the second user with a device configured to test for a plurality of causal genetic variants of rare genetic diseases and at least one ancestry informative marker (AIM); calculating a probability of a child phenotype corresponding to the rare genetic diseases based on results from testing the user and the second user; and delivering the probability of the child phenotype to at least one of the user and the second user. The method can further comprise providing genetic counseling service to at least one of the user and the second user. The method can be carried out as part of a child phenotype prediction service. The method can further comprise obtaining phenotypic information from the user; and using the phenotypic information from the user in the calculating steps. The method can further comprise obtaining family history from the user; and using the family history from the user in the calculating steps.

Also described herein is a method which comprises: marketing a genetic testing service comprising predicting a probability of a child phenotype from a set of parents or potential parents, wherein the prediction is based at least in part on the presence of a plurality of causal genetic variants for more than 85 rare genetic diseases of each of the members of the set of parents or potential parents and based at least in part on the inferred ancestries of each of the members of the set of parents or potential parents; and delivering a probability of the child phenotype from the set of parents or set of potential parents with respect to the more than 85 rare genetic diseases for a fee. The marketing can be conducted in connection with a dating or marriage service. The method can further comprise referring at least one member of the set of parents or set of potential parents to a physician. In some instances, the inferred ancestries are inferred by a test for at least one ancestry informative marker (AIM).

## A. Genetic Testing and Genetic Counseling Referral System

An exemplary business method allows for the provision of a genetic testing service that can predict the probability that an offspring of a couple will have each of a plurality of Mendelian traits. In addition to the results of the genetic testing, referrals to genetic counselors and/or other relevant medical professionals can be provided in order to provide for follow up testing and consultation.

In an embodiment of a business method, genetic testing of a customer begins with a customer order, wherein the a customer pays a fee in exchange for, for example genetic testing materials and referrals to genetic counselors and/or other relevant medical professionals. A customer can be, for example and without limitation, a physician, a genetic counselor, a medical center, an individual, an insurance company, a website, a dating service, a matchmaking service, a pharmaceutical company, a laboratory testing service provider, or a diagnostic platform manufacturer. For example, a customer can be a couple of prospective parents who seek to learn whether their offspring will be at risk for developing some sort of Mendelian disease or rare Mendelian disease. After a customer places an order, a DNA collection kit can be sent to the customer. In an embodiment of a business method of the invention, a customer can deposit a sample into the collection kit. Any sample that would be obvious to one skilled in the art can be deposited into or onto a collection kit. A sample can be any chemical compound that would be obvious to one skilled in the art, such as bodily fluid like saliva or a breath sample containing correlated chemical compounds (see for example, http://www.popularmechanics.com/blogs/science_news/4220196.html), from which it is possible to identify and extract the required Raw Genotypic Information (as discussed herein). The collection kit can then be returned to the company for sending to a genotyping lab or can be returned directly to the genotyping lab for processing. A genotyping lab, either internal within the company, contracted to work with the company, or external from the company, can isolate the customer's DNA from the provided sample. After the DNA has been isolated from the sample, an exemplary device of the invention can test the DNA for the presence of (i) ancestry informative markers and (ii) causal genetic variants (the combination of (i) and (ii) are also referred to herein as, Raw Genotypic Information). In an embodiment, the DNA does not have to be isolated from the sample to test the DNA for the presence of Raw Genotypic Information.

The Raw Genotypic Information can be processed to infer the ancestry of the customer and to confirm or deny the presence of causal genetic variants (also referred to herein as, Processed Genotypic Information). In an embodiment, Processed Genotypic Information can be transmitted to a system of the invention or another calculation system as would be obvious to one skilled in the art to predict the probability that an offspring of the customer will have each of a plurality of traits caused by causal genetic variants found to be present in the customer's DNA.

After analysis of the genotypic testing, the results of the test can be provided to the customer. The results provided to a customer can inform the customer of the carrier status of an individual for a particular Mendelian disease and/or the chances that the individual's future offspring will develop Mendelian disease. In an embodiment, the customer also receives, for example, a) direct phone consultation with a genetic counselor employed by the company, or b) contact information for genetic counselors and/or other medical professionals who can provide the customer with follow up testing and consultation.

Also described herein is a method comprising marketing a genetic testing service in connection with a dating or marriage service, wherein the genetic testing service comprises: (a) predicting the probability that an offspring of the couple will have each of a plurality of traits caused by causal genetic variants, wherein the prediction is determined based on the respective genotypes of the two individuals in the couple and their respective genetically inferred ancestries; and (b) referring the couple to a genetic counselor and/or other medical professional (for example, medical geneticist or obstetrician/gynecologist) based on results of genetic testing. In another embodiment the method further comprises co-marketing a service comprising: c) predicting the probable phenotype of an offspring of a couple for at least one trait, wherein the probability is determined based on the respective phenotypes of the individuals in the couple. In another embodiment the service is an on-line service.

In another component of a business method, a complete list of all genetic counselors and/or other medical professional for follow up testing for all disease conditions can be stored in a database. The people in the database can be those who have previously consented to have their names and contact information used in connection with the genetic testing service. In an embodiment, a particular set of referrals provided to any particular customer can vary and depend on the results of the genetic testing. For example, certain genetic counselors and/or other medical professionals are relevant to display to a customer only in connection with certain Mendelian genetic diseases; such as those for which they have a specialty, for example. So certain referrals will only be shown in connection with certain results of testing.

## B. Genetic Testing and Dating/Marriage Services

Matchmaking services ultimately aim to connect two people typically for the purposes of dating and/or marriage. One may think of any one user of a matchmaking service as evaluating various other users of the matchmaking service to determine the likelihood of a positive match, for example, the likelihood that any two users will be compatible long-term mates. As with any screening process of this kind, numerous factors are evaluated to determine if a match will be successful, for example attractiveness, socio-economic backgrounds, shared interests, shared alma mater are a selected few. As described herein, genetic testing can allow for the evaluation of potential candidates in connection with a matchmaking service according to another important factor: the potential health of future offspring resulting from a given match.

In an aspect of a business method, a genetic testing service can be offered to a customer in connection with a matchmaking service, for example through a single company or a co-marketing or partnership relationship. A user of a matchmaking service can order genetic testing to determine the probability that an offspring resulting from the potential match between the user and a prospective candidate will have each of a plurality of Mendelian traits caused by causative genetic variants found to be present in the respective DNA of the user and the candidate. The user can then use this information to aid in evaluating the candidate for a potential match. The matchmaking service can be an on-line service, such as Shaadi.com, eHarmony.com and Match.com.

In an embodiment, genetic testing begins with a customer order, wherein the customer pays a fee in exchange for genetic testing. For example, a customer can be a user of a matchmaking service who is interested in evaluating another user for a suitable match. Such a customer can use genetic testing to learn whether the potential offspring of a match between said customer and a potential candidate will be at risk for developing Mendelian disease. After selecting a candidate to evaluate, a customer can pay for both the customer's and the candidate's genetic testing with the candidate's consent. The customer and the candidate can also pay separately for genetic testing. After a customer places an order, a DNA collection kit can be sent to the customer. In an embodiment of a business method, a customer can deposit a sample into the collection kit. Any sample that would be obvious to one skilled in the art can be deposited into or onto a collection kit. A sample can be any chemical compound that would be obvious to one skilled in the art -- such as bodily fluid like saliva or a breath sample containing correlated chemical compounds (see for example, http://ww.popularmechanics.com/blogs/science_news/4220196.html) - from which it is possible to identify and extract the required Raw Genotypic Information. The collection kit can then be returned to the company for sending to a genotyping lab or can be returned directly to the genotyping lab for processing. A genotyping lab, either internal within the company, contracted to work with the company, or external from the company, can isolate the customer's DNA from the provided sample. After the DNA has been isolated from the sample, a exemplary device of the invention can be used or other genotyping device can be used to test the DNA for the presence of Raw Genotypic Information. In an embodiment, the DNA does not have to be isolated from the sample to test the DNA for the presence of Raw Genotypic Information.

The Raw Genotypic Information can be processed to infer the ancestry of the customer and to confirm or deny the presence of causal genetic variants (also referred to herein as, Processed Genotypic Information). In an embodiment, Processed Genotypic Information can be transmitted to a system of the invention or another calculation system as would be obvious to one skilled in the art to predict the probability that an offspring of the customer will have each of a plurality of traits caused by causal genetic variants found to be present in the customer's DNA. The results of the testing will then be provided to the customer and the candidate. At this point the customer and the candidate can know the probability that future offspring resulting from a match between the customer and the candidate will develop Mendelian disease.

Also described herein is a method comprising marketing a genetic testing service in connection with a dating or marriage service, wherein the genetic testing service comprises: (a) predicting the probability that an offspring of the couple will have each of a plurality of traits, wherein the prediction is determined based on the respective phenotypes and/or family histories of the two individuals in the couple. In another embodiment the method further comprises (b) referring the couple to a genetic counselor and/or other medical professional (for example, medical geneticist or obstetrician/gynecologist) based on results of genetic testing.

### C. Predicting Offspring Phenotypes from Parental Phenotypes

Genetic testing can allow potential parents or mates to determine the probability that future offspring resulting from their union will develop Mendelian genetic disease. Conceptually, this forecasting is possible because of the predictable mode of inheritance of a Mendelian trait and the generally high correlation between having a variant and having the condition, notwithstanding the issues involved with variable expressivity and penetrance. For complex and/or non-Mendelian traits, it is still difficult to predict their values from genotype measurements alone. Although it is difficult to predict the values of an individual's complex traits from genotype information, it is often easy to predict the values of a child's complex traits from phenotype information on the parents. That is, for phenotypes with high heritability, a measurement of mother and father trait values is sufficient to provide excellent predictive power for child traits (http://www.amazon.com/Genetics-Analysis-Quantitative-Traits-Michael/dp/0878934812).

In another aspect this disclosure provides a method comprising predicting the probable phenotype of an offspring of a couple for at least 10 non-Mendelian traits, wherein the probability is determined based on the respective phenotypes of the individuals in the couple. In one embodiment the phenotypic traits include a member selected from the group consisting of height, weight, cognitive ability, academic achievement, extraversion, neuroticism, agreeableness, conscientiousness, openness, religiosity and conservatism.

In an embodiment, phenotype-phenotype predictions can be offered for free in connection with genetic testing as a means to allow prospective parents or mates to learn about the non-Mendelian traits that their future offspring may have.

A customer can provide information describing his/her phenotype as well as information describing his/her partner's phenotype (Parental Phenotypes). In an embodiment, these Parental Phenotypes can be transmitted to a system of the invention or another calculation system as would be obvious to one skilled in the art to predict the probable phenotype of an offspring of the customer and his/her partner based on the input Parental Phenotypes (Offspring Phenotype). The Offspring Phenotype can then be provided to the customer for free or for a fee.

### D. Computer Systems

In another aspect this disclosure provides a method comprising offering a first and second service, wherein: a) the first service comprises predicting the probability that an offspring of the couple will have each of a plurality of traits caused by causal genetic variants, wherein the prediction is based on the respective genotypes of the two individuals in the couple; and b) the second service comprises predicting the probable phenotype of an offspring of the couple for a plurality of traits, wherein the probability is determined based on the respective phenotypes and/or the family history of the individuals in the couple. In one embodiment at least one prediction is further based on the respective genetically inferred ancestries of the individuals. In another embodiment the first service is offered as a service for a fee and the second service is offered as a free service.

In another aspect this disclosure provides a method comprising: a) taking into computer memory responses to a quiz about family history of each individual of a couple; b) taking into computer memory genetic information about each individual of the couple; c) displaying: i) at least one individual's carrier status for at least one trait determinable by the genetic information or the family history or ii) traits of offspring of the couple determinable by the family histories and/or the genetic information. In one embodiment the carrier status is displayed on a website.

In another aspect this disclosure provides a system comprising: a) computer readable medium configured to store family history information from each member of a couple; b) computer readable medium configured to store data comprising genetic information about each member of the couple; c) computer readable medium comprising computer code that, when executed: i) predicts each individual's carrier status with respect to traits caused by alleles identified in the genetic information; or ii) predicts probable traits of offspring of the couple determinable by the family histories and/or the genetic information; and d) a display that displays: i) carrier status of at least one member of the couple or ii) probable traits of the offspring. In one embodiment the system further comprises e) a webpage configured to accept an offer to purchase a DNA test kit. In another embodiment the display is electronic, for example, a webpage. In another embodiment the system further comprises e) a display that displays referrals to a genetic counselor and/or other medical professional (for example, medical geneticists or obstetrician/gynecologist) based on the genetic information.

The internet and the world wide web offer access to and distribution of information. As such, in an embodiment, a website can be particularly suited to efficiently providing various functionality for allowing customers to purchase genetic testing and receive the results of genetic testing. The system typically will include a server on which the website resides. Users use an interface connected to the server, such as a computer monitor or a telephone screen, to interact with the website by clicking or rolling over links that pop up information or direct the user to another webpage. Websites typically are interactive, allowing the user to input information or a query and obtain a response on the interface.

In an aspect of a system and business method, a website can allow a customer to purchase, manage, and view the results of genetic testing as well as to learn more generally about the probability that potential offspring will develop Mendelian disease. For example, a customer can be a couple of prospective parents who seek to learn whether their offspring will be at risk for developing Mendelian disease.

After a customer creates an account with the company on the website, the customer can first choose to take advantage of a free service that predicts the probability that offspring of the customer will have a trait caused by causative genetic variants. In another embodiment, the previously mentioned service is provided for a fee. The service can be offered to the customer in the form an online quiz that is promoted and accessible via the website. The quiz can also be a quiz that is ordered and physical mailed or emailed to a customer. A quiz can ask the customer a series of questions related to family history and ethnicity that a customer can answer. Once the responses are given by a customer, the responses can be sent to a server for storage and processing. In an example, computer code executes on the server to compute (i) the carrier status of the customer, if any; and (ii) the probability that an offspring of the customer will develop each of a plurality of Mendelian traits, based on the responses provided by the customer to the quiz. The results of this operation are then sent to a server for storage. The server can electronically transmit this information to a user interface, such as the website or an email, for display to the customer. In a particular embodiment, the results of the quiz are presented to the customer in the results section of the website. The results of the quiz comprise (i) the carrier status of the customer, if any; and (ii) a listing of the probability that an offspring of the customer will develop each of a plurality of Mendelian traits.

The customer can then be presented with the offer to purchase genetic testing to determine (i) the carrier status of the customer; and (ii) the probability that an offspring of the customer will develop each of a plurality of Mendelian traits, based on the causative genetic variants to be found present in the customer's DNA.

If the customer chooses to purchase genetic testing, then the customer may pay a fee, for example through an online credit card transaction, in exchange for genetic testing, direct phone consultation with a genetic counselor on the company's staff and/or referrals to genetic counselors and/or other relevant medical professionals. The genetic testing and referrals can be paid for by a fee at the point of purchase or can be included in an initial user registration fee. In an embodiment, the services are free and revenue is generated by the company by advertising other products in conjunction with a particular product. For example, after a customer places an order online, the order is sent to a server for processing. Once payment has been verified, the order processing server can send an electronic notification to a shipping vendor to mail a DNA collection kit to the customer. In an embodiment, the DNA collection kit is separate from the genetic testing service or the user or customer already has or obtains the DNA collection kit from another source. Notifications can also periodically be sent electronically to the customer comprising order confirmation and updates on order and shipping status. Once the customer receives the collection kit, the customer can use it to obtain Raw Genotypic Information (as defined below). In an embodiment of a business method of the invention, a customer can deposit a sample into the collection kit. Any sample that would be obvious to one skilled in the art can be deposited into or onto a collection kit. A sample can be any chemical compound that would be obvious to one skilled in the art,

such as bodily fluid like saliva or a breath sample containing correlated chemical compounds (see for example, http://www.popularmechanics.com/blogs/science_news/4220196.html), from which it is possible to identify and extract the required Raw Genotypic Information (as defined below). The collection kit can then be returned to the company for sending to a genotyping lab or can be returned directly to the genotyping lab for processing. A genotyping lab, either internal within the company, contracted to work with the company, or external from the company, can isolate the customer's DNA from the provided sample. After the DNA has been isolated from the sample, a exemplary device of the invention can be used or other genotyping device can be used to test the DNA for the presence of (i) ancestry informative markers and (ii) causal genetic variants (the combination of (i) and (ii) are also referred to herein as, Raw Genotypic Information). In an embodiment, the DNA does not have to be isolated from the sample to test the DNA for the presence of Raw Genotypic Information.

Raw Genotypic Information can be sent electronically to a server for storage and processing. Computer code on the server can execute on the Raw Genotypic Information to infer the ancestry of the customer and to confirm the presence of causal genetic variants, if any. The Processed Genotypic Information can then be electronically sent to a server, where computer code on the server can execute on the Processed Genotypic Information to predict the probability that an offspring of the customer will have each of a plurality of traits caused by causal genetic variants found to be present in the customer's Processed Genotypic Information. Results can then be electronically transmitted to a server for storage.

In an example, a notification can be sent to the customer to alert the customer to the availability of the results. The notification can be electronic, such as for example without limitation, a text message, an email, or other data packet; or the notification can be non-electronic, such as for example without limitation, a phone call from a genetic counselor or printed communication such as a report sent through the mail. The results provided to a customer can inform the customer of the carrier status of the customer for a particular Mendelian disease and/or the chances that the customer's future offspring will develop Mendelian disease. After the customer has received results and referrals, the customer's order can be considered fulfilled, and results and referrals can remain accessible to the customer through an online website account. The customer can then choose to further pursue a referral offline if the customer so desires but outside of the purview of the website.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## EXAMPLE 1

A method is provided herein of validating or quality controlling a lot of manufactured devices configured to test for causal genetic variants for more than 85 rare genetic diseases. Genomic DNA samples are not publicly available for many rare genotypes, hence it can be difficult to get a proper number samples to test the device. It may also be desirable to avoid dilution of the test with wild type alleles and to avoid requiring a large number of devices to test the manufactured lot. In this example, DNA samples representing the causal genetic variants were generated by synthesis. Double stranded DNA were synthesized de novo and cloned into standard high-copy plasmid vectors. These samples represent 662 disease-causing variants and 139 diseases. This approach has been described in a number of recent reports related to CF carrier testing (Berwouts et al., 2008; Christensen et al., 2007). All plasmids were sequence verified to contain zero errors by double stranded, primer walk, BigDye methodology.

The nucleic acid segments of the causal genetic variants were organized into blocks to avoid interference during detection of any of the causal genetic variants. Variants in the same block were placed in different pools. The total number of pools needed is equal to the size of the largest block. Using a maximal probe overlap of 50% sequence similarity, the largest block is size 34 in this example.

The counts by block sizes are: 320 blocks of size 1 (singleton variants); 84 of size 2; 21 of size 3; 12 of size 4; 2 of size 5; 7 of size 6; 3 of size 7; 1 of size 8; 1 of size 10; 3 of size 12; and 1 of size 34.

Inserts were divided into sets that can be combined into one or more plasmids for synthesis. The first set is the group of 320 inserts with no overlapping regions. The next 2 sets are the groups of inserts from blocks of size two (each with 84 inserts). The next is 3 groups of 21 inserts each for block size 3. The next is 4 groups of 12 inserts each, and so on such that there are n groups generated by the blocks of size n. In total, 92 groups of inserts were created for plasmid synthesis.

**EXAMPLE 2**

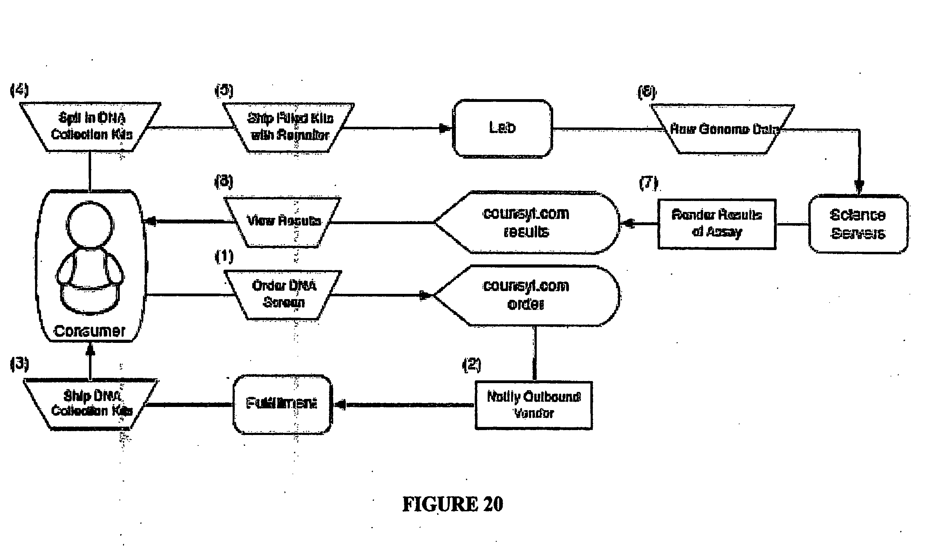

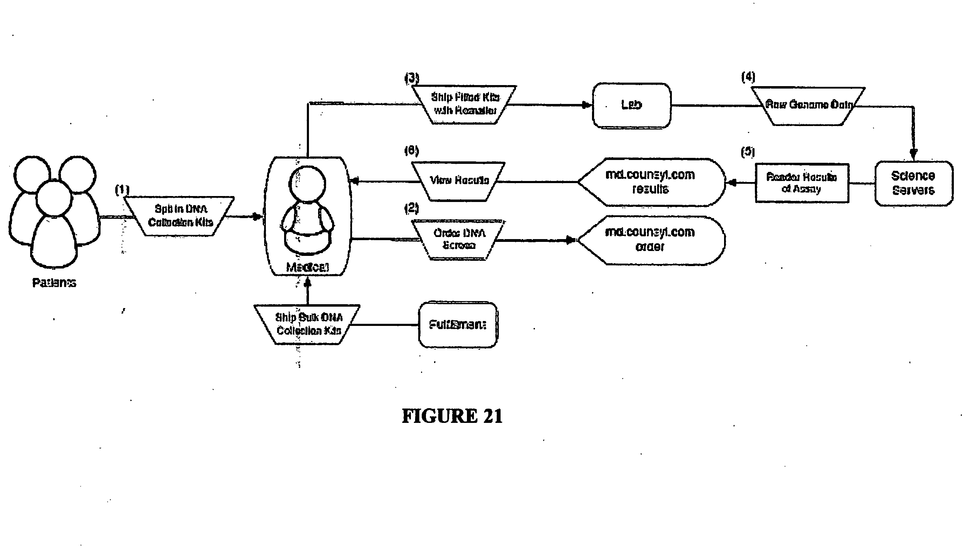

Exemplary processes of delivering a probability that a user is a carrier of rare genetic disease are demonstrated in Figures 20-23. Figures 20-21 illustrate pipelines for order fulfillment for web and medical customers respectively. An order can be placed by a physician or a consumer. An order can be placed for a single test or for a couple or family. The order can be accepted through a web site. The ordering system can accept contact information, demographic details and billing information. Contact information can include, without limitation, name, address, telephone number, and email addresses. Demographic information can include, without limitation, sex, date of birth, and self-reported ethnicity. An order confirmation notification can be sent using the provided contact information. Acceptable orders are added to a database, and the states of these orders can be subsequently maintained by a state machine.

A sample collection kit is then sent to the user. A sample is collected that is any human tissue or fluid. The sample can also be isolated DNA from a human. Examples of samples useful for this example include, but are not limited to: saliva, blood, urine, buccal cells, amniotic fluid, cell scrapings, and cell culture. The sample is then genotyped using a device described herein. Phenotype solicitation, for example, retrieving self-identification of phenotypic traits of a user, can be performed in parallel with sample processing.

Sample collection can be performed at home, in a physician's office, or at a specialized collection site. Sample collection and return can be tracked by advancing the state of the order-tracking state machine. Samples received by the accessioning facility can be registered in the database system by advancing their state in the state machine. After acceptance at the accessioning facility, samples can be delivered to the genotyping facility. The genotyping facility can return raw genome data to a secure data storage server by secure file transfer protocols. File upload can trigger an advance of the state machine. This advance can trigger a server configured to perform genotype calling to retrieve the raw genome data from the data storage server as well as any phenotype data associated with the order. The genotyping algorithm can produce a fully probabilistic genotype call.

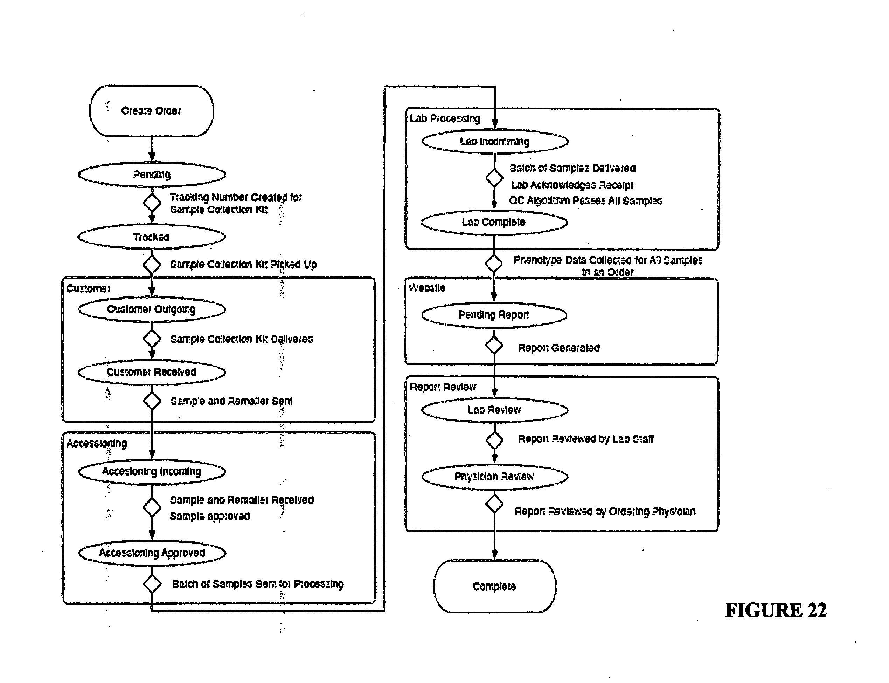

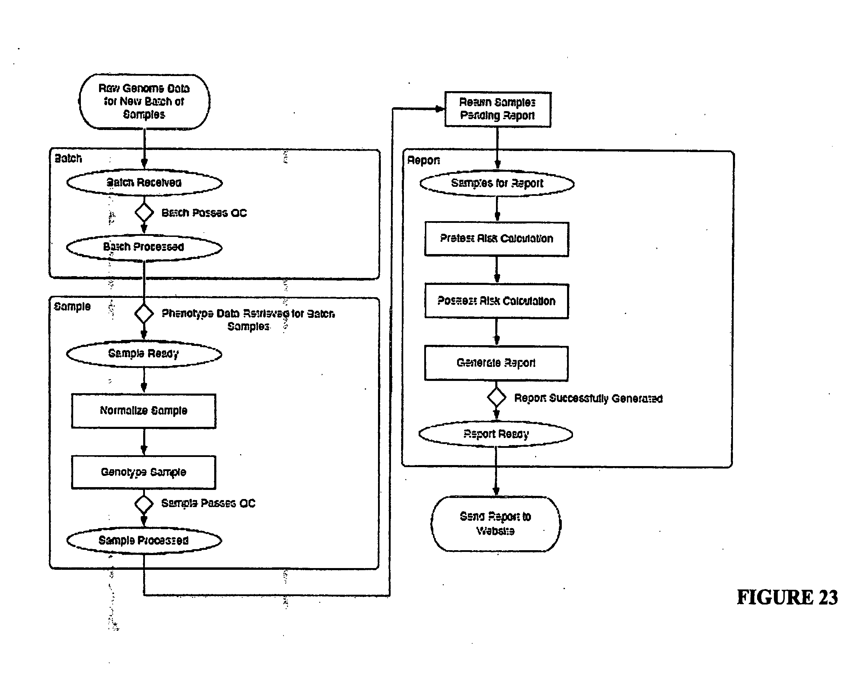

Figures 22-23 illustrate the high level sample processing pipeline and detailed computational pipeline respectively. The algorithms described in the previous Figures and the processing step as shown in Figure 22 outlined in Figure 23. Batches of chips are received and measured for quality control purposes (Batch passes QC). Information such as family history, gender, or self-reported ancestry is used to serve as an independent check on calling for each sample

(Phenotype data retrieved for batch samples). In parallel with this process, a report with child predictions is constantly updated. First pre-test risk calculations are delivered, based on phenotype (such as family history and other answers to an online questionnaire). Once a genotype sample is received and processed, post-test calculations are given. The report is then generated and sent to the final stages of the pipeline, for laboratory staff and physician approval as shown in Figure 22.

Quality control metrics can be generated from the calling process. An example quality control metric is the percentage of probabilistic genotype calls in which at least one genotype has a posterior probability greater than a threshold value. A batch of samples is processed together. When processed as a batch, the individual probabilistic genotype calls can be used to generate batch-level quality control statistics. Probabilistic genotype calls can be stored in a database. Successful genotype calling can trigger an advance of the order state. For a couple or family order, the state machine can hold for completion of the entire order, else single orders can be passed to the next state. If phenotype data is required for risk calculation, then the state machine can delay processing until all phenotype data is collected. The state machine can also trigger a notification to the patient that phenotype data is required. If all genotype and phenotype data are ready, then the state machine can advance, trigger the risk calculation server to perform risk calculation. The results of risk calculation can be serialized and transferred to a results reporting system. This is a machine-readable format of the results. The state machine can advance the order when the transfer is completed. The results reporting server can combine the probabilistic risk calculation with appropriate text and formatting to generate a human-readable report. This human readable report can be further formatted for display on a website. This human readable report can be formatted for other media such as PDF files for printing. The final results reports can be automatically released using an autoverification system. A human can review the reports for release. In one instantiation the reviewers are a clinical laboratory scientist and a physician. The results are accessed via a web portal which links to a view of the results and a summary of the quality control metrics. Acceptance of the report by the clinical laboratory scientist releases the results to the physician. The physician can review the results in a similar portal and approve the final release of the results.

**EXAMPLE 3**

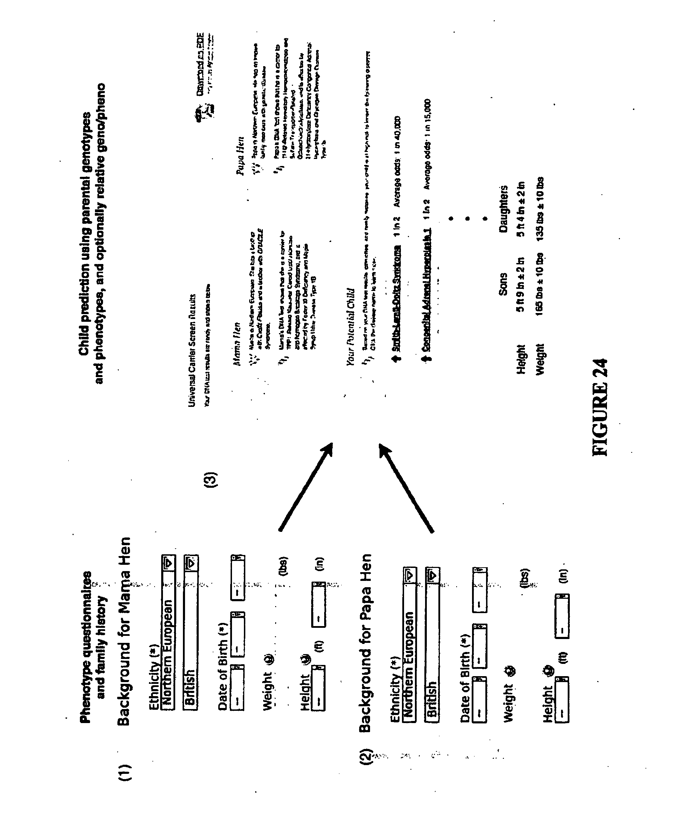

Figure 24 illustrates exemplary the input and output steps for report generation for two hypothetical parents: Mama Hen and Papa Hen. A child prediction is produced that incorporates mother and father genotypes, mother and father phenotypes, and relative genotypes and phenotypes. Any or all of these variables can be missing values, with defaults initialized from demographically similar individuals (and if this is not known, from the world population). The resulting child prediction may include not only disease risk, but also other variables such as height and weight. Different variables in the child prediction will use different weights of genotype and phenotype.

**Claims**

1.  A method comprising:

    a) using a multiplexed genetic test for testing a prospective mother or a prospective father of a potential child to determine a presence of a plurality of causal genetic variants each variant corresponding to at least one rare Mendelian genetic disease and a presence of at least one ancestry informative marker (AIM); and
    b) predicting a probability of a phenotype of the potential child with respect to the at least one rare Mendelian genetic disease based at least in part on the presence of the plurality of causal genetic variants and the presence of the at least one AIM, wherein said predicting comprises performing a fully probabilistic analysis on data collected on said causal genetic variants, wherein the information obtained from the presence of at least one AIM of the prospective mother or prospective father is used to reduce a misclassification probability of said causal genetic variants in the multiplexed genetic test.

2.  The method of any of the preceding claims wherein predicting in step b) is further based on phenotypic information about the prospective mother or prospective father.

3.  The method of any of the preceding claims comprising testing a prospective mother and a prospective father of the potential child.

4.  The method of any of the preceding claims wherein a plurality of the genetic diseases each have a frequency of less than 1 in 1000 in humans.

5.  The method of any of the preceding claims wherein the plurality of rare genetic diseases is more than 85 rare Mendelian genetic diseases.

6.  The method of claim 5 wherein the plurality of the diseases are selected from cystic fibrosis, Tay Sachs, 21-Hydroxylase Deficiency, ABCC8-Related Hyperinsulinism, ARSACS, Achondroplasia, Achromatopsia, Adenosine Monophosphate Deaminase 1, Agenesis of Corpus Callosum with Neuronopathy, Alkaptonuria, Alpha-1-Antitrypsin Deficiency, Alpha-Mannosidosis, Alpha-Sarcoglycanopathy, Alpha-Thalassemia, Angiotensin II Receptor, Type I, Argininosuccinicaciduria, Aspartylglycosaminuria, Ataxia with Vitamin E Deficiency, Ataxia-Telangiectasia, Autoimmune Polyendocrinopathy Syndrome Type 1, Bardet-Biedl Syndrome, Best Vitelliform Macular Dystrophy, Beta-Sarcoglycanopathy, Beta-Thalassemia, Biotinidase Deficiency, Blau Syndrome, Bloom Syndrome, CFTR-Related Disorders, CLN3-Related Neuronal Ceroid-Lipofuscinosis, CLN5-Related Neuronal Ceroid-Lipofuscinosis, CLN8-Related Neuronal Ceroid-Lipofuscinosis, Canavan Disease, Carnitine Palmitoyltransferase IA Deficiency, Carnitine Palmitoyltransferase II Deficiency, Cartilage-Hair Hypoplasia, Choroideremia, Cohen Syndrome, Congenital Cataracts, Facial Dysmorphism, and Neuropathy, Congenital Disorder of Glycosylation, Congenital Disorder of Glycosylation Ib, Congenital Finnish Nephrosis, Cystinosis, DFNA 9 (COCH), Early-Onset Primary Dystonia (DYTI), Epidermolysis Bullosa Junctional, Herlitz-Pearson Type, FANCC-Related Fanconi Anemia, FGFR1-Related Craniosynostosis, FGFR2-Related Craniosynostosis, FGFR3-Related Craniosynostosis, Factor V Leiden Thrombophilia, Factor V R2 Mutation Thrombophilia, Factor XI Deficiency, Factor XIII Deficiency, Familial Dysautonomia, Familial Hypercholesterolemia Type B, Familial Mediterranean Fever, Free Sialic Acid Storage Disorders, Frontotemporal Dementia with Parkinsonism-17, Fumarase deficiency, GJB2-Related DFNA 3 Nonsyndromic Hearing Loss and Deafness, GJB2-Related DFNB 1 Nonsyndromic Hearing Loss and Deafness, GNE-Related Myopathies, Galactosemia, Gaucher Disease, Glucose-6-Phosphate Dehydrogenase Deficiency, Glutaricacidemia Type 1, Glycogen Storage Disease Type 1 a, Glycogen Storage Disease Type Ib, Glycogen Storage Disease Type II, Glycogen Storage Disease Type III, Glycogen Storage Disease Type V, Gracile Syndrome, HFE-Associated Hereditary Hemochromatosis, Hemoglobin S Beta-Thalassemia, Hereditary Fructose Intolerance, Hereditary Pancreatitis, Hereditary Thymine-Uraciluria, Hexosaminidase A Deficiency, Hidrotic Ectodermal Dysplasia 2, Homocystinuria Caused by Cystathionine Beta-Synthase Deficiency, Hyperkalemic Periodic Paralysis Type 1, Hyperornithinemia-Hyperammonemia-Homocitrullinuria Syndrome, Hyperoxaluria, Primary, Type 1, Hyperoxaluria, Primary, Type 2, Hypochondroplasia, Hypokalemic Periodic Paralysis Type 1, Hypokalemic Periodic Paralysis Type 2, Hypophosphatasia, , Isovaleric Acidemias, Krabbe Disease, LGMD2I, French-Canadian Type, Long Chain 3-Hydroxyacyl-CoA Dehydrogenase Deficiency, MTHFR Deficiency, MTHFR Thermolabile Variant, MTTS1-Related Hearing Loss and Deafness, MYH-Associated Polyposis, Maple Syrup Urine Disease Type 1 A, Maple Syrup Urine Disease Type 1B, Medium Chain Acyl-Coenzyme A Dehydrogenase Deficiency, Megalencephalic Leukoencephalopathy with Subcortical Cysts, Metachromatic Leukodystrophy, Mucolipidosis IV, Mucopolysaccharidosis Type I, Mucopolysaccharidosis Type IIIA, Mucopolysaccharidosis Type VII, Multiple Endocrine Neoplasia Type 2, Muscle-Eye-Brain Disease, Nemaline Myopathy, Niemann-Pick Disease Due to Sphingomyelinase Deficiency, Niemann-Pick Disease Type C1, Nijmegen Breakage Syndrome, PPT1-Related Neuronal Ceroid-Lipofuscinosis, PROP1-related pituitary hormone deficiency, Pallister-Hall Syndrome, Paramyotonia Congenita, Pendred Syndrome, Peroxisomal Bifunctional Enzyme Deficiency, Phenylalanine Hydroxylase Deficiency, Plasminogen Activator Inhibitor I, Polycystic Kidney Disease, Autosomal Recessive, Prothrombin G20210A Thrombophilia, Pycnodysostosis, Retinitis Pigmentosa, Autosomal Recessive, Bothnia Type, Rett Syndrome, Rhizomelic Chondrodysplasia Punctata Type 1, Short Chain Acyl-CoA Dehydrogenase Deficiency, Shwachman-Diamond Syndrome, Sjogren-Larsson Syndrome, Smith-Lemli-Opitz Syndrome, Spastic Paraplegia 13, Sulfate Transporter-Related Osteochondrodysplasia, TFR2-Related Hereditary Hemochromatosis, TPP1-Related Neuronal Ceroid-Lipofuscinosis, Thanatophoric Dysplasia, Transthyretin Amyloidosis, Trifunctional Protein Deficiency, Tyrosine Hydroxylase-Deficient DRD, Tyrosinemia Type I, Wilson Disease, X-Linked Juvenile Retinoschisis and Zellweger Syndrome Spectrum.

7. The method of any of the preceding claims wherein the plurality of causal genetic variants correspond to one or more genetic diseases for which East Asian population is at increased risk, one or more genetic diseases for which sub-Saharan African population is at increased risk and one or more genetic diseases for which Western Eurasian population is at increased risk.

8. The method of claim 7 wherein:

   (i) the increased risk is at least 10-fold compared with another of the populations, or
   (ii) the Western Eurasian population is selected from European, Western/Central Asian, South Asian, and North African subpopulations, or
   (iii) the East Asian population is selected from Chinese, Japanese, and Korean subpopulations.

9. The method of any of claims 1-6 wherein the causal genetic variants correspond to one or more genetic diseases for which Native American population is at increased risk or one or more genetic diseases for which Ashkenazi Jewish population is at increased risk.

10. The method of any of the preceding claims wherein at least one AIM is not a causal genetic variant.

11. The method of any of the preceding claims wherein the AIMs include at least one AIM that distinguishes African and European populations, at least one AIM that distinguishes African and Asian populations and at least one AIM that distinguishes European and Asian populations.

12. The method of claim 11 wherein the AIMs include at least one AIM that distinguishes African and American populations; European and American populations, Asian and American populations, Northern European and Southern European populations, Northern European and Azkenazi Jewish populations, Southern European and Azkenazi Jewish populations, Irish and English populations, Spanish and Caucasian populations, Chinese and Japanese populations, or South Asian, Central Asian and East Asian populations.

13. The method of any of the preceding claims wherein the AIMs are selected from AIMs of Figure 3.

14. The method of claim 1 wherein testing comprises use of a nucleic acid array, sequencing by hybridization, sequencing by ligation, sequencing by extension, Sanger sequencing, Maxam Gilbert sequencing or pyrosequencing.

15. A computer readable medium comprising a computer code that when executed predicts a probability of a phenotype of a potential child, from the prospective mother or prospective father, with respect to at least one rare Mendelian genetic disease, based at least in part on the results of a test of the prospective mother or prospective father for the presence of a plurality of causal genetic variants and at least one ancestry informative marker (AIM),
wherein the probability of a phenotype of the potential child is calculated in accordance with the method of any one of claims 1 to 13.

16. A system for predicting a child phenotype comprising:

a multiplex nucleic acid detection device configured to detect a plurality of causal genetic variants corresponding to at least one rare Mendelian genetic disease and at least one ancestry informative marker (AIM), wherein the device is in contact with a sample from a prospective mother or prospective father of a potential child;
a reader configured to read data from the devices; and
a computer readable medium according to claim 15.

17. A system for indicating if a subject is a carrier of a rare Mendelian genetic disease comprising: a reader configured to read data from a multiplex nucleic acid detection device configured to detect a plurality of causal genetic variants corresponding to at least one rare Mendelian genetic disease and at least one ancestry informative marker (AIM); and a computer readable medium according to claim 15.

18. A method comprising:

receiving a sample from a user;
testing the sample with a multiplex nucleic acid detection device configured to test for a plurality of causal genetic variants of rare Mendelian genetic diseases and at least one ancestry informative marker (AIM);
calculating a plurality of probabilities for the possible user genotypes at each location of the plurality of causal genetic variants based on results from the testing step relating to the plurality of causal genetic variants and the at least one AIM,
wherein said calculating comprises performing a fully probabilistic analysis on data collected on said causal genetic variants, wherein the information obtained from the at least one AIM about the ancestry of the user is used to reduce a misclassification probability of said causal genetic variants in the multiplex nucleic acid detection device; and
delivering to the user the plurality of probabilities corresponding to the user being a carrier.

19. The method of any of claims 1-14 wherein at least two of the rare Mendelian genetic diseases occur at frequencies that differ by at least 10-fold in at least two distinct populations, wherein the at least two distinct populations are differentiated by the at least one AIM.

**Patentansprüche**

1. Verfahren, umfassend:

a) das Verwenden eines Mehrfach-Gentests zum Testen einer zukünftigen Mutter oder eines zukünftigen Vaters eines möglichen Kindes, um ein Vorliegen einer Vielzahl kausaler genetischer Varianten, wobei jede Variante zumindest zu einer seltenen Mendelschen Erbkrankheit entspricht, und ein Vorliegen zumindest eines Abstammungsinformationsmarkers (AIM) zu bestimmen; und
b) das Vorhersagen einer Wahrscheinlichkeit eines Phänotyps des möglichen Kindes in Bezug auf die zumindest eine seltene Mendelsche Erbkrankheit, basierend zumindest teilweise auf dem Vorliegen der Vielzahl kausaler genetischer Varianten und dem Vorliegen des zumindest einen AIM, wobei das Vorhersagen das Durchführen einer vollständigen Wahrscheinlichkeitsanalyse auf den zu diesen kausalen genetischen Varianten gesammelten Daten umfasst, wobei die von der Gegenwart des zumindest einen AIM der zukünftigen Mutter oder des zukünftigen Vaters erhaltene Information verwendet wird, um die Wahrscheinlichkeit einer falschen Klassifizierung der kausalen genetischen Varianten in dem Mehrfach-Gentest zu reduzieren.

2. Verfahren nach einem der vorangegangenen Ansprüche, wobei das Vorhersagen in Schritt b) zudem auf phänotypischer Information über die zukünftige Mutter oder den zukünftigen Vater basiert.

3. Verfahren nach einem der vorangegangenen Ansprüche, das das Testen einer zukünftigen Mutter und eines zukünftigen Vaters des möglichen Kindes umfasst.

4. Verfahren nach einem der vorangegangenen Ansprüche, wobei eine Vielzahl der Erbkrankheiten jeweils eine Häufigkeit von weniger als 1 pro 1000 Menschen aufweist.

5. Verfahren nach einem der vorangegangenen Ansprüche, wobei die Vielzahl der seltenen Erbkrankheiten mehr als 85 seltene Mendelsche Erbkrankheiten sind.

**6.** Verfahren nach Anspruch 5, wobei die Vielzahl der Krankheiten ausgewählt ist aus zystischer Fibrose, Tay-Sachs-Syndrom, 21-Hydroxylase-Mangel, ABCC8-bezogenem Hyperinsulinismus, ARSACS, Achondroplasie, Achromatopsie, Adenosinmonophosphatdeaminase 1, Corpus-Callosum-Agenesie mit Neuronopathie, Alkaptonuriem, Alpha-1-Antitrypsin-Mangel, $\alpha$-Mannosidose, $\alpha$-Sarcoglycanopathie, $\alpha$-Thalassämie, Angiotensin-II-Rezeptor Typ I, argininosuccinische Azidopathie, Aspartylglycosaminurie, Ataxie mit Vitamin-E-Mangel, Ataxie-Telangiectasie, autoimmunem Typ-1-Polyendokrinopathiesyndrom, Bardet-Biedl-Syndrom, vitelliformer Makuladystrophie (Morbus Best), $\beta$-Sarcoglycanopathie, $\beta$-Thalassämie, Biotinidasemangel, Blausyndrom, Bloom-Syndrom, CFTR-bezogenen Störungen, CLN3-bezogener neuronaler Ceroid-Lipofuszinose, CLN5-bezogener neuronaler Ceroid-Lipofuszinose, CLN8-bezogener neuronaler Ceroid-Lipofuszinose, Morbus Canavan, Carnitin-Palmitoyltransferase-IA-Mangel, Carnitin-Palmitoyltransferase-II-Mangel, Knorpel-Haar-Hypoplasie, Choroideremie, Cohen-Syndrom, angeborener Linsentrübung, Dysmorphie im Gesicht und Neuropathie, angeborener Störung der Glykosylierung, angeborener Störung der Glycosylierung-Ib, angeborener finnischer Nephrose, Zystinose, DFNA 9 (COCH), frühzeitig auftretender primärer Dystonie (DYTI), Epidermolysis Bullosa Junctional, Herlitz-Pearson-Typ, FANCC-bezogener Fanconi-Anämie, FGFR-bezogener Craniosynostose, FGFR-2-bezogener Craniosynostose, FGFR-3-bezogener Craniosynostose, Faktor-V-Leiden-Thrombophilie, Faktor-V-R2-Mutationsthrombophilie, Faktor-XI-Mangel, Faktor-XIII-Mangel, familiärer Dysautonomie, familiärer Typ-B-Hypercholesterinämie, familiärem Mittelmeerfieber, Speicherstörungen freier Sialinsäure, frontotemporaler Demenz mit Parkinsonismus-17, Fumarasemangel, GJB2-bezogenem DFNA-3-nichtsyndromischem Hörverlust und Gehörlosigkeit, GNE-bezogenen Myopathien, Galactosämie, Morbus Gaucher, Glucose-6-Phosphat-Dehydrogenasemangel, glutarischer Azidopathie Typ 1, Glykogenspeicherkrankheit Typ 1a, Glykogenspeicherkrankheit Typ 1 b, Glykogenspeicherkrankheit Typ II, Glykogenspeicherkrankheit Typ III, Glykogenspeicherkrankheit Typ V, Gracile-Syndrom, HFE-assoziierter erblicher Hämochromatose, Hämoglobin-S-$\beta$-Thalassämie, erblicher Fruktoseintoleranz, erblicher Pankreatitis, erblicher Thymin-Uracilurie, Hexosaminidase-A-Mangel, Hidrotischer ektodermaler Dysplasie 2, durch Cystathionin-$\beta$-Synthasemangel verursachter Homozystinurie, periodischer hyperkalämischer Lähmung Typ 1, Hyperornithinämie-Hyperammonämie-Homocitrullinurie-Syndrom, primärer Hyperoxalurie Typ 1, primärer Hyperoxalurie Typ 2, Hypochondroplasie, periodischer hypokalämischer Lähmung Typ 1, periodischer hypokalämischer Lähmung Typ 2, Hypophosphatasie, isovalerischer Azidämie, Morbus Krabbe, LGMD2I, französisch-kanadischer Typus, Langketten-3-Hydroxyacyl-CoA-Dehydrogenasemangel, MTHFR-Mangel, thermolabiler MTHFR-Variante, MTTS1-bezogenem Hörverlust und Gehörlosigkeit, MYH-assoziierter Polypose, Ahornsirupurinkrankheit Typ 1A, Ahornsirupurinkrankheit Typ 1B, Mittelketten-Acyl-Coenzym-A-Dehydrogenasemangel, megaenzephalischer Leukoenzephalopathie mit subkortikalen Zysten, metachromatischer Leukodystrophie, Mucolipidose IV, Mucopolysaccharidose Typ I, Mucopolysaccharidose Typ IIIA, Mucopolysaccharidose Typ VII, multipler endokriner Neoplasie Typ 2, Muskel-Auge-Gehirn-Krankheit, Nemalin-Myopathie, Niemann-Pick-Krankheit durch Sphingomyelinasemangel, Niemann-Pick-Krankheit Typ C1, Nijmegen-Breakage-Syndrom, PPT1-bezogener neuronaler Ceroid-Lipofuszinose, PROP1-bezogenem Hypophysenhormonmangel, Pallister-Hall-Syndrom, Paramyotonia Congenita, Pendred-Syndrom, peroxisomalem biofunktionalem Enzymmangel, Phenylalaninhydroxylasemangel, Plasminogen-Aktivator-Inhibitor-1, autosomaler rezessiver polyzystischer Nierenerkrankung, Prothrombin-G20210A-Thrombophilie, Pycnodysostose, autosomaler rezessiver Retinitis pigmentosa, Botnia-Typ, Rett-Syndrom, rhizomeler Chondrodysplasia Punctata Typ 1, Kurzketten-Acyl-CoA-Dehydrogenasemangel, Shwachman-Diamond-Syndrom, Sjorgren-Larsson-Syndrom, Smith-Lemli-Opitz-Syndrom, spastischer Paraplegie 13, schwefeltransporterbezogener Skelettdysplasie, TFR2-bezogener erblicher Hämochromatose, TPP1-bezogener neuronaler Ceroid-Lipofuszinose, thanatophorer Dysplasie, Transthyretinamyloidose, trifunktionalem Proteinmangel, tyrosinhydroxylase-defizienter DRD, Tyrosinämie Typ I, Morbus Wilson, X-bezogener jugendlicher Retinoschisis und dem Zellweger-Syndrom-Spektrum.

**7.** Verfahren nach einem der vorangegangenen Ansprüche, wobei die Vielzahl kausaler genetischer Varianten einer oder mehreren Erbkrankheiten, für die die ostasiatische Bevölkerung ein erhöhtes Risiko aufweist, einer oder mehreren Erbkrankheiten, für die die Bevölkerung von Sub-Sahara-Afrika ein erhöhtes Risiko aufweist, und einer oder mehreren Erbkrankheiten, für die die westeurasische Bevölkerung ein erhöhtes Risiko aufweist, entspricht.

**8.** Verfahren nach Anspruch 7, wobei:

(i) das erhöhte Risiko im Vergleich mit anderen Bevölkerungen zumindest 10-fach ist oder
(ii) die westeurasische Bevölkerung aus den europäischen, west-/zentralasiatischen, südasiatischen und nordafrikanischen Bevölkerungsuntergruppen ausgewählt ist oder
(iii) die ostasiatische Bevölkerung aus den chinesischen, japanischen und koreanischen Bevölkerungsuntergruppen ausgewählt ist.

**9.** Verfahren nach einem der Ansprüche 1-6, wobei die kausalen genetischen Varianten einer oder mehreren Erbkrankheiten, für die die amerikanische Ureinwohnerbevölkerung ein erhöhtes Risiko aufweist, oder einer oder mehreren Erbkrankheiten, für die die Bevölkerungsgruppe der aschkenasischen Juden ein erhöhtes Risiko aufweist, entsprechen.

10. Verfahren nach einem der vorangegangenen Ansprüche, wobei zumindest ein AIM keine kausale genetische Variante ist.

11. Verfahren nach einem der vorangegangenen Ansprüche, wobei die AIMs zumindest einen AIM, der zwischen afrikanischen und europäischen Bevölkerungsgruppen unterscheidet, zumindest einen AIM, der zwischen afrikanischen und asiatischen Bevölkerungsgruppen unterscheidet, und zumindest einen AIM, der zwischen europäischen und asiatischen Bevölkerungsgruppen unterscheidet, umfasst.

12. Verfahren nach Anspruch 11, wobei die AIMs zumindest einen AIM umfassen, der zwischen der afrikanischen und amerikanischen Bevölkerung; der europäischen und amerikanischen Bevölkerung, der asiatischen und amerikanischen Bevölkerung, der nordeuropäischen und südeuropäischen Bevölkerung, der nordeuropäischen und aschkenasisch-jüdischen Bevölkerung, der südeuropäischen und aschkenasisch-jüdischen Bevölkerung, der irischen und englischen Bevölkerung, der spanischen und kaukasischen Bevölkerung, der chinesischen und japanischen Bevölkerung oder der südasiatischen, zentralasiatischen und ostasiatischen Bevölkerung unterscheidet.

13. Verfahren nach einem der vorangegangenen Ansprüche, wobei die AIMs aus den AIMs aus Fig. 3 ausgewählt sind.

14. Verfahren nach Anspruch 1, wobei das Testen die Verwendung einer Nucleinsäureanordnung, Sequenzierung durch Hybridisierung, Sequenzierung durch Ligation, Sequenzierung durch Extension, Sanger-Sequenzierung, Maxam-Gilbert-Sequenzierung oder Pyrosequenzierung umfasst.

15. Computerlesbares Medium, das einen Computercode umfasst, der bei Ausführung die Wahrscheinlichkeit eines Phänotyps eines möglichen Kinds von der zukünftigen Mutter oder dem zukünftigen Vater in Bezug auf zumindest eine seltene Mendelsche Erbkrankheit vorhersagt, basierend zumindest teilweise auf den Ergebnissen eines Tests der zukünftigen Mutter oder des zukünftigen Vaters auf das Vorliegen einer Vielzahl kausaler genetischer Varianten und zumindest eines Abstammungsinformationsmarkers (AIM),
wobei die Wahrscheinlichkeit eines Phänotyps für das mögliche Kind in Übereinstimmung mit dem Verfahren aus einem der Ansprüche 1 bis 13 berechnet wird.

16. System zum Vorhersagen eines kindlichen Phänotyps, umfassend:

eine Mehrfach-Nucleinsäuredetektionsvorrichtung, die dazu konfiguriert ist, eine Vielzahl kausaler genetischer Varianten, die zumindest einer seltenen Mendelschen Erberkrankung entsprechen, und zumindest einen Abstammungsinformationsmarker (AIM) zu detektieren, wobei die Vorrichtung mit einer Probe von einer zukünftigen Mutter oder einem zukünftigen Vater eines möglichen Kindes in Kontakt steht; und
ein computerlesbares Medium nach Anspruch 15.

17. System zum Anzeigen, ob ein Individuum ein Träger einer seltenen Mendelschen Erbkrankheit ist, wobei das System umfasst:

ein Lesegerät, das dazu konfiguriert ist, Daten von einer Mehrfach-Nucleinsäuredetektionsvorrichtung, die dazu konfiguriert ist, eine Vielzahl kausaler genetischer Varianten, die zumindest einer seltenen Mendelschen Erberkrankung entsprechen, und zumindest einen Abstammungsinformationsmarker (AIM) zu detektieren, zu lesen; und
ein computerlesbares Medium nach Anspruch 15.

18. Verfahren umfassend:

das Erhalten einer Probe von einem Benutzer;
das Testen der Probe mit einer Mehrfach-Nucleinsäuredetektionsvorrichtung, die dazu konfiguriert ist, auf eine Vielzahl kausaler genetischer Varianten seltener Mendelscher Erbkrankheiten und zumindest einen Abstammungsinformationsmarker (AIM) zu testen;
das Berechnen einer Vielzahl von Wahrscheinlichkeiten für die möglichen Genotypen des Benutzers an jeder Stelle der Vielzahl kausaler genetischer Varianten, basierend auf den Ergebnissen aus dem Testungsschritt in Bezug auf die Vielzahl kausaler genetischer Varianten und den zumindest einen AIM,
wobei das Berechnen das Durchführen einer vollständigen Wahrscheinlichkeitsanalyse auf den zu diesen kausalen genetischen Varianten gesammelten Daten umfasst, wobei die von dem zumindest einen AIM über die Abstammung des Benutzers erhaltene Information verwendet wird, um die Wahrscheinlichkeit einer falschen Klassifizierung der kausalen genetischen Varianten in der Mehrfach-Nucleinsäuredetektionsvorrichtung zu reduzieren; und
das Bereitstellen der Vielzahl von Wahrscheinlichkeiten, die dem Benutzer entsprechen, der ein Träger ist, an den Benutzer.

19. Verfahren nach einem der Ansprüche 1 bis 14, wobei zumindest zwei der seltenen Mendelschen Erbkrankheiten mit einer Häufigkeit, die sich zumindest um das 10-fache in zumindest zwei getrennten Bevölkerungen unterscheidet, auftreten, wobei die zumindest zwei getrennten Bevölkerungen sich um den zumindest einen AIM unterscheiden.

**Revendications**

1. Procédé comprenant :

a) l'utilisation d'un test génétique multiplexe pour le test d'une mère prospective ou d'un père prospectif d'un enfant potentiel afin de déterminer la présence d'une pluralité de variants génétiques causaux, chaque variant correspondant à au moins une maladie génétique mendélienne rare, et la présence d'au moins un marqueur informatif d'ascendance (MIA) ; et
b) la prédiction de la probabilité d'un phénotype de l'enfant potentiel par rapport à la au moins une maladie génétique mendélienne rare basée, en moins en partie, sur la présence de la pluralité de variants génétiques causaux et la présence du au moins un MIA, où ladite prédiction comprend la réalisation d'une analyse entièrement probabiliste sur des données recueillies sur lesdits variants génétiques causaux, où les informations obtenues à partir de la présence du au moins MIA de la mère prospective ou du père prospectif sont utilisées pour réduire une probabilité d'erreur de classification desdits variants génétiques causaux dans le test génétique multiplexe.

2. Procédé selon l'une quelconque des revendications précédentes, dans lequel la prédiction à l'étape b) est en outre basée sur des informations phénotypiques concernant la mère prospective ou le père prospectif.

3. Procédé selon l'une quelconque des revendications précédentes, comprenant le test d'une mère prospective et d'un père prospectif de l'enfant potentiel.

4. Procédé selon l'une quelconque des revendications précédentes, dans lequel la pluralité de maladies génétiques possède chacune une fréquence inférieure à 1 pour 1000 chez les humains.

5. Procédé selon l'une quelconque des revendications précédentes, dans lequel la pluralité de maladies génétiques rares est de plus de 85 maladies génétiques mendéliennes rares.

6. Procédé selon la revendication 5, dans lequel la pluralité des maladies est sélectionnée parmi la fibrose kystique, la maladie de Tay-Sachs, un déficit en 21-hydroxylase, l'hyperinsulinisme lié à ABCC8, l'ARSACS, l'achondroplasie, l'achromatopsie, un déficit en adénosine monophosphate désaminase 1, une neuropathie avec agénésie du corps calleux, l'alcaptonurie, un déficit en alpha-1-antitrypsine, l'alpha-mannosidose, l'alpha-sarcoglycanopathie, l'alpha-thalassémie, un déficit en récepteur de l'angiotensine II de type 1, l'acidurie argininosuccinique, l'aspartylglucosaminurie, l'ataxie avec déficit en vitamine E, l'ataxie-télangiectasie, le syndrome de polyendocrinopathie auto-immune de type 1, le syndrome de Bardet-Biedl, la dystrophie maculaire vitelliforme de Best, la bêta-sarcoglycanopathie, la bêta-thalassémie, un déficit en biotinidase, le syndrome de Blau, le syndrome de Bloom, des troubles liés à CFTR, la céroïde-lipofuscinose neuronale liée à CLN3, la céroïde-lipofuscinose neuronale liée à CLN5, la céroïde-lipofuscinose neuronale liée à CLN8, la maladie de Canavan, un déficit en carnitine palmitoyltransférase IA, un déficit en carnitine palmitoyltransférase II, l'hypoplasie cartilage-cheveux, la choroïdérémie, le syndrome de Cohen, des cataractes congénitales avec dysmorphie faciale et neuropathie, un trouble congénital de la glycosylation, un trouble congénital de la glycosylation de type Ib, le syndrome néphrotique congénital de type finlandais, la cystinose, DFNA 9 (Coch), la dystonie primaire à début précoce (DYTI), l'épidermolyse bulleuse jonctionnelle de type Herlitz-Pearson, l'anémie de Fanconi liée à FANCC, la craniosynostose liée à FGFR1, la craniosynostose liée à FGFR2, la craniosynostose liée à FGFR3, la thrombophilie par mutation du facteur V Leiden, la thrombophilie par mutation du facteur V R2, un déficit en facteur XI, un déficit en facteur XIII, la dysautonomie familiale, l'hypercholestérolémie familiale de type B, la fièvre méditerranéenne familiale, des troubles du stockage de l'acide sialique libre, la démence fronto-temporale avec parkinsonisme liée au chromosome 17, un déficit en fumarase, une perte d'audition et surdité non syndromique (DFNA3) liées à GJB2, une perte d'audition et surdité non syndromique (DFNB1) liées à GJB2, des myopathies liées à GNE, la galactosémie, la maladie de Gaucher, un déficit en glucose-6-phosphate déshydrogénase, l'acidurie glutarique de type 1, la maladie de stockage du glycogène de type 1a, la maladie de stockage du glycogène de type 1b, la maladie de stockage du glycogène de type II, la maladie de stockage du glycogène de type III, la maladie de stockage du glycogène de type V, le syndrome Gracile, l'hémochromatose héréditaire liée à HFE, l'hémoglobine S - bêta-thalassémie, l'intolérance héréditaire au fructose, la pancréatite héréditaire, la thymine-uracilurie héréditaire, un déficit en hexosaminidase A, la dysplasie ectodermique hidrotique de type 2, l'homocystinurie due à un déficit en cystathionine bêta-synthase, la paralysie périodique hyperkaliémique de type 1, le syndrome de l'hyperornithinémie-hyperammoniémie-homocitrullinurie, l'hyperoxalurie primitive de type 1, l'hyperoxalurie primitive de type 2, l'hypochondroplasie, la paralysie périodique hypokaliémique de type 1, la paralysie périodique hypokaliémique de type 2, l'hypophosphatasie, l'acidémie isovalérique, la maladie de Krabbe, la LGMD2I de type franco-canadien, un déficit en 3-hydroxyacyl-CoA déshydrogénase des acides gras à longue chaîne, un déficit en MTHFR, un variant thermolabile de MTHFR, une perte d'audition et une surdité liées à MTTS1, la polypose liée à MYH, la maladie des urines à odeur de sirop d'érable de type 1A, la maladie des urines à odeur de sirop d'érable de type 1B, un déficit en acyl-coenzyme A déshydrogénase des acides gras à chaîne moyenne, la leucoencéphalopathie mégalencéphalique avec kystes sous-corticaux, la leucodystrophie métachromatique, la mucolipidose de type IV, la mucopolysaccharidose de type I, la mucopolysaccharidose de type IIIA, la mucopolysaccharidose de type VII, la néoplasie endocrinienne multiple de type 2, la maladie muscle-oeil-cerveau, la myopathie à némaline, la maladie de Niemann-Pick due à un déficit en sphingomyélinase, la maladie de Niemann-Pick de type C1, le syndrome de rupture de Nimègue, la céroïde-lipofuscinose neuronale liée à PPT1, un déficit hypophysaire lié à PROP1, le syndrome de Pallister-Hall, la paramyotonie congénitale, le syndrome de Pendred, un déficit en enzyme bifonctionnelle peroxysomale, un déficit en phénylalanine hydroxylase, un déficit en inhibiteur de type I de l'activateur de plasminogène, la maladie polykystique des reins autosomique récessive, la thrombophilie par mutation G20210A du gène de la prothrombine, la pycnodysostose, la rétinite pigmentaire autosomique récessive de type de Bothnia, le syndrome de Rett, la chondrodysplasie ponctuée rhizomélique de type 1, un déficit en acyl-CoA déshydrogénase des acides gras à courte chaîne, le syndrome de Shwachman-Diamond, le syndrome de Sjögren-Larsson, le syndrome de Smith-Lemli-Opitz, la paraplégie spastique de type 13, l'ostéochondrodysplasie liée aux transporteurs du sulfate, l'hémochromatose héréditaire liée à TFR2, la céroïde-lipofuscinose neuronale liée à TPP1, la dysplasie thanatophore, l'amylose à transthyrétine, un déficit en protéine trifonctionnelle, la dystonie dopa-sensible (DRD) par déficit en tyrosine hydroxylase, la tyrosinémie de type 1, la maladie de Wilson, le rétinoschisis juvénile liée à l'X et le spectre du syndrome de Zellweger.

7. Procédé selon l'une quelconque des revendications précédentes, dans lequel la pluralité de variants génétiques causaux correspond à une ou plusieurs maladies génétiques pour lesquelles la population d'Asie orientale est exposée à un risque accru, une ou plusieurs maladies génétiques pour lesquelles la population d'Afrique subsaharienne est exposée à un risque accru et une ou plusieurs maladies génétiques pour lesquelles la population d'Eurasie occidentale est exposée à un risque accru.

8. Procédé selon la revendication 7, dans lequel :

   (i) le risque accru est d'au moins 10 fois par comparaison à une autre des populations, ou
   (ii) la population d'Eurasie occidentale est sélectionnée parmi des sous-populations européennes, d'Asie occidentale/centrale, d'Asie du Sud et d'Afrique du Nord, ou
   (iii) la population d'Asie orientale est sélectionnée parmi des sous-populations chinoises, japonaises et coréennes.

9. Procédé selon l'une quelconque des revendications 1 à 6, dans lequel les variants génétiques causaux correspondent à une ou plusieurs maladies génétiques pour lesquelles la population amérindienne est exposée à un risque accru ou une ou plusieurs maladies génétiques pour lesquelles la population juive ashkénaze est exposée à un risque accru.

10. Procédé selon l'une quelconque des revendications précédentes, dans lequel au moins MIA n'est pas un variant génétique causal.

11. Procédé selon l'une quelconque des revendications précédentes, dans lequel les MIA comprennent au moins un MIA qui différencie les populations africaines et européennes, au moins un MIA qui différencie les populations africaines et asiatiques et au moins un MIA qui différencie les populations européennes et asiatiques.

12. Procédé selon la revendication 11, dans lequel les MIA comprennent au moins un MIA qui différencie les populations africaines et américaines, les populations européennes et américaines, les populations asiatiques et américaines, les populations d'Europe du Nord et d'Europe du Sud, les populations d'Europe du Nord et juive ashkénaze, les populations d'Europe du Sud et juive ashkénaze, les populations irlandaises et anglaises, les populations espagnoles et caucasiennes, les populations chinoises et japonaises, ou les populations d'Asie du Sud, d'Asie centrale et d'Asie de l'Est.

13. Procédé selon l'une quelconque des revendications précédentes, dans lequel les MIA sont sélectionnés parmi les MIA de la Figure 3.

14. Procédé selon la revendication 1, dans lequel le test comprend l'utilisation d'un réseau d'acide nucléique, un séquençage par hybridation, un séquençage par ligature, un séquençage par extension, un séquençage de Sanger, un séquençage de Maxam-Gilbert ou un pyroséquençage.

15. Support pouvant être lu par ordinateur comprenant un code informatique qui, lorsqu'il est exécuté, prédit la probabilité d'un phénotype d'un enfant potentiel, à partir de la mère prospective ou du père prospectif, par rapport à au moins une maladie génétique mendélienne rare, basée au moins en partie sur les résultats d'un test de la mère prospective ou du père prospectif pour déterminer la présence d'une pluralité de variants génétiques causaux et d'au moins un marqueur informatif d'ascendance (MIA), où la probabilité d'un phénotype de l'enfant potentiel est calculée conformément au procédé selon l'une quelconque des revendications 1 à 13.

16. Système pour prédire le phénotype d'un enfant comprenant :

un dispositif de détection d'acide nucléique multiplexe configuré pour détecter une pluralité de variants génétiques causaux correspondant à au moins une maladie génétique mendélienne rare et au moins un marqueur informatif d'ascendance (MIA), où le dispositif est en contact avec un échantillon issu d'une mère prospective ou d'un père prospectif d'un enfant potentiel ;
un lecteur configuré pour lire les données des dispositifs ; et
un support pouvant être lu par ordinateur selon la revendication 15.

17. Système pour indiquer si un sujet est porteur d'une maladie génétique mendélienne rare comprenant : un lecteur configuré pour lire les données d'un dispositif de détection d'acide nucléique multiplexe configuré pour détecter une pluralité de variants génétiques causaux correspondant à au moins une maladie génétique mendélienne rare et au moins un marqueur informatif d'ascendance (MIA) ; et un support pouvant être lu par ordinateur selon la revendication 15.

18. Procédé comprenant :

la réception d'un échantillon d'un utilisateur ;
le test de l'échantillon avec un dispositif de détection d'acide nucléique multiplexe configuré pour tester une pluralité de variants génétiques causaux de maladies génétiques mendéliennes rares et au moins un marqueur informatif d'ascendance (MIA) ;
le calcul d'une pluralité de probabilités pour les génotypes possibles de l'utilisateur à chaque emplacement de la pluralité de variants génétiques causaux basé sur les résultats de l'étape de test concernant la pluralité de variants génétiques causaux et le au moins un MIA,
où ledit calcul comprend la réalisation d'une analyse entièrement probabiliste les données recueillies sur lesdits variants génétiques causaux, où les informations obtenues à partir du au moins un MIA concernant l'ascendance de l'utilisateur sont utilisées pour réduire une probabilité d'erreur de classification desdits variants génétiques causaux dans le dispositif de détection d'acide nucléique multiplexe ; et
la mise à disposition pour l'utilisateur de la pluralité de probabilités correspondant au fait que l'utilisateur est un porteur.

19. Procédé selon l'une quelconque des revendications 1 à 14, dans lequel au moins deux des maladies génétiques mendéliennes rares surviennent à des fréquences qui diffèrent d'au moins 10 fois dans au moins deux populations distinctes, où les au moins deux populations distinctes sont différenciées par le au moins un MIA.

## FIGURE 1 – Table of Exemplary Causal Genetic Variants

| Disease | Gene | Variant Name |
|---|---|---|
| 21-Hydroxylase Deficiency | CYP21A2 | F306+t |
| 21-Hydroxylase Deficiency | CYP21A2 | F306+t |
| 21-Hydroxylase Deficiency | CYP21A3 | g.655A/C>G |
| 21-Hydroxylase Deficiency | CYP21A4 | g.655A/C>G |
| 21-Hydroxylase Deficiency | CYP21A6 | G110del8nt |
| 21-Hydroxylase Deficiency | CYP21A5 | G110del8nt |
| 21-Hydroxylase Deficiency | CYP21A7 | I172N, rs34607927 |
| 21-Hydroxylase Deficiency | CYP21A2 | I236N |
| 21-Hydroxylase Deficiency | CYP21A2 | M239K, rs6476 |
| 21-Hydroxylase Deficiency | CYP21A2 | P30L |
| 21-Hydroxylase Deficiency | CYP21A2 | P453S |
| 21-Hydroxylase Deficiency | CYP21A2 | Q318X |
| 21-Hydroxylase Deficiency | CYP21A2 | R356W |
| 21-Hydroxylase Deficiency | CYP21A2 | V237E, rs12530380 |
| 21-Hydroxylase Deficiency | CYP21A2 | V281L, rs6471 |
| ABCC8-Related Hyperinsulinism | ABCC8 | 3992-9G>A |
| ABCC8-Related Hyperinsulinism | ABCC8 | delF1388 |
| ABCC8-Related Hyperinsulinism | ABCC8 | delF1388 |
| ABCC8-Related Hyperinsulinism | ABCC8 | V187D |
| Achondroplasia | FGFR3 | G375C |
| Achondroplasia | FGFR3 | G380R, rs28931614 |
| Achromatopsia | CNGB3 | c.1148delC |
| Achromatopsia | CNGB3 | c.1148delC |
| Achromatopsia | CNGB3 | c.819-826del8 |
| Achromatopsia | CNGB3 | c.819-826del8 |
| Achromatopsia | CNGB3 | c.886-896del11insT |
| Achromatopsia | CNGB3 | c.886-896del11insT |
| Achromatopsia | CNGB3 | c.991-3T>G |
| Achromatopsia | CNGB3 | p.Arg403Gln |
| Achromatopsia | CNGB3 | p.Glu336X |
| Adenosine Monophosphate Deaminase 1 | AMPD1 | P48L |
| Adenosine Monophosphate Deaminase 1 | AMPD1 | Q12X, rs17602729 |
| Agenesis of Corpus Callosum with Neuronopathy | SLC12A6 | c.2436delG |
| Agenesis of Corpus Callosum with Neuronopathy | SLC12A6 | c.2436delG |
| Alkaptonuria | HGD | c.174delA |
| Alkaptonuria | HGD | c.174delA |
| Alkaptonuria | HGD | c.457_458insG |
| Alkaptonuria | HGD | c.457_458insG |
| Alkaptonuria | HGD | G161R |
| Alkaptonuria | HGD | G270R |

## FIGURE 1 – Table of Exemplary Causal Genetic Variants

| Disease | Gene | Variant Name |
|---|---|---|
| Alkaptonuria | HGD | IVS1-1G>A |
| Alkaptonuria | HGD | IVS5+1G>A |
| Alkaptonuria | HGD | Met368Val |
| Alkaptonuria | HGD | P230S |
| Alkaptonuria | HGD | S47L |
| Alkaptonuria | HGD | V300G |
| Alpha-1-Antitrypsin Deficiency | SERPINA1 | Arg101His, rs709932 |
| Alpha-1-Antitrypsin Deficiency | SERPINA1 | Glu264Val |
| Alpha-1-Antitrypsin Deficiency | SERPINA1 | Glu342Lys, rs28929474 |
| Alpha-1-Antitrypsin Deficiency | SERPINA1 | Glu376Asp, rs1303 |
| Alpha-Mannosidosis | MAN2B1 | IVS14+1G>C |
| Alpha-Mannosidosis | MAN2B1 | p.L809P |
| Alpha-Mannosidosis | MAN2B1 | p.R750W |
| Alpha-Sarcoglycanopathy | SGCA | R77C, rs28933693 |
| Alpha-Thalassemia | HBA2 | H19D |
| Alpha-Thalassemia | HBA1 | HbQ |
| Alpha-Thalassemia | HBA1,HBA2 | 3.7 kb (type I) deletion alpha-2 |
| Alpha-Thalassemia | HBA1,HBA2 | 3.7 kb (type I) deletion alpha-2 |
| Alpha-Thalassemia | HBA1,HBA2 | HbVar database id # 1086, --(SEA); deletion of ~20 kb including both alpha-globin genes alpha-Thal-1 |
| Alpha-Thalassemia | HBA1,HBA2 | HbVar database id # 1086, --(SEA); deletion of ~20 kb including both alpha-globin genes alpha-Thal-1 |
| Alpha-Thalassemia | HBA1,HBA2 | HbVar database id # 1087, --(MED-I); deletion of ~17.5 kb including both alpha-globin genes alpha-Thal-1 |
| Alpha-Thalassemia | HBA1,HBA2 | HbVar database id # 1087, --(MED-I); deletion of ~17.5 kb including both alpha-globin genes alpha-Thal-1 |
| Alpha-Thalassemia | HBA1,HBA2 | HbVar database id # 1088, -(alpha)20.5; this 20.5 kb deletion involves alpha2 and the 5' end of alpha1; alpha-Thal-1 |
| Alpha-Thalassemia | HBA1,HBA2 | HbVar database id # 1088, -(alpha)20.5; this 20.5 kb deletion involves alpha2 and the 5' end of alpha1; alpha-Thal-1 |
| Alpha-Thalassemia | HBA1,HBA2 | HbVar database id # 1094, --(FIL); a deletion of 30-34 kb involving the alpha1, alpha2, and zeta genes alpha-Thal-1 |
| Alpha-Thalassemia | HBA1,HBA2 | HbVar database id # 1094, --(FIL); a deletion of 30-34 kb involving the alpha1, alpha2, and zeta genes alpha-Thal-1 |

## FIGURE 1 – Table of Exemplary Causal Genetic Variants

| Disease | Gene | Variant Name |
|---|---|---|
| Alpha-Thalassemia | HBA1,HBA2 | HbVar database id # 1095, -- (THAI); a deletion of 34-38 kb involving the alpha1, alpha2, and zeta genes alpha-Thal-1 |
| Alpha-Thalassemia | HBA1,HBA2 | HbVar database id # 1095, -- (THAI); a deletion of 34-38 kb involving the alpha1, alpha2, and zeta genes alpha-Thal-1 |
| Alpha-Thalassemia | HBA1,HBA2 | HbVar database id # 1097, --(MED-II); a deletion of 26.5 kb involving the two alpha and zeta genes alpha-Thal-1 |
| Alpha-Thalassemia | HBA1,HBA2 | HbVar database id # 1097, --(MED-II); a deletion of 26.5 kb involving the two alpha and zeta genes alpha-Thal-1 |
| Alpha-Thalassemia | HBA2 | HbVar database id # 187 |
| Alpha-Thalassemia | HBA2 | HbVar database id # 2598, IVS I-5 (G>A) |
| Alpha-Thalassemia | HBA1,HBA2 | HbVar database id # 703 |
| Alpha-Thalassemia | HBA1,HBA2 | HbVar database id # 704 |
| Alpha-Thalassemia | HBA1,HBA2 | HbVar database id # 705, Hb Koya Dora |
| Alpha-Thalassemia | HBA1,HBA2 | HbVar database id # 707, rs41412046 |
| Alpha-Thalassemia | HBA1 | HbVar database id # 87 |
| Alpha-Thalassemia | HBA1,HBA2 | HbVar database id # 969, Poly A (A->G); AATAAA->AATGAA beta+ |
| Alpha-Thalassemia | HBA1,HBA2 | HbVar database id # 971, Poly A (A->G); AATAAA->AATAAG beta+ |
| Alpha-Thalassemia | HBA2 | M1T |
| Alpha-Thalassemia | HBA1 | W14X |
| Angiotensin II Receptor, Type 1 | AGTR1 | A1166C |
| Apolipoprotein E Genotyping | APOE | p.C112R, rs429358 |
| Apolipoprotein E Genotyping | APOE | p.R158C, rs7412 |
| Argininosuccinicaciduria | ASL | R385C |
| ARSACS | SACS | 5254C>T |
| ARSACS | SACS | 6594delT |
| ARSACS | SACS | 6594delT |
| Aspartylglycosaminuria | AGA | c.199_200delGA |
| Aspartylglycosaminuria | AGA | c.199_200delGA |
| Aspartylglycosaminuria | AGA | C163S |
| Ataxia with Vitamin E Deficiency | TTPA | 744delA |
| Ataxia with Vitamin E Deficiency | TTPA | 744delA |
| Ataxia-Telangiectasia | ATM | R35X |

## FIGURE 1 – Table of Exemplary Causal Genetic Variants

| Disease | Gene | Variant Name |
|---|---|---|
| Autoimmune Polyendocrinopathy Syndrome Type 1 | AIRE | c.1163_1164insA |
| Autoimmune Polyendocrinopathy Syndrome Type 1 | AIRE | c.1163_1164insA |
| Autoimmune Polyendocrinopathy Syndrome Type 1 | AIRE | c.769C>T |
| Autoimmune Polyendocrinopathy Syndrome Type 1 | AIRE | c.967_979del |
| Autoimmune Polyendocrinopathy Syndrome Type 1 | AIRE | c.967_979del |
| Autoimmune Polyendocrinopathy Syndrome Type 1 | AIRE | Y85C |
| Bardet-Biedl Syndrome | BBS1 | M390R |
| Bardet-Biedl Syndrome | BBS10 | p.C91LfsX4 |
| Bardet-Biedl Syndrome | BBS10 | p.C91LfsX4 |
| Best Vitelliform Macular Dystrophy | BEST1 | c.G383C |
| Beta-Sarcoglycanopathy | SGCB | S114F |
| Beta-Thalassemia | HBB | -28 (A->G) beta+ |
| Beta-Thalassemia | HBB | -29 (A->G) beta+ |
| Beta-Thalassemia | HBB | -29A>G |
| Beta-Thalassemia | HBB | -30 (T->A) beta+ |
| Beta-Thalassemia | HBB | -87 (C->G) beta+ |
| Beta-Thalassemia | HBB | -88C>T |
| Beta-Thalassemia | HBB | CAP+1 (A->C) beta+ |
| Beta-Thalassemia | HBB | Codon 15 (G->A); TGG(Trp)->TAG(stop codon) beta0, rs34716011 |
| Beta-Thalassemia | HBB | Codon 15 (G->A); TGG(Trp)->TAG(stop codon) beta0, rs34716011 |
| Beta-Thalassemia | HBB | Codon 16 (-C); GGC(Gly)->GG- beta0 |
| Beta-Thalassemia | HBB | Codon 16 (-C); GGC(Gly)->GG- beta0 |
| Beta-Thalassemia | HBB | Codon 17 (A->T); AAG(Lys)->TAG(stop codon) beta0 |
| Beta-Thalassemia | HBB | Codon 24 (T->A); GGT(Gly)->GGA(Gly) beta+ |
| Beta-Thalassemia | HBB | Codon 39 (C->T); CAG(Gln)->TAG(stop codon) beta0 |
| Beta-Thalassemia | HBB | Codon 5 (-CT); CCT(Pro)->C-- beta0 |
| Beta-Thalassemia | HBB | Codon 5 (-CT); CCT(Pro)->C-- beta0 |
| Beta-Thalassemia | HBB | Codon 6 (-A); GAG(Glu)->G-G beta0 |

## FIGURE 1 – Table of Exemplary Causal Genetic Variants

| Disease | Gene | Variant Name |
|---|---|---|
| Beta-Thalassemia | HBB | Codon 6 (-A); GAG(Glu)->G-G beta0 |
| Beta-Thalassemia | HBB | Codon 8 (-AA); AAG(Lys)->--G beta0 |
| Beta-Thalassemia | HBB | Codon 8 (-AA); AAG(Lys)->--G beta0 |
| Beta-Thalassemia | HBB | Codons 41/42 (-TTCT); TTCTTT(Phe-Phe)->----TT beta0 |
| Beta-Thalassemia | HBB | Codons 41/42 (-TTCT); TTCTTT(Phe-Phe)->----TT beta0 |
| Beta-Thalassemia | HBB | Codons 71/72 (+A); TTT AGT(Phe Ser)->TTT A AGT; beta0 |
| Beta-Thalassemia | HBB | Codons 71/72 (+A); TTT AGT(Phe Ser)->TTT A AGT; beta0 |
| Beta-Thalassemia | HBB | Codons 8/9 (+G); AAG TCT(Lys;Ser)->AAG G TCT beta0 |
| Beta-Thalassemia | HBB | Codons 8/9 (+G); AAG TCT(Lys;Ser)->AAG G TCT beta0 |
| Beta-Thalassemia | HBB | HbVar database id # 889, IVS-II-654 (C->T); AAGGCAATA->AAG^GTAATA beta+(severe) |
| Beta-Thalassemia | HBB | HbVar database id # 890, IVS-II-705 (T->G); GATGTAAGA->GAG^GTAAGA beta+ |
| Beta-Thalassemia | HBB | HbVar database id # 891, IVS-II-745 (C->G); CAGCTACCAT->CAG^GTACCAT beta+ |
| Beta-Thalassemia | HBB | HbVar database id # 979, 619 bp deletion beta0 |
| Beta-Thalassemia | HBB | 619 bp deletion beta0 |
| Beta-Thalassemia | HBB | IVS-I-1 (G->A); AG^GTTGGT->AGATTGGT beta0 |
| Beta-Thalassemia | HBB | IVS-I-1 (G->T); AG^GTTGGT->AGTTTGGT beta0 |
| Beta-Thalassemia | HBB | IVS-I-110 (G->A) beta+; the mutation is 21 nucleotides 5' to the acceptor splice site AG^GC |
| Beta-Thalassemia | HBB | IVS-I-5 (G->C) beta+(severe) |
| Beta-Thalassemia | HBB | IVS-II-1 (G->A); beta0 |
| Beta-Thalassemia | HBB | IVS-II-844 (C->G); beta+ |
| Beta-Thalassemia | HBB | IVS1+6T>C |
| Beta-Thalassemia | HBB | IVS11-849A>C |
| Beta-Thalassemia | HBB | IVS11-849A>G |
| Biotinidase Deficiency | BTD | A171T, rs13073139 |
| Biotinidase Deficiency | BTD | D252G, rs28934601 |
| Biotinidase Deficiency | BTD | D444H, rs13078881 |
| Biotinidase Deficiency | BTD | F403V |
| Biotinidase Deficiency | BTD | G98:d7i3 |
| Biotinidase Deficiency | BTD | G98:d7i3 |
| Biotinidase Deficiency | BTD | Q456H |
| Biotinidase Deficiency | BTD | R157H |
| Biotinidase Deficiency | BTD | R538C |

## FIGURE 1 – Table of Exemplary Causal Genetic Variants

| Disease | Gene | Variant Name |
|---|---|---|
| Blau Syndrome | NOD2 | E383K |
| Blau Syndrome | NOD2 | L469F |
| Blau Syndrome | NOD2 | R334Q |
| Blau Syndrome | NOD2 | R334W |
| Bloom Syndrome | BLM | 2407insT |
| Bloom Syndrome | BLM | 2407insT |
| Bloom Syndrome | BLM | 736delATCTGAinsTAGATTC (2281del6/ins7) |
| Bloom Syndrome | BLM | 736delATCTGAinsTAGATTC (2281del6/ins7) |
| BRCA1 Hereditary Breast/Ovarian Cancer | BRCA1 | 185delAG |
| BRCA1 Hereditary Breast/Ovarian Cancer | BRCA1 | 185delAG |
| BRCA1 Hereditary Breast/Ovarian Cancer | BRCA1 | 5382insC |
| BRCA1 Hereditary Breast/Ovarian Cancer | BRCA1 | 5382insC |
| BRCA1 Hereditary Breast/Ovarian Cancer | BRCA1 | Tyr978X |
| BRCA2 Hereditary Breast/Ovarian Cancer | BRCA2 | 6174delT |
| BRCA2 Hereditary Breast/Ovarian Cancer | BRCA2 | 6174delT |
| BRCA2 Hereditary Breast/Ovarian Cancer | BRCA2 | 8765delAG |
| BRCA2 Hereditary Breast/Ovarian Cancer | BRCA2 | 8765delAG |
| Canavan Disease | ASPA | A305E (914C>A), rs28940574 |
| Canavan Disease | ASPA | E285A (854A>C), rs28940279 |
| Canavan Disease | ASPA | IVS2-2A>G (433-2A>G) |
| Canavan Disease | ASPA | Y231X (693C>A) |
| Carnitine Palmitoyltransferase IA Deficiency | CPT1A | G710E |
| Carnitine Palmitoyltransferase IA Deficiency | CPT1A | P479L |
| Carnitine Palmitoyltransferase II Deficiency | CPT2 | G549D |
| Carnitine Palmitoyltransferase II Deficiency | CPT2 | L178F 534 ins/25 bp del |
| Carnitine Palmitoyltransferase II Deficiency | CPT2 | L178F 534 ins/25 bp del |
| Carnitine Palmitoyltransferase II Deficiency | CPT2 | P227L |
| Carnitine Palmitoyltransferase II Deficiency | CPT2 | P50H |

# FIGURE 1 – Table of Exemplary Causal Genetic Variants

| Disease | Gene | Variant Name |
|---|---|---|
| Carnitine Palmitoyltransferase II Deficiency | CPT2 | P604S |
| Carnitine Palmitoyltransferase II Deficiency | CPT2 | Q413fs |
| Carnitine Palmitoyltransferase II Deficiency | CPT2 | Q413fs |
| Carnitine Palmitoyltransferase II Deficiency | CPT2 | Q550R |
| Carnitine Palmitoyltransferase II Deficiency | CPT2 | R124X |
| Carnitine Palmitoyltransferase II Deficiency | CPT2 | R503C |
| Carnitine Palmitoyltransferase II Deficiency | CPT2 | R631C |
| Carnitine Palmitoyltransferase II Deficiency | CPT2 | S113L |
| Carnitine Palmitoyltransferase II Deficiency | CPT2 | s38fs |
| Carnitine Palmitoyltransferase II Deficiency | CPT2 | s38fs |
| Carnitine Palmitoyltransferase II Deficiency | CPT2 | Y628S, rs28936673 |
| Cartilage-Hair Hypoplasia | RMRP | g.262G>T |
| Cartilage-Hair Hypoplasia | RMPR | g.70A>G |
| CFTR-Related Disorders | CFTR | 1811+1.6kbA->G |
| CFTR-Related Disorders | CFTR | 2183AA>G |
| CFTR-Related Disorders | CFTR | 2183AA>G |
| CFTR-Related Disorders | CFTR | 3849+10kbC>T |
| CFTR-Related Disorders | CFTR | A455E |
| CFTR-Related Disorders | CFTR | A559T |
| CFTR-Related Disorders | CFTR | C524X |
| CFTR-Related Disorders | CFTR | 574delA, 574delA |
| CFTR-Related Disorders | CFTR | 574delA, 574delA |
| CFTR-Related Disorders | CFTR | 2108delA, 2108delA |
| CFTR-Related Disorders | CFTR | 2108delA, 2108delA |
| CFTR-Related Disorders | CFTR | 3171delC, 3171delC |
| CFTR-Related Disorders | CFTR | 3171delC, 3171delC |
| CFTR-Related Disorders | CFTR | 621+1G->T |
| CFTR-Related Disorders | CFTR | 2105-2117del13insAGAAA |
| CFTR-Related Disorders | CFTR | 2105-2117del13insAGAAA |
| CFTR-Related Disorders | CFTR | 711+1G->T |

## FIGURE 1 – Table of Exemplary Causal Genetic Variants

| Disease | Gene | Variant Name |
|---|---|---|
| CFTR-Related Disorders | CFTR | 711+5G->A |
| CFTR-Related Disorders | CFTR | 712-1G->T |
| CFTR-Related Disorders | CFTR | 1288insTA, 1288insTA |
| CFTR-Related Disorders | CFTR | 1288insTA, 1288insTA |
| CFTR-Related Disorders | CFTR | 936delTA |
| CFTR-Related Disorders | CFTR | 936delTA |
| CFTR-Related Disorders | CFTR | [delta]F311 |
| CFTR-Related Disorders | CFTR | [delta]F311 |
| CFTR-Related Disorders | CFTR | 1078delT, 1078delT |
| CFTR-Related Disorders | CFTR | 1078delT, 1078delT |
| CFTR-Related Disorders | CFTR | 1161delC, 1161delC |
| CFTR-Related Disorders | CFTR | 1161delC, 1161delC |
| CFTR-Related Disorders | CFTR | 1609delCA, 1609delCA |
| CFTR-Related Disorders | CFTR | 1609delCA, 1609delCA |
| CFTR-Related Disorders | CFTR | [delta]I507 |
| CFTR-Related Disorders | CFTR | [delta]I507 |
| CFTR-Related Disorders | CFTR | rs332, [delta]F508 |
| CFTR-Related Disorders | CFTR | rs332, [delta]F508 |
| CFTR-Related Disorders | CFTR | 1677delTA, 1677delTA |
| CFTR-Related Disorders | CFTR | 1677delTA, 1677delTA |
| CFTR-Related Disorders | CFTR | 1717-1G->A |
| CFTR-Related Disorders | CFTR | 1812-1G->A |
| CFTR-Related Disorders | CFTR | 1898+1G->A |
| CFTR-Related Disorders | CFTR | 1898+1G->T |
| CFTR-Related Disorders | CFTR | 1898+5G->T |
| CFTR-Related Disorders | CFTR | 1949del84, 1949del84 |
| CFTR-Related Disorders | CFTR | 1949del84, 1949del84 |
| CFTR-Related Disorders | CFTR | 2043delG, 2043delG |
| CFTR-Related Disorders | CFTR | 2043delG, 2043delG |
| CFTR-Related Disorders | CFTR | 2055del9->A |
| CFTR-Related Disorders | CFTR | 2055del9->A |
| CFTR-Related Disorders | CFTR | 2143delT, 2143delT |
| CFTR-Related Disorders | CFTR, | 2143delT, 2143delT |
| CFTR-Related Disorders | CFTR | 2184delA, 2184delA |
| CFTR-Related Disorders | CFTR | 2184delA, 2184delA |
| CFTR-Related Disorders | CFTR | 2184insA, 2184insA |
| CFTR-Related Disorders | CFTR | 2184insA, 2184insA |
| CFTR-Related Disorders | CFTR | 2307insA, 2307insA |
| CFTR-Related Disorders | CFTR | 2307insA, 2307insA |
| CFTR-Related Disorders | CFTR | 296+12T->C |
| CFTR-Related Disorders | CFTR | 2789+5G->A |
| CFTR-Related Disorders | CFTR | 2869insG, 2869insG |
| CFTR-Related Disorders | CFTR | 2869insG, 2869insG |
| CFTR-Related Disorders | CFTR | 3120G->A |
| CFTR-Related Disorders | CFTR | 3120+1G->A |
| CFTR-Related Disorders | CFTR | 3272-26A->G |
| CFTR-Related Disorders | CFTR | 3659delC, 3659delC |
| CFTR-Related Disorders | CFTR | 3659delC, 3659delC |

41

## FIGURE 1 – Table of Exemplary Causal Genetic Variants

| Disease | Gene | Variant Name |
|---|---|---|
| CFTR-Related Disorders | CFTR | 3667del4, 3667del4 |
| CFTR-Related Disorders | CFTR | 3667del4, 3667del4 |
| CFTR-Related Disorders | CFTR | 3791delC, 3791delC |
| CFTR-Related Disorders | CFTR | 3791delC, 3791delC |
| CFTR-Related Disorders | CFTR | 3821delT, 3821delT |
| CFTR-Related Disorders | CFTR | 3821delT, 3821delT |
| CFTR-Related Disorders | CFTR | 3905insT, 3905insT |
| CFTR-Related Disorders | CFTR | 3905insT, 3905insT |
| CFTR-Related Disorders | CFTR | 4016insT, 4016insT |
| CFTR-Related Disorders | CFTR | 4016insT, 4016insT |
| CFTR-Related Disorders | CFTR | 394delTT, 394delTT |
| CFTR-Related Disorders | CFTR | 394delTT, 394delTT |
| CFTR-Related Disorders | CFTR | 405+1G->A |
| CFTR-Related Disorders | CFTR | 405+3A->C |
| CFTR-Related Disorders | CFTR | 444delA |
| CFTR-Related Disorders | CFTR | 444delA |
| CFTR-Related Disorders | CFTR | 3876delA, 3876delA |
| CFTR-Related Disorders | CFTR | 3876delA, 3876delA |
| CFTR-Related Disorders | CFTR | 457TAT->G |
| CFTR-Related Disorders | CFTR | 457TAT->G |
| CFTR-Related Disorders | CFTR | 3199del6, 3199del6 |
| CFTR-Related Disorders | CFTR | 3199del6, 3199del6 |
| CFTR-Related Disorders | CFTR | 406-1G->A |
| CFTR-Related Disorders | CFTR | 663delT, 663delT |
| CFTR-Related Disorders | CFTR | 663delT, 663delT |
| CFTR-Related Disorders | CFTR | 935delA, 935delA |
| CFTR-Related Disorders | CFTR | 935delA, 935delA |
| CFTR-Related Disorders | CFTR | CFTR dele2,3 (21kb) |
| CFTR-Related Disorders | CFTR | CFTR dele2,3 (21kb) |
| CFTR-Related Disorders | CFTR | D1152H |
| CFTR-Related Disorders | CFTR | E60X |
| CFTR-Related Disorders | CFTR | E92X |
| CFTR-Related Disorders | CFTR | F508C, rs1800093 |
| CFTR-Related Disorders | CFTR | G178R |
| CFTR-Related Disorders | CFTR | G330X |
| CFTR-Related Disorders | CFTR | G480C |
| CFTR-Related Disorders | CFTR | G542X |
| CFTR-Related Disorders | CFTR | G551D |
| CFTR-Related Disorders | CFTR | G622D |
| CFTR-Related Disorders | CFTR | G85E |
| CFTR-Related Disorders | CFTR | G91R |
| CFTR-Related Disorders | CFTR | I148T, rs35516286 |
| CFTR-Related Disorders | CFTR | I506V |
| CFTR-Related Disorders | CFTR | IVS8-5T |
| CFTR-Related Disorders | CFTR | IVS8-7T |
| CFTR-Related Disorders | CFTR | IVS8-9T |
| CFTR-Related Disorders | CFTR | K710X |
| CFTR-Related Disorders | CFTR | L206W |

## FIGURE 1 – Table of Exemplary Causal Genetic Variants

| Disease | Gene | Variant Name |
|---|---|---|
| CFTR-Related Disorders | CFTR | M1101K, rs36210737 |
| CFTR-Related Disorders | CFTR | N1303K |
| CFTR-Related Disorders | CFTR | P574H |
| CFTR-Related Disorders | CFTR | Q1238X |
| CFTR-Related Disorders | CFTR | Q359K/T360K_wt |
| CFTR-Related Disorders | CFTR | Q493X |
| CFTR-Related Disorders | CFTR | Q552X |
| CFTR-Related Disorders | CFTR | Q890X |
| CFTR-Related Disorders | CFTR | R1066C |
| CFTR-Related Disorders | CFTR | R1070Q |
| CFTR-Related Disorders | CFTR | R1158X |
| CFTR-Related Disorders | CFTR | R1162X |
| CFTR-Related Disorders | CFTR | R117C |
| CFTR-Related Disorders | CFTR | R117H |
| CFTR-Related Disorders | CFTR | R1283M |
| CFTR-Related Disorders | CFTR | R334W |
| CFTR-Related Disorders | CFTR | R347H |
| CFTR-Related Disorders | CFTR | R347P |
| CFTR-Related Disorders | CFTR | R352Q |
| CFTR-Related Disorders | CFTR | R553X |
| CFTR-Related Disorders | CFTR | R560T |
| CFTR-Related Disorders | CFTR | R709X |
| CFTR-Related Disorders | CFTR | R75X |
| CFTR-Related Disorders | CFTR | R764X |
| CFTR-Related Disorders | CFTR | S1196X |
| CFTR-Related Disorders | CFTR | S1235R, rs34911792 |
| CFTR-Related Disorders | CFTR | S1251N |
| CFTR-Related Disorders | CFTR | S1255X |
| CFTR-Related Disorders | CFTR | S364P |
| CFTR-Related Disorders | CFTR | S549I |
| CFTR-Related Disorders | CFTR | S549N |
| CFTR-Related Disorders | CFTR | S549R |
| CFTR-Related Disorders | CFTR | S549R |
| CFTR-Related Disorders | CFTR | T338I |
| CFTR-Related Disorders | CFTR | V520F |
| CFTR-Related Disorders | CFTR | W1089X |
| CFTR-Related Disorders | CFTR | W1204X |
| CFTR-Related Disorders | CFTR | W1204X |
| CFTR-Related Disorders | CFTR | W1282X |
| CFTR-Related Disorders | CFTR | Y1092X |
| CFTR-Related Disorders | CFTR | Y122X |
| Choroideremia | CHM | c.1609+2dupT |
| Choroideremia | CHM | c.1609+2dupT |
| CLN3-Related Neuronal Ceroid-Lipofuscinosis | CLN3 | c.461_677del |
| CLN3-Related Neuronal Ceroid-Lipofuscinosis | CLN3 | c.461_677del |
| CLN3-Related Neuronal Ceroid-Lipofuscinosis | CLN3 | c.791_1056del |

## FIGURE 1 – Table of Exemplary Causal Genetic Variants

| Disease | Gene | Variant Name |
|---|---|---|
| CLN3-Related Neuronal Ceroid-Lipofuscinosis | CLN3 | c.791_1056del |
| CLN5-Related Neuronal Ceroid-Lipofuscinosis | CLN5 | c.1175_1176delAT |
| CLN5-Related Neuronal Ceroid-Lipofuscinosis | CLN5 | c.1175_1176delAT |
| CLN5-Related Neuronal Ceroid-Lipofuscinosis | CLN5 | c.225G>A |
| CLN8-Related Neuronal Ceroid-Lipofuscinosis | CLN8 | c.70C>G |
| Cohen Syndrome | VPS13B | c.3348_3349delCT |
| Cohen Syndrome | VPS13B | c.3348_3349delCT |
| Congenital Cataracts, Facial Dysmorphism, and Neuropathy | CTDP1 | IVS6+389C>T |
| Congenital Disorder of Glycosylation Ia | PMM2 | p.F119L |
| Congenital Disorder of Glycosylation Ia | PMM2 | p.R141H |
| Congenital Disorder of Glycosylation Ib | MPI | R295H, rs28928906 |
| Congenital Finnish Nephrosis | NPHS1 | c.121_122del |
| Congenital Finnish Nephrosis | NPHS1 | c.121_122del |
| Congenital Finnish Nephrosis | NPHS1 | c.3325C>T |
| Crohn Disease | NOD2 | 3020 ins C |
| Crohn Disease | NOD2 | 3020 ins C |
| Crohn Disease | NOD2 | G908R, rs2066845 |
| Crohn Disease | NOD2 | R702W, rs2066844 |
| Cystinosis | CTNS | 1035insC |
| Cystinosis | CTNS | 1035insC |
| Cystinosis | CTNS | 537del21 |
| Cystinosis | CTNS | 537del21 |
| Cystinosis | CTNS | 57kb deletion |
| Cystinosis | CTNS | 57kb deletion |
| Cystinosis | CTNS | D205N |
| Cystinosis | CTNS | L158P |
| Cystinosis | CTNS | W138X |
| DFNA 9 (COCH) | COCH | P51S |
| Diabetes and Hearing Loss | mtDNA | 3234A>G |
| Diabetes and Hearing Loss | mtDNA | 3271T>C |
| Diabetes and Hearing Loss | mtDNA | G8363A |
| Diabetes and Hearing Loss | mtDNA | T14709C |
| Early-Onset Primary Dystonia (DYT1) | TOR1A | 904_906delGAG |
| Early-Onset Primary Dystonia (DYT1) | TOR1A | 904_906delGAG |
| Epidermolysis Bullosa Junctional, Herlitz-Pearson Type | LAMB3 | 3024delT |

## FIGURE 1 – Table of Exemplary Causal Genetic Variants

| Disease | Gene | Variant Name |
|---|---|---|
| Epidermolysis Bullosa Junctional, Herlitz-Pearson Type | LAMB3 | 3024delT |
| Epidermolysis Bullosa Junctional, Herlitz-Pearson Type | LAMB3 | p.Q243X |
| Epidermolysis Bullosa Junctional, Herlitz-Pearson Type | LAMB3 | R144X |
| Epidermolysis Bullosa Junctional, Herlitz-Pearson Type | LAMB3 | R42X |
| Epidermolysis Bullosa Junctional, Herlitz-Pearson Type | LAMB3 | R635X |
| Epidermolysis Bullosa Junctional, Herlitz-Pearson Type | LAMA3 | R650X |
| Epidermolysis Bullosa Junctional, Herlitz-Pearson Type | LAMC2 | R95X |
| Factor V Leiden Thrombophilia | F5 | H1299R |
| Factor V Leiden Thrombophilia | F5 | R506Q, rs6025 |
| Factor V R2 Mutation Thrombophilia | F5 | rs6027 |
| Factor XI Deficiency | F11 | E117X (576G>T) |
| Factor XI Deficiency | F11 | F283L (1074T>C) |
| Factor XI Deficiency | F11 | IVS14 +1G>A |
| Factor XI Deficiency | F11 | IVS14del14 |
| Factor XI Deficiency | F11 | IVS14del14 |
| Factor XIII Deficiency | F13A1 | V34L, rs5985 |
| Familial Adenomatous Polyposis | APC | I1307K, rs1801155 |
| Familial Dysautonomia | IKBKAP | 2507+6T>C |
| Familial Dysautonomia | IKBKAP | P914L |
| Familial Dysautonomia | IKBKAP | R696P |
| Familial Hypercholesterolemia Type B | APOB | R3500Q, rs5742904 |
| Familial Hypercholesterolemia Type B | APOB | R3500W |
| Familial Hypercholesterolemia Type B | APOB | R3531C, rs12713559 |
| Familial Mediterranean Fever | MEFV | A744S (2230G>T) |
| Familial Mediterranean Fever | MEFV | delI692 (del2076_2078) |
| Familial Mediterranean Fever | MEFV | delI692 (del2076_2078) |

## FIGURE 1 – Table of Exemplary Causal Genetic Variants

| Disease | Gene | Variant Name |
|---|---|---|
| Familial Mediterranean Fever | MEFV | E148Q (442 G>C), rs3743930 |
| Familial Mediterranean Fever | MEFV | E167D (501 G>C) |
| Familial Mediterranean Fever | MEFV | F479L (1437 C>G) |
| Familial Mediterranean Fever | MEFV | K695R (2084A>G) |
| Familial Mediterranean Fever | MEFV | M680I (2040G>C) |
| Familial Mediterranean Fever | MEFV | M694I (2082G>A), rs28940578 |
| Familial Mediterranean Fever | MEFV | M694V (2080A>G) |
| Familial Mediterranean Fever | MEFV | P369S (1105 C>T), rs11466023 |
| Familial Mediterranean Fever | MEFV | R408Q (1223G>A), rs11466024 |
| Familial Mediterranean Fever | MEFV | R653H (1958G>A) |
| Familial Mediterranean Fever | MEFV | R761H (2282G>A) |
| Familial Mediterranean Fever | MEFV | T267I (800 C>T) |
| Familial Mediterranean Fever | MEFV | V726A (2177T>C), rs28940579 |
| FANCC-Related Fanconi Anemia | FANCC | 322delG |
| FANCC-Related Fanconi Anemia | FANCC | 322delG |
| FANCC-Related Fanconi Anemia | FANCC | IVS4+4A>T (711 +4A>T) |
| FANCC-Related Fanconi Anemia | FANCC | Q13X (37C>T) |
| FANCC-Related Fanconi Anemia | FANCC | R547X |
| FGFR1-Related Craniosynostosis | FGFR1 | P252R |
| FGFR2-Related Craniosynostosis | FGFR2 | P253R |
| FGFR2-Related Craniosynostosis | FGFR2 | S252W |
| FGFR3-Related Craniosynostosis | FGFR3 | A391E, rs28931615 |
| FGFR3-Related Craniosynostosis | FGFR3 | P250R, rs4647924 |
| Free Sialic Acid Storage Disorders | SLC17A5 | c.1007_1008delTA |
| Free Sialic Acid Storage Disorders | SLC17A5 | c.1007_1008delTA |
| Free Sialic Acid Storage Disorders | SLC17A5 | c.115C>T |
| Frontotemporal Dementia with Parkinsonism-17 | MAPT | IVS10+16 |

## FIGURE 1 – Table of Exemplary Causal Genetic Variants

| Disease | Gene | Variant Name |
|---|---|---|
| Frontotemporal Dementia with Parkinsonism-17 | MAPT | P301L |
| Frontotemporal Dementia with Parkinsonism-17 | MAPT | P301S |
| Frontotemporal Dementia with Parkinsonism-17 | MAPT | R406W |
| Fumarase deficiency | FH | c.1431_1433dupAAA |
| Fumarase deficiency | FH | c.1431_1433dupAAA |
| Galactosemia | GALT | 5.0Kb gene deletion |
| Galactosemia | GALT | 5.0Kb gene deletion |
| Galactosemia | GALT | 5'UTR-119del |
| Galactosemia | GALT | 5'UTR-119del |
| Galactosemia | GALT | IVS2-2 A>G |
| Galactosemia | GALT | K285N |
| Galactosemia | GALT | L195P T>C |
| Galactosemia | GALT | L218L |
| Galactosemia | GALT | N314D, rs2070074 |
| Galactosemia | GALT | Phe171Ser |
| Galactosemia | GALT | Q169K |
| Galactosemia | GALT | Q188R |
| Galactosemia | GALT | S135L |
| Galactosemia | GALT | T138M C>T |
| Galactosemia | GALT | X380R |
| Galactosemia | GALT | Y209C A>G |
| Gaucher Disease | GBA | 1035insG |
| Gaucher Disease | GBA | 1035insG |
| Gaucher Disease | GBA | 84insG |
| Gaucher Disease | GBA | 84insG |
| Gaucher Disease | GBA | D409H, rs1064651 |
| Gaucher Disease | GBA | D409V |
| Gaucher Disease | GBA | IVS2(+1)G>A |
| Gaucher Disease | GBA | L444P (1448T>C), rs35095275 |
| Gaucher Disease | GBA | N370S |
| Gaucher Disease | GBA | R463C |
| Gaucher Disease | GBA | R463H |
| Gaucher Disease | GBA | R496H (1604G>A) |
| Gaucher Disease | GBA | V394L |
| GJB2-Related DFNA 3 Nonsyndromic Hearing Loss and Deafness | GJB2 | 167delT |
| GJB2-Related DFNA 3 Nonsyndromic Hearing Loss and Deafness | GJB2 | 167delT |
| GJB2-Related DFNA 3 Nonsyndromic Hearing Loss and Deafness | GJB2 | 235delC |
| GJB2-Related DFNA 3 Nonsyndromic Hearing Loss and Deafness | GJB2 | 235delC |

## FIGURE 1 – Table of Exemplary Causal Genetic Variants

| Disease | Gene | Variant Name |
|---|---|---|
| GJB2-Related DFNA 3 Nonsyndromic Hearing Loss and Deafness | GJB2 | 35delG |
| GJB2-Related DFNA 3 Nonsyndromic Hearing Loss and Deafness | GJB2 | 35delG |
| GJB2-Related DFNA 3 Nonsyndromic Hearing Loss and Deafness | GJB2 | IVS1+1G>A |
| GJB2-Related DFNB 1 Nonsyndromic Hearing Loss and Deafness | GJB2 | 101delAG |
| GJB2-Related DFNB 1 Nonsyndromic Hearing Loss and Deafness | GJB2 | 313del14 |
| GJB2-Related DFNB 1 Nonsyndromic Hearing Loss and Deafness | GJB2 | 313del14 |
| GJB2-Related DFNB 1 Nonsyndromic Hearing Loss and Deafness | GJB2 | delE120 |
| GJB2-Related DFNB 1 Nonsyndromic Hearing Loss and Deafness | GJB2 | delE120 |
| GJB2-Related DFNB 1 Nonsyndromic Hearing Loss and Deafness | GJB2 | M34T, rs35887622 |
| GJB2-Related DFNB 1 Nonsyndromic Hearing Loss and Deafness | GJB2 | Q124X |
| GJB2-Related DFNB 1 Nonsyndromic Hearing Loss and Deafness | GJB2 | R184P |
| GJB2-Related DFNB 1 Nonsyndromic Hearing Loss and Deafness | GJB2 | V37I |
| GJB2-Related DFNB 1 Nonsyndromic Hearing Loss and Deafness | GJB2 | W24X |
| GJB2-Related DFNB 1 Nonsyndromic Hearing Loss and Deafness | GJB2 | W77R |
| GJB2-Related DFNB 1 Nonsyndromic Hearing Loss and Deafness | GJB2 | W77X |
| Glucose-6-Phosphate Dehydrogenase Deficiency | G6PD | A335V |
| Glucose-6-Phosphate Dehydrogenase Deficiency | G6PD | R459L |
| Glucose-6-Phosphate Dehydrogenase Deficiency | G6PD | R459P |
| Glucose-6-Phosphate Dehydrogenase Deficiency | G6PD | rs1050828, rs1050828 |

## FIGURE 1 – Table of Exemplary Causal Genetic Variants

| Disease | Gene | Variant Name |
|---|---|---|
| Glucose-6-Phosphate Dehydrogenase Deficiency | G6PD | rs1050829, rs1050829 |
| Glucose-6-Phosphate Dehydrogenase Deficiency | G6PD | rs5030868, rs5030868 |
| Glutaricacidemia Type 1 | GCDH | A421V |
| Glutaricacidemia Type 1 | GCDH | R402W |
| Glycogen Storage Disease Type 1a | G6PC | 459insTA |
| Glycogen Storage Disease Type 1a | G6PC | 459insTA |
| Glycogen Storage Disease Type 1a | G6PC | 727G/T |
| Glycogen Storage Disease Type 1a | G6PC | del F327 |
| Glycogen Storage Disease Type 1a | G6PC | del F327 |
| Glycogen Storage Disease Type 1a | G6PC | G188R |
| Glycogen Storage Disease Type 1a | G6PC | G270V |
| Glycogen Storage Disease Type 1a | G6PC | Q242X |
| Glycogen Storage Disease Type 1a | G6PC | Q27fsdelC |
| Glycogen Storage Disease Type 1a | G6PC | Q27fsdelC |
| Glycogen Storage Disease Type 1a | G6PC | Q347X |
| Glycogen Storage Disease Type 1a | G6PC | R83C |
| Glycogen Storage Disease Type 1a | G6PC | R83H |
| Glycogen Storage Disease Type 1b | G6PT1 | 1211delCT |
| Glycogen Storage Disease Type 1b | G6PT1 | A367T |
| Glycogen Storage Disease Type 1b | G6PT1 | G339C |
| Glycogen Storage Disease Type 1b | G6PT1 | G339D |
| Glycogen Storage Disease Type 1b | G6PT1 | W118R |
| Glycogen Storage Disease Type II | GAA | Arg854X |
| Glycogen Storage Disease Type II | GAA | Asp645Glu, rs28940868 |
| Glycogen Storage Disease Type II | GAA | IVS1(-13t>g) |
| Glycogen Storage Disease Type III | AGL | 1484delT |
| Glycogen Storage Disease Type III | AGL | 1484delT |
| Glycogen Storage Disease Type III | AGL | 17delAG |

## FIGURE 1 – Table of Exemplary Causal Genetic Variants

| Disease | Gene | Variant Name |
|---|---|---|
| Glycogen Storage Disease Type III | AGL | 17delAG |
| Glycogen Storage Disease Type III | AGL | Q6X |
| Glycogen Storage Disease Type V | PYGM | G204S |
| Glycogen Storage Disease Type V | PYGM | K542T |
| Glycogen Storage Disease Type V | PYGM | K542X |
| Glycogen Storage Disease Type V | PYGM | R49X |
| GNE-Related Myopathies | GNE | M712T, rs28937594 |
| Gracile Syndrome | BCS1L | c.232A>G, rs28937590 |
| Hemoglobin S Beta-Thalassemia | HBB | c.19G>A |
| Hemoglobin S Beta-Thalassemia | HBB | c.20A>T |
| Hemoglobin S Beta-Thalassemia | HBB | c.79G>A |
| Hemoglobin S Beta-Thalassemia | HBB | Hb CS |
| Hemoglobin S Beta-Thalassemia | HBB | Hb D |
| Hemoglobin S Beta-Thalassemia | HBB | Hb O |
| Hereditary Fructose Intolerance | ALDOB | A149P, rs1800546 |
| Hereditary Fructose Intolerance | ALDOB | A174D |
| Hereditary Fructose Intolerance | ALDOB | Delta4E4 |
| Hereditary Fructose Intolerance | ALDOB | Delta4E4 |
| Hereditary Fructose Intolerance | ALDOB | N334K |
| Hereditary Fructose Intolerance | ALDOB | Y203X |
| Hereditary Pancreatitis | PRSS1 | A16V |
| Hereditary Pancreatitis | SPINK1 | M1T |
| Hereditary Pancreatitis | PRSS1 | N29I |
| Hereditary Pancreatitis | SPINK1 | N34S, rs17107315 |
| Hereditary Pancreatitis | PRSS1 | R122C |
| Hereditary Pancreatitis | PRSS1 | R122H |
| Hereditary Thymine-Uraciluria | DPYD | rs3918290 |
| Hexosaminidase A Deficiency | HEXA | 1278insTATC |
| Hexosaminidase A Deficiency | HEXA | 1278insTATC |
| Hexosaminidase A Deficiency | HEXA | G269S (805G>A) |

## FIGURE 1 – Table of Exemplary Causal Genetic Variants

| Disease | Gene | Variant Name |
|---|---|---|
| Hexosaminidase A Deficiency | HEXA | IVS12 +1G>C |
| Hexosaminidase A Deficiency | HEXA | IVS7 +1G>A |
| Hexosaminidase A Deficiency | HEXA | IVS9 +1G>A |
| Hexosaminidase A Deficiency | HEXA | R178C |
| Hexosaminidase A Deficiency | HEXA | R178H |
| Hexosaminidase A Deficiency | HEXA | R247W (739C>T) |
| Hexosaminidase A Deficiency | HEXA | R249W (745C>T) |
| HFE-Associated Hereditary Hemochromatosis | HFE | E168Q |
| HFE-Associated Hereditary Hemochromatosis | HFE | E168X |
| HFE-Associated Hereditary Hemochromatosis | HFE | HM971246, H63H |
| HFE-Associated Hereditary Hemochromatosis | HFE | P160delC |
| HFE-Associated Hereditary Hemochromatosis | HFE | P160delC |
| HFE-Associated Hereditary Hemochromatosis | HFE | Q127H, rs28934595 |
| HFE-Associated Hereditary Hemochromatosis | HFE | Q283P |
| HFE-Associated Hereditary Hemochromatosis | HFE | rs1799945, rs1799945 |
| HFE-Associated Hereditary Hemochromatosis | HFE | rs1800562, rs1800562 |
| HFE-Associated Hereditary Hemochromatosis | HFE | rs1800730, rs1800730 |
| HFE-Associated Hereditary Hemochromatosis | HFE | V53M |
| HFE-Associated Hereditary Hemochromatosis | HFE | V59M |
| HFE-Associated Hereditary Hemochromatosis | HFE | W169X |
| Hidrotic Ectodermal Dysplasia 2 | GJB6 | A88V, rs28937872 |
| Hidrotic Ectodermal Dysplasia 2 | GJB6 | G11R |
| Hidrotic Ectodermal Dysplasia 2 | GJB6 | V37E |
| Homocystinuria Caused by Cystathionine Beta-Synthase Deficiency | CBS | G307S 919G->A |
| Homocystinuria Caused by Cystathionine Beta-Synthase Deficiency | CBS | I278T 833T->C, rs5742905 |
| Hyperkalemic Periodic Paralysis Type 1 | SCN4A | I693T |

## FIGURE 1 – Table of Exemplary Causal Genetic Variants

| Disease | Gene | Variant Name |
|---|---|---|
| Hyperkalemic Periodic Paralysis Type 1 | SCN4A | L689I |
| Hyperkalemic Periodic Paralysis Type 1 | SCN4A | L689V |
| Hyperkalemic Periodic Paralysis Type 1 | SCN4A | M1360V |
| Hyperkalemic Periodic Paralysis Type 1 | SCN4A | M1592V |
| Hyperkalemic Periodic Paralysis Type 1 | SCN4A | p.A1156T |
| Hyperkalemic Periodic Paralysis Type 1 | SCN4A | p.M1370V |
| Hyperkalemic Periodic Paralysis Type 1 | SCN4A | p.R1448C |
| Hyperkalemic Periodic Paralysis Type 1 | SCN4A | p.T1313M |
| Hyperkalemic Periodic Paralysis Type 1 | SCN4A | R675G |
| Hyperkalemic Periodic Paralysis Type 1 | SCN4A | R675Q |
| Hyperkalemic Periodic Paralysis Type 1 | SCN4A | R675W |
| Hyperkalemic Periodic Paralysis Type 1 | SCN4A | T704M |
| Hyperkalemic Periodic Paralysis Type 1 | SCN4A | V781I |
| Hyperornithinemia-Hyperammonemia-Homocitrullinuria Syndrome | SLC25A15 | F188del |
| Hyperornithinemia-Hyperammonemia-Homocitrullinuria Syndrome | SLC25A15 | F188del |
| Hyperoxaluria, Primary, Type 1 | AGXT | 33insC |
| Hyperoxaluria, Primary, Type 1 | AGXT | 33insC |
| Hyperoxaluria, Primary, Type 1 | AGXT | F152I |
| Hyperoxaluria, Primary, Type 1 | AGXT | G170R |
| Hyperoxaluria, Primary, Type 1 | AGXT | I244T |
| Hyperoxaluria, Primary, Type 2 | GRHPR | 103delG |
| Hyperoxaluria, Primary, Type 2 | GRHPR | 103delG |
| Hypochondroplasia | FGFR3 | Asn328Ile |
| Hypochondroplasia | FGFR3 | I538V |
| Hypochondroplasia | FGFR3 | K650M |
| Hypochondroplasia | FGFR3 | K650N 1950G>T |
| Hypochondroplasia | FGFR3 | K650Q |
| Hypochondroplasia | FGFR3 | N540K 1620C>A |

## FIGURE 1 – Table of Exemplary Causal Genetic Variants

| Disease | Gene | Variant Name |
|---|---|---|
| Hypochondroplasia | FGFR3 | N540S |
| Hypochondroplasia | FGFR3 | N540T |
| Hypokalemic Periodic Paralysis Type 1 | CACNA1S | R528G |
| Hypokalemic Periodic Paralysis Type 1 | CACNA1S | R528H |
| Hypokalemic Periodic Paralysis Type 1 | CACNA1S | rs28930068, rs28930068 |
| Hypokalemic Periodic Paralysis Type 1 | CACNA1S | rs28930069, rs28930069 |
| Hypokalemic Periodic Paralysis Type 2 | SCN4A | R669H |
| Hypokalemic Periodic Paralysis Type 2 | SCN4A | R672C |
| Hypokalemic Periodic Paralysis Type 2 | SCN4A | R672G |
| Hypokalemic Periodic Paralysis Type 2 | SCN4A | R672H |
| Hypokalemic Periodic Paralysis Type 2 | SCN4A | R672S |
| Hypophosphatasia | ALPL | Asp361Val |
| Hypophosphatasia | ALPL | c.1559delT |
| Hypophosphatasia | ALPL | c.1559delT |
| Hypophosphatasia | ALPL | E174K |
| Hypophosphatasia | ALPL | G317D |
| Hypophosphatasia | ALPL | Phe310Leu |
| Isovaleric Acidemia | IVD | A282V |
| Isovaleric Acidemia | IVD | rs28940889 |
| Krabbe Disease | GALC | EX11-17DEL |
| Krabbe Disease | GALC | EX11-17DEL |
| Krabbe Disease | GALC | G270D |
| Krabbe Disease | GALC | rs1805078, rs1805078 |
| Krabbe Disease | GALC | rs398607 |
| Leber Hereditary Optic Neuropathy | mtDNA | 14484T>C |
| Leber Hereditary Optic Neuropathy | mtDNA | 15257G>A |
| Leber Hereditary Optic Neuropathy | mtDNA | G14459A |
| Leber Hereditary Optic Neuropathy | mtDNA | G3460A |
| Leber Hereditary Optic Neuropathy | mtDNA | m.11778G>A |
| Leber Hereditary Optic Neuropathy | mtDNA | m.13708G>A |
| Leber Hereditary Optic Neuropathy | mtDNA | m.15812G>A |
| Leber Hereditary Optic Neuropathy | mtDNA | m.3394T>C |
| Leber Hereditary Optic Neuropathy | mtDNA | m.4216T>C |

## FIGURE 1 – Table of Exemplary Causal Genetic Variants

| Disease | Gene | Variant Name |
|---|---|---|
| Leber Hereditary Optic Neuropathy | mtDNA | m.4917A>G |
| Leigh Syndrome, French-Canadian Type | LRPPRC | A354V |
| LGMD2I | FKRP | L276I, rs28937900 |
| Long Chain 3-Hydroxyacyl-CoA Dehydrogenase Deficiency | HADHA | E474Q c.1528G>C |
| Long Chain 3-Hydroxyacyl-CoA Dehydrogenase Deficiency | HADHA | Q342X 1132C>T |
| Maple Syrup Urine Disease Type 1A | BCKDHA | Y438N |
| Maple Syrup Urine Disease Type 1B | BCKDHB | E372X |
| Maple Syrup Urine Disease Type 1B | BCKDHB | G278S |
| Maple Syrup Urine Disease Type 1B | BCKDHB | R183P |
| McCune-Albright Syndrome | GNAS | R201C |
| McCune-Albright Syndrome | GNAS | R201G |
| McCune-Albright Syndrome | GNAS | R201H |
| Medium Chain Acyl-Coenzyme A Dehydrogenase Deficiency | ACADM | 244insT |
| Medium Chain Acyl-Coenzyme A Dehydrogenase Deficiency | ACADM | 244insT |
| Medium Chain Acyl-Coenzyme A Dehydrogenase Deficiency | ACADM | 250C>T |
| Medium Chain Acyl-Coenzyme A Dehydrogenase Deficiency | ACADM | 583G>A |
| Medium Chain Acyl-Coenzyme A Dehydrogenase Deficiency | ACADM | 616C>T |
| Medium Chain Acyl-Coenzyme A Dehydrogenase Deficiency | ACADM | 617G>A |
| Medium Chain Acyl-Coenzyme A Dehydrogenase Deficiency | ACADM | 799G>A |
| Medium Chain Acyl-Coenzyme A Dehydrogenase Deficiency | ACADM | K304E |
| Medium Chain Acyl-Coenzyme A Dehydrogenase Deficiency | ACADM | Y42H |

## FIGURE 1 – Table of Exemplary Causal Genetic Variants

| Disease | Gene | Variant Name |
|---|---|---|
| Megalencephalic Leukoencephalopathy with Subcortical Cysts | MLC1 | 135insC |
| Megalencephalic Leukoencephalopathy with Subcortical Cysts | MLC1 | 135insC |
| MELAS | mtDNA | 3243A>G |
| MELAS | mtDNA | 3250T>C |
| MELAS | mtDNA | 3252A>G |
| MELAS | mtDNA | A12770G |
| MELAS | mtDNA | C3256T |
| MELAS | mtDNA | G13513A |
| MELAS | mtDNA | T3291C |
| MELAS | mtDNA | T8356C |
| MELAS | mtDNA | T9957C |
| MERRF | mtDNA | 8361G>A |
| MERRF | mtDNA | A8296G |
| MERRF | mtDNA | m.8344A>G |
| Metachromatic Leukodystrophy | ARSA | c.459+1G>A |
| Metachromatic Leukodystrophy | ARSA | p.P426L, rs28940893 |
| Metachromatic Leukodystrophy | ARSA | p.T274M |
| Metachromatic Leukodystrophy | ARSA | P377L |
| Mitochondrial Cardiomyopathy | mtDNA | A3260T |
| Mitochondrial Cardiomyopathy | mtDNA | A4300G |
| Mitochondrial Cardiomyopathy | mtDNA | C3303T |
| Mitochondrial Cardiomyopathy | mtDNA | T9997C |
| Mitochondrial DNA-Associated Leigh Syndrome and NARP | mtDNA | 5537insT |
| Mitochondrial DNA-Associated Leigh Syndrome and NARP | mtDNA | 5537insT |
| Mitochondrial DNA-Associated Leigh Syndrome and NARP | mtDNA | 8993T>C |
| Mitochondrial DNA-Associated Leigh Syndrome and NARP | mtDNA | 8993T>G |
| Mitochondrial DNA-Associated Leigh Syndrome and NARP | mtDNA | C11777A |
| Mitochondrial DNA-Associated Leigh Syndrome and NARP | mtDNA | T10158C |

## FIGURE 1 – Table of Exemplary Causal Genetic Variants

| Disease | Gene | Variant Name |
| --- | --- | --- |
| Mitochondrial DNA-Associated Leigh Syndrome and NARP | mtDNA | T10191C |
| Mitochondrial DNA-Associated Leigh Syndrome and NARP | mtDNA | T8851C |
| Mitochondrial DNA-Associated Leigh Syndrome and NARP | mtDNA | T9176C |
| Mitochondrial DNA-Associated Leigh Syndrome and NARP | mtDNA | T9176G |
| MTHFR Deficiency | MTHFR | 1298A>C |
| MTHFR Deficiency | MTHFR | rs1801133, rs1801133 |
| MTRNR1-Related Hearing Loss and Deafness | mtDNA | 1095T>C |
| MTRNR1-Related Hearing Loss and Deafness | mtDNA | 1494C>T |
| MTRNR1-Related Hearing Loss and Deafness | mtDNA | 1555A>G |
| MTRNR1-Related Hearing Loss and Deafness | mtDNA | 961T>G |
| MTRNR1-Related Hearing Loss and Deafness | mtDNA | A7445G |
| MTTS1-Related Hearing Loss and Deafness | mtDNA | 7443A>G |
| MTTS1-Related Hearing Loss and Deafness | mtDNA | 7444G>A |
| MTTS1-Related Hearing Loss and Deafness | mtDNA | 7472insC |
| MTTS1-Related Hearing Loss and Deafness | mtDNA | 7472insC |
| MTTS1-Related Hearing Loss and Deafness | mtDNA | 7510T>C |
| MTTS1-Related Hearing Loss and Deafness | mtDNA | 7511T>C |
| MTTS1-Related Hearing Loss and Deafness | mtDNA | 7512T>C |
| Mucolipidosis IV | MCOLN1 | delEx1 3 Ex7 (511>6944del) |
| Mucolipidosis IV | MCOLN1 | delEx1 3 Ex7 (511>6944del) |
| Mucolipidosis IV | MCOLN1 | IVS-2A>G |
| Mucopolysaccharidosis Type I | IDUA | c.46_57del |
| Mucopolysaccharidosis Type I | IDUA | c.46_57del |
| Mucopolysaccharidosis Type I | IDUA | p.A327P |
| Mucopolysaccharidosis Type I | IDUA | p.P533R |
| Mucopolysaccharidosis Type I | IDUA | Q70X |
| Mucopolysaccharidosis Type I | IDUA | W402X |

## FIGURE 1 – Table of Exemplary Causal Genetic Variants

| Disease | Gene | Variant Name |
| --- | --- | --- |
| Mucopolysaccharidosis Type IIIA | SGSH | p.R245H |
| Mucopolysaccharidosis Type IIIA | SGSH | p.R74C |
| Mucopolysaccharidosis Type IIIA | SGSH | p.S66W |
| Mucopolysaccharidosis Type VII | GUSB | p.D152N |
| Multiple Endocrine Neoplasia Type 2 | RET | 2047T>A |
| Multiple Endocrine Neoplasia Type 2 | RET | 2047T>A |
| Multiple Endocrine Neoplasia Type 2 | RET | 2047T>C |
| Multiple Endocrine Neoplasia Type 2 | RET | 2047T>G |
| Multiple Endocrine Neoplasia Type 2 | RET | 2048G>A |
| Multiple Endocrine Neoplasia Type 2 | RET | A883F 2647 G>T |
| Multiple Endocrine Neoplasia Type 2 | RET | Glu768Asp G>C |
| Multiple Endocrine Neoplasia Type 2 | RET | M918T |
| Muscle-Eye-Brain Disease | POMGNT1 | c.1539+1G>A |
| MYH-Associated Polyposis | MUTYH | c.1376C>A |
| MYH-Associated Polyposis | MUTYH | c.494A>G, rs34612342 |
| MYH-Associated Polyposis | GENE_SYMBOL_TBD | rs36053993, rs36053993 |
| Niemann-Pick Disease Due to Sphingomyelinase Deficiency | SMPD1 | c.990delC |
| Niemann-Pick Disease Due to Sphingomyelinase Deficiency | SMPD1 | c.990delC |
| Niemann-Pick Disease Due to Sphingomyelinase Deficiency | SMPD1 | fsP330 |
| Niemann-Pick Disease Due to Sphingomyelinase Deficiency | SMPD1 | fsP330 |
| Niemann-Pick Disease Due to Sphingomyelinase Deficiency | SMPD1 | L302P |
| Niemann-Pick Disease Due to Sphingomyelinase Deficiency | SMPD1 | R496L |
| Niemann-Pick Disease Due to Sphingomyelinase Deficiency | SMPD1 | R608del |
| Niemann-Pick Disease Type C1 | NPC1 | I1061T |

## FIGURE 1 – Table of Exemplary Causal Genetic Variants

| Disease | Gene | Variant Name |
|---|---|---|
| Nijmegen Breakage Syndrome | NBN | 657del5 |
| Nijmegen Breakage Syndrome | NBN | 657del5 |
| Pallister-Hall Syndrome | GLI3 | 2012delG |
| Pallister-Hall Syndrome | GLI3 | 2012delG |
| Pallister-Hall Syndrome | GLI3 | 2023delG |
| Pallister-Hall Syndrome | GLI3 | 2023delG |
| Pendred Syndrome | SLC26A4 | 1197delT |
| Pendred Syndrome | SLC26A4 | 1197delT |
| Pendred Syndrome | SLC26A4 | E384G |
| Pendred Syndrome | SLC26A4 | IVS8+1(G->A) |
| Pendred Syndrome | SLC26A4 | L236P |
| Pendred Syndrome | SLC26A4 | T416P |
| Peroxisomal Bifunctional Enzyme Deficiency | HSD17B4 | c.302+1G>C |
| Peroxisomal Bifunctional Enzyme Deficiency | HSD17B4 | c.303-1G>A |
| Pervasive Developmental Disorders | NLGN4X | D396X |
| Pervasive Developmental Disorders | NLGN4X | D396X |
| Pervasive Developmental Disorders | NLGN4X | NLGN4X:1253delAG |
| Pervasive Developmental Disorders | NLGN4X | NLGN4X:1253delAG |
| Pervasive Developmental Disorders | NLGN3 | R451C |
| Phenylalanine Hydroxylase Deficiency | PAH | G272X |
| Phenylalanine Hydroxylase Deficiency | PAH | I65T |
| Phenylalanine Hydroxylase Deficiency | PAH | IVS12+1G>T |
| Phenylalanine Hydroxylase Deficiency | PAH | L48S, rs5030841 |
| Phenylalanine Hydroxylase Deficiency | PAH | R158Q, rs5030843 |
| Phenylalanine Hydroxylase Deficiency | PAH | R252W, rs5030847 |
| Phenylalanine Hydroxylase Deficiency | PAH | R261Q, rs5030849 |
| Phenylalanine Hydroxylase Deficiency | PAH | R408Q, rs5030859 |
| Phenylalanine Hydroxylase Deficiency | PAH | R408W, rs5030858 |
| Phenylalanine Hydroxylase Deficiency | PAH | rs5030855 |
| Phenylalanine Hydroxylase Deficiency | PAH | rs5030861 |
| Phenylalanine Hydroxylase Deficiency | PAH | Y414C, rs5030860 |

## FIGURE 1 – Table of Exemplary Causal Genetic Variants

| Disease | Gene | Variant Name |
|---|---|---|
| Plasminogen Activator Inhibitor I | SERPINE1 | -844 G>A |
| Plasminogen Activator Inhibitor I | SERPINE1 | 4G/5G |
| Polycystic Kidney Disease, Autosomal Recessive | PKHD1 | c.10412T>G |
| Polycystic Kidney Disease, Autosomal Recessive | PKHD1 | c.107C>T |
| Polycystic Kidney Disease, Autosomal Recessive | PKHD1 | c.1486C>T |
| Polycystic Kidney Disease, Autosomal Recessive | PKHD1 | c.5895dupA |
| Polycystic Kidney Disease, Autosomal Recessive | PKHD1 | c.5895dupA |
| Polycystic Kidney Disease, Autosomal Recessive | PKHD1 | c.9689delA |
| Polycystic Kidney Disease, Autosomal Recessive | PKHD1 | c.9689delA |
| PPT1-Related Neuronal Ceroid-Lipofuscinosis | PPT1 | c.364A>T |
| PPT1-Related Neuronal Ceroid-Lipofuscinosis | PPT1 | p.L10X |
| PPT1-Related Neuronal Ceroid-Lipofuscinosis | PPT1 | p.R151X |
| PPT1-Related Neuronal Ceroid-Lipofuscinosis | PPT1 | T75P |
| PROP1-related pituitary hormome deficiency | PROP1 | 301-302delAG |
| PROP1-related pituitary hormome deficiency | PROP1 | 301-302delAG |
| Prothrombin Thrombophilia | F2 | rs1799963 |
| Prothrombin Thrombophilia | F2 | rs6025, rs6025 |
| Pseudovitamin D Deficiency Rickets | CYP27B1 | 7bp duplication in exon8 |
| Pseudovitamin D Deficiency Rickets | CYP27B1 | 7bp duplication in exon8 |
| Pseudovitamin D Deficiency Rickets | CYP27B1 | 958delG |
| Pseudovitamin D Deficiency Rickets | CYP27B1 | 958delG |
| Rett Syndrome | MECP2 | 806delG |
| Rett Syndrome | MECP2 | 806delG |
| Rett Syndrome | MECP2 | A140V, rs28934908 |
| Rett Syndrome | MECP2 | P152R |
| Rett Syndrome | MECP2 | P225R |
| Rett Syndrome | MECP2 | R106W, rs28934907 |
| Rett Syndrome | MECP2 | R133C |
| Rett Syndrome | MECP2 | R168X |
| Rett Syndrome | MECP2 | R255X |
| Rett Syndrome | MECP2 | R270X |
| Rett Syndrome | MECP2 | R294X |

## FIGURE 1 – Table of Exemplary Causal Genetic Variants

| Disease | Gene | Variant Name |
| --- | --- | --- |
| Rett Syndrome | MECP2 | R306C, rs28935468 |
| Rett Syndrome | MECP2 | S134C |
| Rett Syndrome | MECP2 | T158M, rs28934906 |
| Rhizomelic Chondrodysplasia Punctata Type 1 | PEX7 | p.A218V |
| Rhizomelic Chondrodysplasia Punctata Type 1 | PEX7 | p.G217R |
| Rhizomelic Chondrodysplasia Punctata Type 1 | PEX7 | p.L292X, rs1805137 |
| Short Chain Acyl-CoA Dehydrogenase Deficiency | ACADS | c.511C>T, rs1800556 |
| Short Chain Acyl-CoA Dehydrogenase Deficiency | ACADS | c.625G>A |
| Short Chain Acyl-CoA Dehydrogenase Deficiency | ACADS | R107C |
| Shwachman-Diamond Syndrome | SBDS | 183_184TA>CT |
| Shwachman-Diamond Syndrome | SBDS | 183_184TA>CT |
| Shwachman-Diamond Syndrome | SBDS | 258+2T>C |
| Sjogren-Larsson Syndrome | ALDH3A2 | c.943C>T |
| Smith-Lemli-Opitz Syndrome | DHCR7 | C380Y |
| Smith-Lemli-Opitz Syndrome | DHCR7 | IVS8-1G>C |
| Smith-Lemli-Opitz Syndrome | DHCR7 | L109P |
| Smith-Lemli-Opitz Syndrome | DHCR7 | L157P |
| Smith-Lemli-Opitz Syndrome | DHCR7 | R352Q |
| Smith-Lemli-Opitz Syndrome | DHCR7 | R352W |
| Smith-Lemli-Opitz Syndrome | DHCR7 | R404C |
| Smith-Lemli-Opitz Syndrome | DHCR7 | R446Q |
| Smith-Lemli-Opitz Syndrome | DHCR7 | T93M |
| Smith-Lemli-Opitz Syndrome | DHCR7 | V326L |
| Smith-Lemli-Opitz Syndrome | DHCR7 | W151X |
| Smith-Lemli-Opitz Syndrome | DHCR7 | W151X |
| Spastic Paraplegia 13 | HSPD1 | V72I |
| Sulfate Transporter-Related Osteochondrodysplasia | SLC26A2 | 340delV |
| Sulfate Transporter-Related Osteochondrodysplasia | SLC26A2 | 340delV |

## FIGURE 1 – Table of Exemplary Causal Genetic Variants

| Disease | Gene | Variant Name |
| --- | --- | --- |
| Sulfate Transporter-Related Osteochondrodysplasia | SLC26A2 | c.837C>T |
| Sulfate Transporter-Related Osteochondrodysplasia | SLC26A2 | C653S |
| Sulfate Transporter-Related Osteochondrodysplasia | SLC26A2 | IVS1+2T>C |
| Sulfate Transporter-Related Osteochondrodysplasia | SLC26A2 | R178X |
| TFR2-Related Hereditary Hemochromatosis | TFR2 | AVAQ594-597del |
| TFR2-Related Hereditary Hemochromatosis | TFR2 | AVAQ594-597del |
| TFR2-Related Hereditary Hemochromatosis | TFR2 | E60X |
| TFR2-Related Hereditary Hemochromatosis | TFR2 | E60X |
| TFR2-Related Hereditary Hemochromatosis | TFR2 | M172K |
| TFR2-Related Hereditary Hemochromatosis | TFR2 | Y250X |
| Thanatophoric Dysplasia | FGFR3 | G370C |
| Thanatophoric Dysplasia | FGFR3 | K650E |
| Thanatophoric Dysplasia | FGFR3 | R248C |
| Thanatophoric Dysplasia | FGFR3 | S249C |
| Thanatophoric Dysplasia | FGFR3 | S371C |
| Thanatophoric Dysplasia | FGFR3 | X807C A>T |
| Thanatophoric Dysplasia | FGFR3 | X807G |
| Thanatophoric Dysplasia | FGFR3 | X807L |
| Thanatophoric Dysplasia | FGFR3 | X807R |
| Thanatophoric Dysplasia | FGFR3 | X807S |
| Thanatophoric Dysplasia | FGFR3 | X807W |
| Thanatophoric Dysplasia | FGFR3 | Y373C |
| TPP1-Related Neuronal Ceroid-Lipofuscinosis | TPP1 | c.509-1G>A |
| TPP1-Related Neuronal Ceroid-Lipofuscinosis | TPP1 | c.509-1G>C |
| TPP1-Related Neuronal Ceroid-Lipofuscinosis | TPP1 | G284V |
| TPP1-Related Neuronal Ceroid-Lipofuscinosis | TPP1 | p.R208X |
| Transthyretin Amyloidosis | TTR | c.148G>A |
| Tyrosine Hydroxylase-Deficient DRD | TH | L205P |
| Tyrosine Hydroxylase-Deficient DRD | TH | R202H |
| Tyrosinemia Type I | FAH | E357X |
| Tyrosinemia Type I | FAH | IVS12+5 G>A |
| Tyrosinemia Type I | FAH | IVS7-6 T>G |
| Tyrosinemia Type I | FAH | IVS8-1G>C |
| Tyrosinemia Type I | FAH | p.W262X |
| Tyrosinemia Type I | FAH | P261L |
| Tyrosinemia Type I | FAH | Q64H |

## FIGURE 1 – Table of Exemplary Causal Genetic Variants

| Disease | Gene | Variant Name |
|---|---|---|
| Wilson Disease | ATP7B | 1340del4 |
| Wilson Disease | ATP7B | 3402delC |
| Wilson Disease | ATP7B | 3402delC |
| Wilson Disease | ATP7B | H1069Q |
| Wilson Disease | ATP7B | R778G |
| Wilson Disease | ATP7B | W779X |
| Wilson Disease | ATP7B | W779X |
| X-Linked Juvenile Retinoschisis | RS1 | E72K |
| X-Linked Juvenile Retinoschisis | RS1 | G109R |
| X-Linked Juvenile Retinoschisis | RS1 | G74V |
| Zellweger Syndrome Spectrum | PEX1 | c.2097_2098insT |
| Zellweger Syndrome Spectrum | PEX1 | c.2097_2098insT |
| Zellweger Syndrome Spectrum | PEX1 | c.2916delA |
| Zellweger Syndrome Spectrum | PEX1 | c.2916delA |
| Zellweger Syndrome Spectrum | PEX1 | p.G843D |

**FIGURE 2**

Populations that are notable with respect to having elevated risks for some Mendelian diseases:

1. Continental Level:

Europe

East Asia

Africa

America

2. Sub-continental level:

Arabia

Ashkenazi Jewish

Canada

Central

Cyprus

Denmark

Dominican Republic

England

European Ancestry

Finland

Hungary

India

Iran

Israel

Italy

**FIGURE 2**

Ivory Coast

Japan

Kenya

Korea

Kuwait

Mediterranean

Mediterranean

Micronesia

Middle East

Morocco

Navajo, western

New Zealand

Nigeria

Norway

Poland

Portugal

Russian

Saudi Arabia

Scandinavia

Slovakia

Slovenia

Southeast

Switzerland

Taiwan

FIGURE 2

Tunisia

Turkey

USA

Vanuatu

Local level:

Brittany, France

Quebec, Canada

Charlevoix-Saguenay region of Quebec

Newfoundland, Canada

Adra Pradesh, India

Jewish Iranians

Sardian, Italy

Bedouin in Kuwait

Pingelap, Caroine Islands

Ashkenazi in US

Yupik Eskimos of Alaska

## FIGURE 3

AIMs that distinguish African and European populations

rs376350, rs675924, rs676290, rs708156, rs717090, rs717091, rs717225,
rs718092, rs718387, rs719776, rs720225, rs720888, rs720891, rs720966,
rs721684, rs723632, rs723802, rs724729, rs725416, rs725438, rs725472,
rs725510, rs725667, rs725908, rs726391, rs726700, rs726777, rs726996,
rs727342, rs728606, rs728647, rs736723, rs764051, rs874816, rs951308,
rs951412, rs951431, rs951666, rs952109, rs952165, rs952902, rs953035,
rs959960, rs1002835, rs1027583, rs1057187, rs1074075, rs1074086,
rs1112730, rs1113480, rs1152537, rs1153849, rs1342008, rs1352405,
rs1365720, rs1366842, rs1367996, rs1369290, rs1377456, rs1382934,
rs1404694, rs1405467, rs1407716, rs1431948, rs1465648, rs1478785,
rs1485765, rs1487214, rs1506069, rs1516238, rs1526028, rs1541836,
rs1584385, rs1831024, rs1858465, rs1861498, rs1904464, rs1923416,
rs1928415, rs1929609, rs1945248, rs1950284, rs1979541, rs2021779,
rs2021781, rs2077681, rs2078588, rs2169462, rs2207782, rs2211771,
rs2225251, rs2263039, rs2317212, rs2341823


AIMs that distinguish African and Asian populations

rs1063, rs326798, rs376350, rs441728, rs717225, rs717531, rs718387,
rs718424, rs719213, rs719776, rs720225, rs720888, rs720891, rs720966,
rs721684, rs723632, rs723802, rs724729, rs725472, rs725667, rs725908,
rs726391, rs726700, rs726777, rs726996, rs728606, rs736723, rs874816,
rs879831, rs952109, rs952902, rs959157, rs967445, rs1073768, rs1074075,
rs1074086, rs1074182, rs1113480, rs1152537, rs1318822, rs1366842,
rs1369290, rs1371048, rs1377456, rs1382934, rs1391681, rs1404694,
rs1405467, rs1407716, rs1431948, rs1465648, rs1478785, rs1485765,
rs1487214, rs1506069, rs1526028, rs1807912, rs1820556, rs1831024,
rs1851204, rs1858465, rs1861498, rs1904464, rs1923416, rs1928415,
rs1929609, rs1934009, rs1945248, rs1961640, rs1979541, rs2017684,
rs2021779, rs2021781, rs2077681, rs2078588, rs2115467, rs2169462,
rs2341823


AIMs that distinguish African and American populations

1063, rs326798, rs376350, rs441728, rs483109, rs717090, rs717091, rs717171,
rs717225, rs717531, rs718424, rs719213, rs719776, rs720225, rs720888,
rs720891, rs720966, rs721684, rs722559, rs722869, rs723632, rs723802,
rs723822, rs724246, rs724247, rs724729, rs725416, rs725472, rs725667,
rs725908, rs726700, rs726777, rs726996, rs728606, rs764419, rs768324,
rs874816, rs879831, rs925140, rs938431, rs951554, rs951784, rs952159,

## FIGURE 3

rs953899, rs956065, rs959030, rs959157, rs963170, rs963171, rs967445,
rs998410, rs1048610, rs1073768, rs1074075, rs1074086, rs1074182,
rs1112806, rs1112828, rs1113480, rs1152537, rs1318822, rs1320892,
rs1333208, rs1344870, rs1352405, rs1366842, rs1366847, rs1369290,
rs1371048, rs1377456, rs1382934, rs1391681, rs1396799, rs1401385,
rs1401608, rs1403454, rs1404694, rs1405467, rs1407716, rs1411106,
rs1431948, rs1435090, rs1465648, rs1478785, rs1485765, rs1487214,
rs1506069, rs1526028, rs1533224, rs1584385, rs1807912, rs1820556,
rs1831024, rs1832443, rs1851204, rs1858465, rs1861498, rs1900099,
rs1904464, rs1923416, rs1928415, rs1929609, rs1934009, rs1945248,
rs1953054, rs1961273, rs1961320, rs1961640, rs1979541, rs1980888,
rs1986644, rs2006996, rs2017684, rs2060319, rs2077681, rs2078588,
rs2115467, rs2137636, rs2169462, rs2203099, rs2889670

AIMs that distinguish European and Asian populations

rs1063, rs242687, rs326798, rs441728, rs483109, rs708156, rs708726,
rs717090, rs717091, rs717373, rs717531, rs718092, rs718686, rs719213,
rs720496, rs722559, rs722869, rs723822, rs724246, rs724247, rs724496,
rs724806, rs725264, rs725438, rs727342, rs728647, rs754798, rs764051,
rs879831, rs925140, rs938431, rs951308, rs951378, rs951412, rs951431,
rs951554, rs951666, rs952165, rs953035, rs953899, rs959030, rs959157,
rs959960, rs963170, rs963171, rs967445, rs1002835, rs1037796, rs1048610,
rs1074182, rs1112730, rs1112806, rs1153849, rs1318822, rs1320892,
rs1327805, rs1333208, rs1350462, rs1364184, rs1365720, rs1371048,
rs1391681, rs1396799, rs1401385, rs1401608, rs1403454, rs1411106,
rs1435090, rs1461227, rs1516238, rs1541836, rs1584385, rs1807912,
rs1820556, rs1851204, rs1934009, rs1938684, rs1939546, rs1944872,
rs1950284, rs1953054, rs1961273, rs1961640, rs1980888, rs2017684,
rs2021215, rs2115467, rs2137636, rs2203099, rs2207782, rs2225251,
rs2263039, rs2312211, rs2317212, rs2366882, rs2889670

AIMs that distinguish European and American populations

1063, rs242687, rs441728, rs483109, rs675924, rs676290, rs708156, rs708726,
rs717171, rs717373, rs717531, rs718092, rs718686, rs719213, rs720050,
rs720051, rs720496, rs722559, rs722869, rs723822, rs724246, rs724247,
rs724496, rs724806, rs725264, rs727342, rs754798, rs764051, rs764419,
rs768324, rs925140, rs938431, rs951378, rs951412, rs951554, rs951666,

**FIGURE 3**

rs951784, rs952159, rs952165, rs953035, rs953899, rs956065, rs957862,
rs958790, rs959030, rs959157, rs959960, rs963170, rs963171, rs998410,
rs1002835, rs1027582, rs1027583, rs1037796, rs1048610, rs1112730,
rs1112806, rs1112828, rs1153849, rs1318822, rs1320892, rs1327805,
rs1333208, rs1344870, rs1350462, rs1364184, rs1365720, rs1366847,
rs1371048, rs1395475, rs1396799, rs1401385, rs1401608, rs1403454,
rs1411106, rs1435090, rs1461227, rs1533224, rs1541836, rs1820556,
rs1822488, rs1832443, rs1900099, rs1934009, rs1938684, rs1939180,
rs1939546, rs1944872, rs1950284, rs1953054, rs1961273, rs1961320,
rs1980888, rs1986644, rs2006996, rs2021215, rs2060319, rs2086214,
rs2115467, rs2137636, rs2203099, rs2207782, rs2211771, rs2225251,
rs2312211, rs2317212, rs2366882, rs2889670

AIMs that distinguish Asian and American populations

1900099, rs768324, rs1344870, rs717171, rs958790, rs1939180, rs956065,
rs1366847, rs1822488, rs1980888, rs951784, rs720051, rs720050, rs2203099,
rs1533224, rs1403454, rs720496, rs1037796, rs1832443, rs1961320,
rs764419, rs2317212, rs2060319, rs1401385, rs959030, rs1435090,
rs1112828, rs1320892, rs1027582, rs0, rs2086214, rs1027583, rs1411106,
rs957862, rs242687, rs998410, rs723822, rs725264, rs708726, rs1395475,
rs2366882, rs0, rs2006996, rs1327805, rs1939546, rs1986644, rs963170,
rs952159, rs1350462, rs952109, rs963171, rs676290, rs959157, rs675924,
rs2207782, rs2211771, rs0, rs2021215, rs1342008, rs717373, rs722559,
rs725510, rs708156, rs483109, rs953899, rs754798, rs719213, rs1401608,
rs2137636, rs718092, rs725908, rs2889670, rs1112730, rs952165, rs1112806,
rs951666, rs1953054, rs1333208, rs0, rs1365720, rs2341823, rs1938684,
rs1961273, rs717531, rs967445, rs1073768, rs2021779, rs441728, rs726391,
rs1944872, rs2021781, rs727342, rs2312211, rs736723, rs1153849, rs951554,
rs1057187, rs2225251, rs1934009

AIMs that distinguish Northern Europeans from Southern Europeans:

rs1129038, rs3769005, rs2596501, rs1364394, rs969539, rs974020,
rs1922286, rs2171209, rs1560569, rs17443616, rs2596834, rs1890131,
rs2418844, rs1157492, rs1922086, rs6432110, rs8041327, rs1416467,
rs33706, rs379773, rs10496610, rs725974, rs822759, rs1517407, rs2187684,
rs7997100, rs959763, rs1003306, rs1854226, rs2367191, rs202546,
rs2905347, rs2236876, rs10508372, rs959260, rs920590, rs986642,
rs2219248, rs923031, rs495347, rs103294, rs2003092, rs1373557,

## FIGURE 3

rs16891982, rs1073321, rs2014303, rs1873195, rs1408794, rs9290675,
rs7108371, rs1045873, rs523776, rs7163907, rs10853962, rs1777689,
rs1032143, rs10512122, rs7908825, rs4859259, rs10516982, rs7965049,
rs153595, rs7277342, rs10486207, rs3809125, rs2847502, rs10509384,
rs2097884, rs4639533, rs10255965, rs1976033, rs1107820, rs10483853,
rs4686497, rs3822616, rs4832640, rs2086085, rs2685159, rs12502036,
rs9861816, rs2251432, rs8040452, rs7552548, rs3007711, rs1879558,
rs1476162, rs17864053, rs477627, rs2047058, rs1065674, rs9860730,
rs1448314, rs554788, rs1582398, rs1660964, rs10509954, rs17457687,
rs4307284, rs11586379, rs12124147, rs7548659, rs760607, rs1981135,
rs749663, rs1334804, rs10889750, rs1288367, rs213496, rs11207865,
rs4915691, rs1566246, rs3101336, rs544858, rs10493649, rs12758138,
rs6660484, rs2968485, rs712886, rs12061503, rs12046602, rs731756,
rs10776798, rs6587597, rs912572, rs1419074, rs488150, rs1322780,
rs859362, rs6680701, rs2146060, rs2784101, rs2494302, rs10494878,
rs1930903, rs2275302, rs4916113, rs587913, rs6723966, rs10929646,
rs4260216, rs340747, rs1489688, rs4665797, rs1438131, rs13032262,
rs10205008, rs605832, rs7594173, rs6544718, rs6713506, rs7577894,
rs2075375, rs6720799, rs41420, rs13032535, rs11164050, rs11123861,
rs6739285, rs1955394, rs16829231, rs6730157, rs2117742, rs1037266,
rs6752189, rs1227131, rs6749847, rs7558428, rs9288172, rs3769823,
rs10189499, rs1955117, rs10189760, rs6436553, rs6436883, rs10510268,
rs3804989, rs17043611, rs342042, rs2128162, rs3916092, rs11914832,
rs744751, rs285327, rs11544593, rs6773085, rs11130841, rs6795735,
rs2054956, rs9310279, rs6551458, rs7630043, rs9289584, rs4535234,
rs7634702, rs4678297, rs9839394, rs10513729, rs7636818, rs12635682,
rs4859259, rs6775595, rs6449375, rs13132286, rs6831024, rs6448770,
rs9884706, rs10008492, rs12186184, rs1389037, rs6832891, rs1109501,
rs4261956, rs3923243, rs1455313, rs6820697, rs1343921, rs13349989,
rs7669241, rs2850971, rs2194860, rs1448817, rs1557815, rs4574434,
rs1541745, rs4128688, rs692157, rs1390009, rs10519410, rs1507500,
rs10517660, rs1492468, rs1430975, rs11730565, rs1435442, rs4241802,
rs10032784, rs2736122, rs7718757, rs1533019, rs29460, rs464923,
rs1001935, rs2279095, rs1373967, rs173686, rs6452788, rs174015,
rs10478046, rs6863510, rs6866231, rs346650, rs1528961, rs171718,
rs4868204, rs2135053, rs7727897, rs248327, rs1933652, rs2326106,
rs4960257, rs2876167, rs1992387, rs2237148, rs7763768, rs6918101,
rs12660883, rs382259, rs2076173, rs804847, rs12524885, rs4610536,
rs638473, rs2451688, rs9389124, rs7753036, rs4896663, rs17551120,
rs11756366, rs10428822, rs10950641, rs2041362, rs2189947, rs10267453,
rs697518, rs2529015, rs10951140, rs1003549, rs933360, rs17135491,
rs4020771, rs7791143, rs4730287, rs7781715, rs12705973, rs12706793,
rs1593306, rs11761774, rs4571660, rs7795021, rs7806048, rs1548353,
rs4565458, rs12548107, rs13273386, rs1127379, rs10109984, rs7830163,

## FIGURE 3

rs6993747, rs7827918, rs2380646, rs4634634, rs11780821, rs11784678,
rs1026804, rs1841316, rs4871195, rs4736413, rs396861, rs10758823,
rs566820, rs10962589, rs2840790, rs10511715, rs10812520, rs867469,
rs2130118, rs10746763, rs10908907, rs884886, rs10739277, rs7860625,
rs1009473, rs7090242, rs7921493, rs11255712, rs1324322, rs4948508,
rs3858126, rs4746826, rs7072160, rs4691, rs499437, rs1857459, rs7097946,
rs11186543, rs701873, rs1023331, rs7071247, rs10509826, rs1325172,
rs540609, rs11146457, rs2045272, rs7931276, rs1477569, rs4755844,
rs2218868, rs2237997, rs12804561, rs2282504, rs7342241, rs2015747,
rs488753, rs7131355, rs4936969, rs740851, rs7976721, rs10770437,
rs699039, rs12370505, rs2088170, rs2860493, rs2723891, rs11107018,
rs7307510, rs12231308, rs7312155, rs991817, rs11066320, rs1955105,
rs2650170, rs1405050, rs3815210, rs11059335, rs2398513, rs377318,
rs7323018, rs1979558, rs9539642, rs13379032, rs4885167, rs2175075,
rs1678386, rs9556553, rs4982420, rs7152286, rs2038281, rs10498472,
rs7492698, rs2180611, rs1531631, rs8003492, rs17126387, rs9671457,
rs4906226, rs1947745, rs11634609, rs2611605, rs1153860, rs1906433,
rs10519005, rs2292745, rs1320205, rs387727, rs8025028, rs3922394,
rs1510058, rs8043261, rs2072986, rs1891325, rs896401, rs949429, rs30237,
rs8047148, rs7205880, rs12446160, rs1872678, rs9937047, rs4238802,
rs3751834, rs6539986, rs2253820, rs7215135, rs7220080, rs685098,
rs2165846, rs11870879, rs196948, rs12939848, rs8071270, rs736632,
rs400839, rs1786153, rs11564361, rs16948113, rs4941246, rs17079195,
rs11151863, rs4799268, rs4536588, rs6510672, rs1552046, rs715159,
rs8111998, rs2082455, rs17496703, rs7976, rs7252868, rs8103620,
rs4814697, rs6076623, rs6118234, rs1041200, rs8116153, rs1884783,
rs6126462, rs6129532, rs6032343, rs477627, rs4811158, rs2041317,
rs6092326, rs3916504, rs6071491, rs977712, rs1735899, rs2832643,
rs2836824, rs11702531, rs2235338, rs848728, rs11147509, rs4131364


AIMs that distinguish Northern Europeans from Ashkenazi Jewish Europeans:

rs3769005, rs7551844, rs2596501, rs3007711, rs1129038, rs6996185,
rs847851, rs17443616, rs1922086, rs974020, rs7108371, rs1065674,
rs554788, rs1364394, rs1890131, rs1922286, rs10516982, rs477627,
rs6448770, rs13084044, rs1355170, rs6432110, rs9860730, rs12713956,
rs6028505, rs9576338, rs986642, rs4756052, rs7983897, rs10506555,
rs822759, rs6751522, rs1991718, rs10508372, rs10493430, rs33706,
rs379773, rs202546, rs920590, rs4307284, rs7163907, rs2251432,
rs10762340, rs1660964, rs10853962, rs764255, rs4859259, rs923031,
rs959260, rs8029021, rs2596834, rs2418844, rs998401, rs10496610,
rs10509384, rs2003092, rs1560569, rs8016025, rs6477998, rs1517407,
rs224378, rs17079195, rs10460810, rs6687300, rs2252815, rs1032143,

**FIGURE 3**

rs1476162, rs17457687, rs4642918, rs4832640, rs7637803, rs7997100,
rs6432398, rs6490700, rs4771165, rs359291, rs1373557, rs2171209,
rs1073321, rs4787923, rs920559, rs90213, rs10497233, rs3822616,
rs7819806, rs12430668, rs12441220, rs10486207, rs2377689, rs4336881,
rs1598452, rs605832, rs2219248, rs8078782, rs1045873, rs552976,
rs10431948, rs2793471, rs2905347, rs7316723, rs1118962, rs1032355,
rs1582398, rs6841252, rs1743789, rs1408794, rs1710807, rs914505,
rs2154328, rs1870590, rs12706769, rs1854226, rs2014303, rs881433,
rs10868791, rs10504275, rs6502048, rs1904406, rs17026435, rs8041327,
rs1400174, rs11744977, rs9290675, rs1462368, rs518457, rs1435260,
rs2063092, rs1011340, rs718387, rs9285474, rs2236876, rs2847502,
rs1777689, rs706397, rs9993173, rs2363127, rs217627, rs10512122,
rs725974, rs7699090, rs7038314, rs7301622, rs10495413, rs1381795,
rs2047058, rs969539, rs10494870, rs489381, rs2804756, rs7975512,
rs3927745, rs17149147, rs11713873, rs1994952, rs523776, rs10520475,
rs349187, rs618897, rs6021183, rs4815707, rs313478, rs3844253,
rs10509954, rs12502036, rs1531746, rs7561808, rs163018, rs2176246,
rs11248060, rs11245545, rs6864793, rs9861816, rs17310125, rs1157492,
rs103294, rs7501724, rs3764796, rs3809125, rs284509, rs11586379,
rs153595, rs2419063, rs730566, rs2187684, rs838958, rs10825992,
rs4782202, rs12500040, rs7965049, rs10484547, rs7920193, rs4686497,
rs2241332, rs7689609, rs12251462, rs10458810, rs2097884, rs7598757,
rs2287101


AIMs that distinguish Southern Europeans from Ashkenazi Jewish Europeans:

rs7551844, rs6996185, rs1129038, rs6477998, rs1710807, rs1743789,
rs6841252, rs6448770, rs11586379, rs1462368, rs8029021, rs13084044,
rs847851, rs764255, rs17079195, rs10431948, rs12706769, rs8016025,
rs3007711, rs12713956, rs7983897, rs2793471, rs8060233, rs12441220,
rs1355170, rs4756052, rs554788, rs881433, rs6687300, rs1065674,
rs10493430, rs1011340, rs10825992, rs8078782, rs40459, rs920559,
rs605832, rs9285474, rs4815707, rs838958, rs10460810, rs1976033,
rs10506555, rs9576338, rs6432398, rs914505, rs969539, rs477627,
rs1531746, rs6028505, rs224378, rs6578195, rs6502048, rs4642918,
rs6751522, rs1991718, rs4797909, rs11744977, rs1870590, rs2685159,
rs2154328, rs7598757, rs9860730, rs163018, rs6678209, rs7108371,
rs1994952, rs8040452, rs10516982, rs262216, rs618897, rs284509,
rs6490700, rs17026435, rs11713873, rs6907950, rs11259206, rs10762340,
rs9993173, rs17149147, rs4639533, rs1381795, rs7316723, rs359291,
rs2446653, rs273747, rs2063092, rs10255965, rs4976606, rs7155872,
rs730566, rs4307284, rs2377689, rs7689609, rs2411128, rs10517807,
rs1435260, rs3927745, rs10458810, rs17310125, rs1660964, rs7301622,

## FIGURE 3

rs706397, rs1416467, rs998401, rs313478, rs11854557, rs4131048, rs13008,
rs2241332, rs4243408, rs951412, rs7920193, rs3844253, rs718387, rs777710,
rs4771165, rs132549, rs1389560, rs3856744, rs6460989, rs2252815,
rs2251432, rs349187, rs2363127, rs10504275, rs7372209, rs495347,
rs4770401, rs1410418, rs1118962, rs7975512, rs7038314, rs1904406,
rs489381, rs7277342, rs7699090, rs10497233, rs10494870, rs9323490,
rs1157492, rs1032355, rs552976, rs10495413, rs12430668, rs440431,
rs6063178, rs12645879, rs1598452, rs2285369, rs7679675, rs7561808,
rs7900067, rs1024487, rs7637803, rs217627, rs4787923, rs17457687,
rs1400174, rs17439723, rs1435090, rs7819806, rs90213, rs2547116,
rs3764796, rs9435, rs986642, rs2589654, rs16922018, rs1352411, rs1984282,
rs2287101, rs4859259, rs1107820, rs1476162, rs959763, rs6511703,
rs12500040, rs6021183, rs10868791, rs7501724, rs7163907, rs10853962,
rs4336881, rs959567, rs6864793, rs10520475, rs11245545, rs17799799,
rs518457, rs10509384, rs2366687, rs1003306, rs2367191, rs10504924,
rs10507632, rs2728945, rs1905135, rs10516096


AIMs that distinguish Irish from English (and distinguish other Northern
European populations from one another):

rs354690, rs1200826, rs6133219, rs4937688, rs17595617, rs592229,
rs7632151, rs10158939, rs6673923, rs11761305, rs2824976, rs13143572,
rs11735755, rs1491610, rs1697143, rs12677633, rs2331295, rs12028395,
rs1035218, rs11812285, rs17487804, rs2831507, rs11119274, rs2866904,
rs3211663, rs2180046, rs1791648, rs1874777, rs11169282, rs10735745,
rs11629910, rs7637171, rs1975944, rs17751406, rs3133719, rs7802273,
rs4682664, rs1424341, rs17495345, rs2101869, rs622917, rs11234095,
rs11579554, rs2574824, rs985246, rs7172022, rs17043135, rs3934253,
rs6796183, rs9400660, rs6542847, rs9537662, rs4391081, rs921656,
rs12621435, rs608082, rs969517, rs2279578, rs4741859, rs697387,
rs9939450, rs300386, rs201930, rs6569343, rs6978997, rs2742059,
rs2616982, rs7834280, rs17057633, rs4521229, rs3132069, rs13322103,
rs4568821, rs2241466, rs1546963, rs1039621, rs11152343, rs7615026,
rs1124602, rs7998134, rs1168553, rs8027997, rs7585767, rs7015718,
rs4854502, rs1916977, rs11759031, rs12282752, rs924247, rs3115769,
rs2489772, rs2066284, rs12589358, rs2284759, rs692612, rs1420109,
rs17166207, rs6855114, rs2292702, rs150881, rs213759, rs7937428,
rs150139, rs2029121, rs3805331, rs9366778, rs2389197, rs4242426,
rs2154254, rs1345941, rs2120991, rs384267, rs10982246, rs283310,
rs2832146, rs3936367, rs906671, rs11163055, rs725926, rs9353982,
rs2251530, rs13032261, rs9981318, rs4561129, rs12193402, rs4854647,
rs10045343, rs3937773, rs10016411, rs12618502, rs4857192, rs11706497,
rs1361168, rs603089, rs7521746, rs3096700, rs1859218, rs6727787,

## FIGURE 3

rs3798315, rs1812458, rs12327639, rs10496354, rs6593652, rs12202888,
rs12427378, rs126280, rs1496292, rs13281284, rs10197026, rs3764622,
rs632853, rs1334811, rs11258614, rs1432770, rs762324, rs10506479,
rs17583414, rs4889126, rs11635677, rs7538393, rs4607068, rs2148379,
rs1544908, rs1604528, rs10148024, rs5761163, rs1495085, rs6727258,
rs8718, rs1409590, rs13071295, rs12132550, rs4693421, rs1861532,
rs10896958, rs6738275, rs9348266, rs1925391, rs42985, rs516925,
rs11162802, rs4771561, rs12706465, rs13215804, rs4818059, rs201739,
rs13059876, rs17745881, rs3823518, rs4903820, rs2214020, rs6992907,
rs9556820, rs10249706, rs3798236, rs7701328, rs12229047, rs10846585,
rs936110, rs11851852, rs9300943, rs1926150, rs7872577, rs4868253,
rs6586395, rs4832680, rs1979689, rs17325754, rs2803353, rs2109302,
rs2840131, rs12645288, rs4241627, rs12065099, rs10511771, rs17264096,
rs3026886, rs569908, rs6749268, rs7591709, rs1450878, rs8015618,
rs17068823, rs4699356, rs6926578, rs10095717, rs11841049, rs17735123,
rs1918098, rs17158154, rs1928027, rs7685881, rs4130719, rs6742202,
rs1294875, rs12319392, rs9788522, rs6061663, rs12407446, rs6705555,
rs9551221, rs4688639, rs494791, rs11748140, rs10885355, rs4395073,
rs6824429, rs11204020, rs2644640, rs6455468, rs10163400, rs2005104,
rs907942, rs1007042, rs6452431, rs2901100, rs6799064, rs16880382,
rs1805313, rs12199314, rs1243974, rs10171287, rs352418, rs10953428,
rs4974389, rs7254735, rs7257916, rs639813, rs6431565, rs1435887,
rs10761660, rs10118378, rs12533837, rs2503848, rs12363937, rs6752254,
rs12906896, rs17537169, rs2268933, rs6939639, rs3775479, rs1691018,
rs17463958, rs6741326, rs7019271, rs4509106, rs4743564, rs13395560,
rs4751890, rs8058014, rs7946208, rs2023952, rs6066892, rs153645,
rs4443343, rs9297395, rs10895115, rs10419661, rs1833208, rs129074,
rs1889085, rs12127377, rs32441, rs10886671, rs1363926, rs13221445,
rs10501445, rs11811998, rs13225753, rs8034124, rs7906816, rs11189831,
rs11147671, rs2809823, rs6538980, rs500906, rs13182055, rs7143468,
rs4636972, rs10519510, rs7521399, rs278729, rs1468329, rs10515441,
rs566821, rs1882396, rs11906231, rs7909464, rs12030971, rs10047997,
rs17866606, rs170644, rs4129526, rs1869092, rs2149973, rs4660438,
rs12404676, rs16928653, rs11138141, rs11756439, rs3925053, rs10843894,
rs4259484, rs11772445, rs1598859, rs6476962, rs6676680, rs2836604,
rs4843426, rs12626954, rs4621050, rs1794275, rs1801212, rs10889272,
rs3847580, rs808868, rs10741808, rs2622892, rs17497293, rs7171366,
rs7685182, rs12280220, rs4944000, rs6030839, rs1437683, rs11000000,
rs1122821, rs6767356, rs10795942, rs10917268, rs605383, rs957881,
rs10780596, rs7720751, rs1928091, rs3760440, rs12129709, rs1259866,
rs17600119, rs17141252, rs10954006, rs137296, rs9623117, rs10517796,
rs1755833, rs17680913, rs4321843, rs10209427, rs7123425, rs17590228,
rs16957064, rs1941603, rs4403789, rs827628, rs6782694, rs2335629,
rs1405134, rs2651458, rs6478230, rs7152796, rs531539, rs1874494,

## FIGURE 3

rs4843507, rs17273267, rs7320534, rs10868262, rs2113574, rs4814983,
rs7587228, rs17624713, rs17508376, rs1997458, rs2176313, rs251867,
rs1413710, rs7257183, rs1554808, rs1572228, rs7006668, rs338926,
rs7135506, rs6427832, rs6766822, rs7591141, rs4781927, rs13106298,
rs12874003, rs13071953, rs1910863, rs10433559, rs6092900, rs10196343,
rs6448638, rs2110420, rs2871647, rs9873664, rs12634613, rs9465438,
rs11767724, rs16999738, rs12488465, rs6992802, rs4902059, rs4308943,
rs11183847, rs2887631, rs4278155, rs10486014, rs10795076, rs7204868,
rs945704, rs3742673, rs10468473, rs897646, rs11916893, rs4897646,
rs1523902, rs972124, rs10510254, rs2718324, rs4740358, rs12440104,
rs6582999, rs4257073, rs2127247, rs17006704, rs11165281, rs11642715,
rs4968938, rs10235162, rs2218935, rs9353512, rs13096852, rs8037001,
rs992499, rs7305812, rs12643434, rs7094594, rs9330294, rs7798936,
rs1008645, rs2272786, rs11757369, rs4886026, rs243099, rs1131620,
rs10279772, rs7619189, rs10792322, rs11539202, rs2145449, rs11119145,
rs12570141, rs3851384, rs527507, rs11590681, rs3804639, rs7584576,
rs201328, rs201430, rs10097270, rs133074, rs1964196, rs2135091, rs16885,
rs4394088, rs16943989, rs11055697, rs9646474, rs16854813, rs9290227,
rs3928425, rs6949364, rs7586333, rs12091966, rs6831436, rs10083961,
rs10932040, rs6798997, rs861079, rs12867481, rs6881628, rs753281,
rs10496342, rs3117582, rs1463242, rs2408239, rs7744432, rs564968,
rs7870926, rs609305, rs4887443, rs1931902, rs11692435, rs2048161,
rs4684146, rs4628026, rs1930146, rs11735972, rs1866997, rs3745509,
rs12412656, rs295117, rs11686077, rs10282703, rs10869031, rs2047171,
rs2616612, rs16874127, rs12249377, rs845787, rs4072374, rs2552353,
rs1377554, rs400688, rs1507599, rs4728490, rs10484867, rs845561,
rs4334271, rs385773, rs4480424, rs2304572, rs11117791, rs10964808,
rs9813516, rs2034781, rs11607746, rs626364, rs6536415, rs6090443,
rs2328492, rs1379411, rs2583692, rs9938659, rs9315681, rs11622517,
rs13359372, rs10143058, rs7590571, rs12064002, rs10835941, rs17134141,
rs1343227, rs10077241, rs10132, rs2761291, rs6432594, rs10499748,
rs6746287, rs7709399, rs12669805, rs11793897, rs17762729, rs12596290,
rs11153162, rs6940057, rs1316883, rs7186783, rs6982890, rs937816,
rs7725438, rs10504180, rs8094458, rs241529, rs980274, rs855395,
rs10948327, rs12149783, rs12275853, rs2155854, rs4816612, rs2669331,
rs497321, rs669336, rs1333592, rs2270834, rs6076248, rs9692165,
rs4776567, rs4652192, rs6942609, rs6565261, rs1339287, rs631661,
rs12345503, rs10491367, rs1348338, rs2876507, rs17069574, rs6416581,
rs1370031, rs10174187, rs10783028, rs7904364, rs16932027, rs12993717,
rs12568609, rs550010, rs4896634, rs6574859, rs1558160, rs1772579,
rs1878424, rs288057, rs12647859, rs130539, rs7934426, rs7278046,
rs1886690, rs1950221, rs17747739, rs13034680, rs3790116, rs6031215,
rs10016081, rs966149, rs793096, rs8044334, rs16987794, rs12156286,
rs2803183, rs7871735, rs1187763, rs858550, rs2301570, rs1010656,

## FIGURE 3

rs1815619, rs2184925, rs10521057, rs11184219, rs2726675, rs10485678,
rs7782814, rs489466, rs1093309, rs12439063, rs2396081, rs4887111,
rs6979324, rs4233629, rs2189864, rs1267472, rs7006436, rs2839475,
rs6874663, rs952579, rs10794696, rs11634215, rs1256992, rs6747820,
rs8024685, rs1477798, rs8109759, rs6076954, rs17034929, rs4915886,
rs2560301, rs6700849, rs1368406, rs2724031, rs2292719, rs1553664,
rs3935801, rs4148298, rs4698844, rs4459724, rs1423089, rs12021671,
rs16839940, rs8021670, rs4948235, rs13200910, rs308793, rs10924440,
rs7329258, rs4780267, rs282152, rs10824914, rs16907003, rs7153598,
rs3010888, rs6957465, rs12441839, rs10192315, rs727637, rs11786174,
rs1488214, rs11635749, rs2729910, rs4869578, rs9459128, rs212434,
rs1863471, rs12372169, rs472465, rs2908201, rs6010652, rs807704,
rs6695712, rs1282557, rs10496954, rs2295682, rs9820762, rs12198061,
rs11698898, rs2139747, rs700278, rs2192883, rs6312, rs1486731, rs2684796,
rs12795982, rs2839589, rs2184221, rs9640302, rs4746826, rs632547,
rs7764278, rs6651164, rs16750, rs13020203, rs9905820, rs1951797,
rs10969030, rs10002315, rs4920104, rs7257017, rs9514044, rs2872338,
rs3750111, rs1512155, rs4785173, rs2151842, rs2218724, rs1822917,
rs1688015, rs11221731, rs887106, rs7809872, rs4532570, rs12554199,
rs2773207, rs12314080, rs11881242, rs7225060, rs2518201, rs1010779,
rs8048863, rs9299261, rs4586385, rs12642133, rs10777342, rs16901689,
rs1859569, rs9300717, rs1154278, rs11174549, rs7140955, rs10108974,
rs7852159, rs9419387, rs10831905, rs10498377, rs269850, rs9454096,
rs6967514, rs13132569, rs12327342, rs11799558, rs1325156, rs2844651,
rs2556097, rs2891409, rs12538916, rs8005808, rs2456220, rs6859290,
rs924884, rs12501016, rs11644921, rs907238, rs2331175, rs2800257,
rs717966, rs10825066, rs7112734, rs1524600, rs10491030, rs1387329,
rs7996510, rs17461905, rs11839068, rs9312336, rs7204900, rs1672376,
rs12677340, rs9575619, rs2765584, rs7205649, rs2839349, rs1454149,
rs4907404, rs2166488, rs1230313, rs908822, rs2402800, rs913984,
rs17651643, rs10979066, rs3024536, rs17173637, rs567384, rs9562077,
rs10942957, rs4658702, rs7785682, rs2392147, rs613479, rs12710777,
rs2719163, rs12094260, rs483241, rs12083651, rs12279152, rs7971883,
rs17527048, rs2231895, rs512458, rs7321658, rs9923061, rs2298608,
rs865151, rs11754258, rs270274, rs17567580, rs8021112, rs6506515,
rs1553255, rs2492501, rs3781578, rs10780514, rs11695049, rs2022725,
rs6959225, rs12796185, rs1369256, rs10063082, rs11966566, rs4427879,
rs2395653, rs12018676, rs10154899, rs135551, rs9538094, rs10518452,
rs17482565, rs2761384, rs10496557, rs7707491, rs943068, rs4713949,
rs4233167, rs767249, rs12191488, rs6855792, rs1541335, rs2868146,
rs6564940, rs1497305, rs1465010, rs11603501, rs10230087, rs899069,
rs7864459, rs10517677, rs4708459, rs1545255, rs11634465, rs2284944,
rs894392, rs10021708, rs7748977, rs9385824, rs7116354, rs11642445,
rs3806300, rs4705295, rs2303802, rs791656, rs4387618, rs16972092,

## FIGURE 3

rs4891825, rs11712075, rs1978368, rs16858789, rs1446394, rs2060530,
rs157237, rs1482363, rs9502971, rs3757791, rs11768706, rs10888101,
rs16936082, rs6585474, rs3857176, rs9510565, rs7787246, rs10954638,
rs10968042, rs2700230, rs7652486, rs11602256, rs12492695, rs1197701,
rs4918844, rs12646895, rs12377016, rs11995144, rs227906, rs4735339,
rs7673403, rs7855536, rs760560, rs913259, rs9442235, rs11940490,
rs3742076, rs9407554, rs7321548, rs1523250, rs5751691, rs2691244,
rs6089457, rs849734, rs897111, rs9613630, rs1432053, rs2616667,
rs10159191, rs12619236, rs7660552, rs12731666, rs2841455, rs6454198,
rs1466835, rs637001, rs7594872, rs470490, rs1017104, rs10403583,
rs7526907, rs10484854, rs1355829, rs12273605, rs691127, rs10930718,
rs1543603, rs4936578, rs722113, rs3846456, rs3934711, rs11101312,
rs10068902, rs11988550, rs4331742, rs12923978, rs582962, rs12247354,
rs10923142, rs9912143, rs2468638, rs11543651, rs16997896, rs2827845,
rs10112398, rs1412471, rs1012769, rs153785, rs4368537, rs7820789,
rs3813135, rs1554690, rs4890891, rs2114088, rs2327506, rs445275,
rs6842443, rs9423942, rs11593576, rs244050, rs1007018, rs2192754,
rs945249, rs17048973, rs17088268, rs6105221, rs2798950, rs9855729,
rs4808416, rs10883479, rs6729229, rs875569, rs2475229, rs2047153,
rs10493770, rs10128640, rs7154324, rs17780310, rs1944362, rs3793504,
rs12452093, rs4283980, rs2070713, rs11118045, rs12585880, rs7598637,
rs10887047, rs915538, rs17293152, rs904269, rs2987380, rs717751,
rs6765166, rs10486461, rs7858808, rs6599127, rs11136596, rs16943315,
rs7336525, rs9366950, rs6073394, rs1908984, rs2280699, rs2238508,
rs7460771, rs7502772, rs7038373, rs1020042, rs11791419, rs11780780,
rs11111689, rs743082, rs17242960, rs10500956, rs10431150, rs1689803,
rs12743521, rs11778372, rs17271148, rs1360646, rs8066468, rs11831940,
rs2966487, rs7018906, rs11180273, rs544603, rs4786780, rs7658820,
rs13068538, rs7963499, rs4902562, rs10515998, rs4331170, rs204538,
rs666649, rs12691565, rs16876243, rs10504268, rs4397025, rs6793927,
rs2735202, rs11172147, rs11632449, rs10733358, rs2048816, rs10942889,
rs1475048, rs7698580, rs12504106, rs716953, rs12111723, rs7075768,
rs11854173, rs10520656, rs316582, rs13295839, rs824453, rs4596664,
rs9289539, rs10275488, rs4981674, rs4955713, rs2387698, rs17277113,
rs250413, rs17590785, rs10462465, rs833010, rs7814885, rs10733333,
rs6772196, rs1342744, rs10168477, rs10273, rs7221778, rs17187133,
rs17827858, rs17279118, rs373531, rs12372697, rs10357, rs2822548,
rs1387910, rs4859864, rs17319408, rs1265203, rs12904370, rs10518627,
rs10050980, rs3782814, rs17820124, rs2475357, rs1795065, rs4414922,
rs7487814, rs13194998, rs7807708, rs17660328, rs4471501, rs8077267,
rs4304313, rs2387673, rs3846677, rs17012485, rs4234474, rs11731647,
rs6712681, rs2058131, rs2270409, rs6089784, rs17698283, rs6494039,
rs2937785, rs260445, rs9641312, rs4911442, rs4688104, rs905570, rs99521,
rs12997609, rs504341, rs1748168, rs11196967, rs1791468, rs17455729,

## FIGURE 3

rs1393861, rs4623797, rs4819591, rs1009728, rs17019119, rs4943731, rs1036295, rs2793471, rs9283119, rs4248914, rs733044,


AIMs that distinguish Spanish from Caucasians:

rs118003, rs166673, rs169088, rs261056, rs280181, rs286804, rs347413, rs374672, rs382515, rs382558, rs455249, rs578363, rs600558, rs640330, rs688858, rs707077, rs717251, rs717471, rs717614, rs718996, rs719628, rs719750, rs720030, rs720229, rs721128, rs721246, rs721288, rs721361, rs721362, rs721363, rs724130, rs724658, rs726512, rs726610, rs727923, rs728506, rs741933, rs754291, rs831451, rs869538, rs894198, rs950132, rs951572, rs951784, rs952020, rs952562, rs953111, rs955770, rs955771, rs956193, rs956377, rs957862, rs958985, rs959763, rs961463, rs965892, rs965917, rs967294, rs967906, rs979050, rs979322, rs988462, rs1016120, rs1023534, rs1032143, rs1033042, rs1035701, rs1073354, rs1074149, rs1074716, rs1074805, rs1075263, rs1075906, rs1108929, rs1113082, rs1113144, rs1114000, rs1116138, rs1116853, rs1158619, rs1202645, rs1210110, rs1331819, rs1343809, rs1359247, rs1363234, rs1363235, rs1366233, rs1368387, rs1368697, rs1373904, rs1375575, rs1375989, rs1376392, rs1377470, rs1378702, rs1380415, rs1382859, rs1384118, rs1388175, rs1390476, rs1391961, rs1393736, rs1395162, rs1395604, rs1399664, rs1400347, rs1402458, rs1404759, rs1405633, rs1410592, rs1411445, rs1412171, rs1414149, rs1417503, rs1418706, rs1419702, rs1420117, rs1449050, rs1451371, rs1454027, rs1478810, rs1479285, rs1485254, rs1498458, rs1501626, rs1537371, rs1537523, rs1546561, rs1548299, rs1549944, rs1572902, rs1579029, rs1585217, rs1585946, rs1587120, rs1599710, rs1603681, rs1605727, rs1820072, rs1824683, rs1830876, rs1854673, rs1900672, rs1917152, rs1917199, rs1927014, rs1929646, rs1931621, rs1951095, rs1951207, rs1951507, rs1951519, rs1961491, rs1986508, rs1990542, rs1992539, rs1999333, rs2008163, rs2008927, rs2010392, rs2011812, rs2014303, rs2014533, rs2015963, rs2017892, rs2017903, rs2028021, rs2040847, rs2040848, rs2057127, rs2067084, rs2078426, rs2134897, rs2139246, rs2194127, rs2196302, rs2210254, rs2216629, rs2226672, rs2351002, rs2355205, rs2358295, rs2366101, rs2366977, rs2369860, rs2373584, rs2374545, rs2389774, rs2409523, rs2418400, rs2419925, rs2865116, rs2868932, rs2886937, rs2887280, rs2899875


AIMs that distinguish South, Central and East Asians:

rs32826, rs63319, rs170872, rs187145, rs187861, rs191644, rs205474, rs219562, rs235946, rs266175, rs266177, rs303450, rs305851, rs353599,

**FIGURE 3**

rs355191, rs366220, rs409914, rs411301, rs428294, rs498046, rs632159,
rs664574, rs714857, rs717205, rs717757, rs718137, rs719006, rs719866,
rs719910, rs720376, rs720578, rs720844, rs721002, rs721407, rs721684,
rs722559, rs722653, rs722711, rs723195, rs723212, rs723686, rs723698,
rs723723, rs723794, rs724246, rs724247, rs724258, rs724259, rs725389,
rs726108, rs726217, rs726472, rs726493, rs726692, rs727424, rs727708,
rs728497, rs728618, rs728647, rs730991, rs791856, rs864736, rs896019,
rs910552, rs923976, rs950198, rs950303, rs950400, rs951062, rs951199,
rs951998, rs952503, rs952785, rs952976, rs952993, rs953624, rs953680,
rs956070, rs956408, rs958478, rs958976, rs963096, rs963170, rs963171,
rs966134, rs966285, rs967990, rs978486, rs978488, rs985933, rs985934,
rs988913, rs1000313, rs1004246, rs1011873, rs1013063, rs1018616,
rs1029554, rs1035076, rs1073109, rs1073119, rs1073985, rs1114000,
rs1178328, rs1318710, rs1336672, rs1340846, rs1343981, rs1352514,
rs1356763, rs1359932, rs1362987, rs1366267, rs1371063, rs1374499,
rs1381331, rs1381972, rs1383965, rs1383970, rs1391221, rs1391961,
rs1392096, rs1404501, rs1405567, rs1406012, rs1406218, rs1407043,
rs1408830, rs1413724, rs1416615, rs1417503, rs1418102, rs1422004,
rs1422323, rs1422895, rs1427356, rs1428702, rs1434193, rs1446966,
rs1448229, rs1470248, rs1480951, rs1509421, rs1532517, rs1549690,
rs1563353, rs1565138, rs1565534, rs1570699, rs1578243, rs1583355,
rs1586149, rs1586597, rs1812624, rs1817186, rs1822811, rs1822812,
rs1822813, rs1823100, rs1826873, rs1876545, rs1892114, rs1903189,
rs1903746, rs1924257, rs1928045, rs1934008, rs1940006, rs1944086,
rs1945146, rs1950284, rs1954119, rs1985835, rs2007397, rs2008011,
rs2008686, rs2013708, rs2035799, rs2035801, rs2050137, rs2052291,
rs2065443, rs2077811, rs2101551, rs2134897, rs2138212, rs2145231,
rs2192874, rs2327790, rs2369898, rs2370672, rs2382193, rs2392002,
rs2406519, rs2421172, rs2877455


AIMs that distinguish Chinese and Japanese populations:

rs466256, rs1024052, rs6462942, rs6685064, rs244757, rs3924094,
rs11087655, rs944664, rs11087655, rs2402820, rs2280100, rs11118295,
rs721953, rs2377527, rs296364, rs10894532, rs2842186, rs6462942,
rs4820389, rs4632285, rs4838113, rs16966142, rs1500127, rs4925258,
rs11118295, rs2280100, rs766144, rs6462942, rs766144, rs2241880,
rs1159114, rs4962061, rs5924101, rs7900633, rs11087655, rs715846,
rs6817562, rs2185369, rs331332, rs4937787, rs2241880, rs12484697,
rs4669614, rs11087655, rs2377527, rs5924101, rs4838113, rs2838665,
rs12493799, rs12505641, rs3019711, rs5924101, rs1286041, rs4669614,
rs614502, rs2280100, rs4362557, rs5924766, rs6937974, rs782758,
rs10488533, rs614502, rs2240800, rs7003901, rs4362557, rs1858234,

**FIGURE 3**

rs2724863, rs1748944, rs1077834, rs1259807, rs12576800, rs2970869,
rs9308712, rs6855567, rs6457300, rs331332, rs2293072, rs4829981,
rs1077834, rs4947803, rs7016174, rs7991928, rs2271738, rs9397264,
rs4849159, rs1554656, rs1383887, rs12221798, rs1077834, rs2358521,
rs1383887, rs12025272, rs2763002, rs9397264, rs4669614, rs7660417,
rs2114716, rs4749159, rs1383887, rs542172, rs3805466, rs7016174,
rs7991928, rs9397264, rs16993741, rs7016174, rs1259807

## Population 1

### AA .25, AG .50, GG .25
### ...ACTG[A/G]GTAG...

### EM parameters
(AA,AG,GG)

$$\pi_1 = [.25, .50, .25]$$
$$\mu_1 = [[.9, .1], [.5, .3], [.1, .7]$$
$$\Sigma_1 = [I, I, I]$$

**FIGURE 4**

**Population 2**

AA .16, AG .48, GG .36

...ACTG[A/G]GTAG...

EM parameters
(AA,AG,GG)

$$\pi_2 \;=\; [.16, .48, .36]$$
$$\mu_2 \;=\; [[.9, .1], [.5, .3], [.1, .7]$$
$$\Sigma_2 \;=\; [I, I, I]$$

Compare to Pop 1

$$\pi_1 \;\neq\; \pi_2$$
$$\mu_1 \;=\; \mu_2$$
$$\Sigma_1 \;=\; \Sigma_2$$

**FIGURE 5**

**Population 3**

AA .2, AG .60, GG .2

...GCTG[A/G]GGAG...

EM parameters
(AA,AG,GG)

$$\pi_3 = [.2, .60, .2]$$

$$\mu_3 = [[.9, .1], [.5, .5], [.1, .9]]$$

$$\Sigma_3 = [I, I, \mathrm{diag}(1, 1.2)]$$

Compare to Pop 1

$$\pi_1 \neq \pi_3$$

$$\mu_1 \neq \mu_3$$

$$\Sigma_1 \neq \Sigma_3$$

**FIGURE 6**

# Population 4

## A- .50, G- .50

## ...ACTG[A|G/-]GTAG...

EM parameters
(AA,AG,GG)

$$\pi_4 \;=\; [.50, .50]$$

$$\mu_4 \;=\; [[.6, .1], [.1, .6]]$$

$$\Sigma_4 \;=\; [\mathrm{diag}(1.2, 1), \mathrm{diag}(1, 1.2)]$$

Compare to Pop 1

$$\pi_1 \;\neq\; \pi_4$$

$$\mu_1 \;\neq\; \mu_4$$

$$\Sigma_1 \;\neq\; \Sigma_4$$

**FIGURE 7**

EP 2 288 724 B1

FIGURE 8

Intensity Scatterplot
for Beta-Thalassemia Deletion Allele

C=1 (carrier)

A

C=0 (non-carrier)

Deletion channel
intensity

Insertion channel
intensity

## Resolving boundary calls with use of ancestry information

Random Variables

$C \in 0,1$     0 if non-carrier, 1 if carrier

$X \in \mathbb{R}^2$   two dimensional intensity vector

$A \in 0,1$    Italian (0), Northern Euro (1)

### Italian

$$\pi_{C=1|A=0} = P(C = 1|A = 0) \quad = .06$$
$$\pi_{C=0|A=0} = P(C = 0|A = 0) \quad = .94$$
$$\mu_{C=1|A=0} = [.5, .5]$$
$$\mu_{C=0|A=0} = [0, 0]$$
$$\Sigma_{C=1|A=0} = I$$
$$\Sigma_{C=0|A=0} = I$$

### Northern Euro

$$\pi_{C=1|A=1} = P(C = 1|A = 1) \quad = .001$$
$$\pi_{C=0|A=1} = P(C = 0|A = 1) \quad = .999$$
$$\mu_{C=1|A=1} = [.5, .5]$$
$$\mu_{C=0|A=1} = [0, 0]$$
$$\Sigma_{C=1|A=1} = I$$
$$\Sigma_{C=0|A=1} = I$$

**FIGURE 9**

EP 2 288 724 B1

Conditional probability of being a carrier (C=1) given
measured intensity (X=x) & known ancestry (A=0,Italian)

$$P(C = 1|X = x, A = 0) = \frac{P(X = x|C = 1)P(C = 1|A = 0)}{P(X = x|C = 1)P(C = 1|A = 0) + P(X = x|C = 0)P(C = 0|A = 0)}$$

Example values if X=[.25,.25], equidistant between the two cluster centers.

$$P(C = 1|X = x, A = 0) = .047$$

$$P(C = 1|X = x, A = 1) = .00077$$

$$\frac{P(C = 1|X = x, A = 0)}{P(C = 1|X = x, A = 1)} \approx 61$$

**FIGURE 10**

EP 2 288 724 B1

```
# R source code for example
library(mvtnorm)
pi1 = .06
pi2 = 1-pi1
mu1 = c(0,1)
mu2 = c(.5,.5)
sigma1 = matrix(c(1,0,0,1),2,2)
sigma2 = sigma1
x = c(.25,.25)


t1 = dmvnorm(x,mu1,sigma1) * pi1
t2 = dmvnorm(x,mu2,sigma2) * pi2
p.italian = t1 / (t1 + t2)
#[1] 0.04735656


pi1 = .001
pi2 = 1-pi1
t1 = dmvnorm(x,mu1,sigma1) * pi1
t2 = dmvnorm(x,mu2,sigma2) * pi2
p.cauc = t1 / (t1 + t2)
#[1] 0.0007789731
```

EP 2 288 724 B1

FIGURE 11

**Probabilistic population information (e.g. provided by AIMs) can be incorporated by a simple modification to this equation**

$C \in 0,1$         0 if non-carrier, 1 if carrier

$X \in \mathbb{R}^2$         two dimensional intensity vector

$Y \in \mathbb{R}^n$    n dimensional intensity vector w/ all AIM data

$A \in 0,1$         Italian (0), Northern Euro (1)

$$P(C = 1|X = x, Y = y) = \frac{\sum_{a=0,1} P(X = x|C = 1)P(C = 1|A = a)P(A = a|Y = y)}{\sum_{c=0,1} \sum_{a=0,1} P(X = x|C = c)P(C = c|A = a)P(A = a|Y = y)}$$

$P(A = a|Y = y)$    This term is the conditional probability that this region of the genome is inherited from an individual of ancestry A=a given AIM intensity signal Y=y

**FIGURE 12**

EP 2 288 724 B1

## 1) Mother
true phased genotype

ABC
_____
_____
abc

Chip genotyping
⟶

1) Removes phase
2) Introduces errors from false pos, false neg

## 2) Mother
measured genotypes

| Measured Genotype | | | Binary rep | | | Probability |
|---|---|---|---|---|---|---|
| Aa | Bb | Cc | 1 | 1 | 1 | $(1-\beta)^3$ |
| Aa | Bb | CC | 1 | 1 | 0 | $\beta(1-\beta)^2$ |
| Aa | BB | Cc | 1 | 0 | 1 | $\beta(1-\beta)^2$ |
| Aa | BB | CC | 1 | 0 | 0 | $\beta^2(1-\beta)$ |
| AA | Bb | Cc | 0 | 1 | 1 | $\beta(1-\beta)^2$ |
| AA | Bb | CC | 0 | 1 | 0 | $\beta^2(1-\beta)$ |
| AA | BB | Cc | 0 | 0 | 1 | $\beta^2(1-\beta)$ |
| AA | BB | CC | 0 | 0 | 0 | $\beta^3$ |

False Negative Rate: β  (probability of calling risk allele as healthy)

False Positive Rate: α  (probability of calling healthy allele as risk)

**FIGURE 13**

3) Mother
inferred haplotypes

Haplotype inference

→

(shown for 1st row
for clarity)

| Measured Unphased Genotype | | | Inferred Phased | AB in phase | BC in phase | Probability |
|---|---|---|---|---|---|---|
| Aa | Bb | Cc | ABC/abc | 1 | 1 | p1 |
| Aa | Bb | Cc | aBC/Abc | 0 | 1 | p2 |
| Aa | Bb | Cc | AbC/aBc | 0 | 0 | p2 |
| Aa | Bb | Cc | ABc/abC | 1 | 0 | p4 |

↑

Probability determined empirically from
population data or via haplotype
inference algorithm (e.g. Clark's algorithm)

**FIGURE 14**

EP 2 288 724 B1

Generate gametes
for inferred haplotype

→

1) shown for 1st row
2) Use genetic map dists
from Hapmap/NCBI

4a) Recombination probability table

| Inferred Phased | AB recomb | BC recomb | Gametes | Probability |
|---|---|---|---|---|
| ABC/abc | 0 | 0 | ABC, abc | $(1 - r_{AB})(1 - r_{BC})$ |
| ABC/abc | 0 | 1 | ABc, abC | $(1 - r_{AB})(r_{BC})$ |
| ABC/abc | 1 | 0 | Abc, aBC | $(r_{AB})(1 - r_{BC})$ |
| ABC/abc | 1 | 1 | AbC, aBc | $(r_{AB})(r_{BC})$ |

4b) Gamete probability table

| Gametes | Probability |
|---|---|
| ABC | $.5(1 - r_{AB})(1 - r_{BC})$ |
| ABc | $.5(1 - r_{AB})(r_{BC})$ |
| Abc | $.5(r_{AB})(1 - r_{BC})$ |
| AbC | $.5(r_{AB})(r_{BC})$ |
| abc | $.5(1 - r_{AB})(1 - r_{BC})$ |
| abC | $.5(1 - r_{AB})(r_{BC})$ |
| aBC | $.5(r_{AB})(1 - r_{BC})$ |
| aBc | $.5(r_{AB})(r_{BC})$ |

**FIGURE 15**

EP 2 288 724 B1

5) Child unions of gametes =
possible phased genotypes

4) Mother possible gametes

Sample from pools of
gametes to generate
possible children;
here we assume mother
& father both have
same gamete pools

4) Father possible gametes

| Mother/Father Gametes | ABC | ABc | Abc | AbC | abc | abC | aBC | aBc |
|---|---|---|---|---|---|---|---|---|
| ABC | | | | | | | | |
| ABc | | | | | | | | |
| Abc | | | | | | | | |
| AbC | | | | | | | | |
| abc | | | | | | | | |
| abC | | | | | | | | |
| aBC | | | | | | | | |
| aBc | | | | | | | | |

$$P_{ABC,\text{mother}} \times P_{ABc,\text{father}} = [.5(1 - r_{AB})(1 - r_{BC})] \times [.5(1 - r_{AB})r_{BC}]$$

Example
probability of child phased genotype =
multiply probability of sampling both these gametes

**FIGURE 16**

| Phenotype Interpretation | Phenotype value (conditional on geno) | Child Genotype |
|---|---|---|
| Wild type | 0.0 | AABBCC |
| Carrier | 1.0 | AABbCC |
| Carrier | 1.0 | AABBCc |
| ... | ... | ... |
| Affected | 2.0 | aaBBCC |

6b) Another geno/pheno map: lookup table on mother/father haplotypes

| Phenotype Interpretation | Phenotype value (conditional on haplos) | Mother Haplotype 1 | Father Haplotype 2 |
|---|---|---|---|
| Wild type | 0.0 | ABC | ABC |
| Carrier | 1.0 | ABC | ABc |
| Carrier | 1.0 | ABc | ABC |
| ... | ... | ... | ... |
| Affected | 2.0 | aBc | aBc |

6c) More complex geno/pheno map: conditional distribution given mother/father haplotypes
Note: In this example, each lowercase allele right-shifts the distribution towards more height)
Note also: for simplicity, we are representing the conditional distribution here as a vector of 101 floating point values.

| Phenotype value (e.g height) (0th percentile = min) | Phenotype value 1st percentile | ... | Phenotype value 99th percentile | Phenotype value 100th percentile (=max) | Mother Haplotype 1 | Father Haplotype 2 |
|---|---|---|---|---|---|---|
| 50in | 53in | ... | 77in | 79in | ABC | ABC |
| 52in | 54in | ... | 78in | 80in | ABC | ABc |
| 53in | 56in | ... | 79in | 80in | ABc | ABC |
| ... | ... | ... | ... | ... | ... | ... |
| 56in | 58in | ... | 80in | 81in | aBc | aBc |

6d) Finally, the conceptually most general geno/pheno map is a function giving conditional distribution of phenotype given child genotype "or" mother & father haplotypes for child

**FIGURE 17**

Fully Probabilistic Risk Calculation
Account for all sources of uncertainty

**Random Variables**
$Y_c$ = child phenotype
$H_c$ = child haplotypes
$G_m$ = mother gamete
$R_m$ = mother recombinations
$H_m$ = mother haplotypes
$\hat{X}_m$ = mother inferred genotype
$X_m$ = mother actual genotype
(Substitute an $f$ subscript for the father)

The full expression for the distribution over <u>estimated child phenotype</u> (e.g. disease ri given the parental genotypes is:

$$P(Y_c|X_m, X_f) = \sum_{h_c} \sum_{g_m, g_f} P(Y_c|H_c)P(H_c|G_m, G_f)P(G_m|X_m)P(G_f|X_f)$$

$P(Y_c|X_m, X_f)$ = CD of child phenotype given true parental genotypes
$P(Y_c|H_c)$ = CD of child phenotype given child haplotypes
$P(H_c|G_m, G_f)$ = CD of child haplotypes given mother/father gametes
$P(G_m|X_m)$ = CD of mother gamete given true mother genotype
$P(G_f|X_f)$ = CD of father gamete given true father genotype

The subexpression for the distribution over the mother's possible gametes given her true genotype is expanded as:

$$P(G_m|X_m) = \sum_{r_m} \sum_{h_m} \sum_{\hat{x}_m} P(G_m|R_m)P(R_m|H_m)P(H_m|\hat{X}_m)P(\hat{X}_m|X_m)$$

$P(G_m|R_m)$ = CD of gametes given recombinant
$P(R_m|H_m)$ = CD of recombinants given haplotype
$P(H_m|\hat{X}_m)$ = CD of haplotypes given estimated genotype
$P(\hat{X}_m|X_m)$ = CD of estimated genotype given true genotype

The corresponding expression for the father can be obtained by substituting f for m. **FIGURE 18**

EP 2 288 724 B1

94

FIGURE 19

**FIGURE 20**

**FIGURE 21**

Create Order

Pending

Tracking Number Created for Sample Collection Kit

Tracked

Sample Collection Kit Picked Up

**Customer**

Customer Outgoing

Sample Collection Kit Delivered

Customer Received

Sample and Remailer Sent

**Accessioning**

Accessioning Incoming

Sample and Remailer Received
Sample approved

Accessioning Approved

Batch of Samples Sent for Processing

**Lab Processing**

Lab Incoming

Batch of Samples Delivered
Lab Acknowledges Receipt
QC Algorithm Passes All Samples

Lab Complete

Phenotype Data Collected for All Samples in an Order

**Website**

Pending Report

Report Generated

**Report Review**

Lab Review

Report Reviewed by Lab Staff

Physician Review

Report Reviewed by Ordering Physician

Complete

**FIGURE 22**

EP 2 288 724 B1

FIGURE 23

**Phenotype questionnaires and family history**

(1) Background for Mama Hen

Ethnicity (*)
Northern European
British

Date of Birth (*)

Weight
(lbs)

Height
(ft)  (in)

(2) Background for Papa Hen

Ethnicity (*)
Northern European
British

Date of Birth (*)

Weight
(lbs)

Height
(ft)  (in)

(3)

**Child prediction using parental genotypes and phenotypes, and optionally relative geno/pheno**

Universal Carrier Screen Results
Your DNA test results are ready and shown below

*Mama Hen*

*Papa Hen*

*Your Potential Child*

Smith-Lemli-Opitz Syndrome   1 In 2   Average odds: 1 in 40,000

Congenital Adrenal Hyperplasia 1   1 In 2   Average odds: 1 in 15,000

| | Sons | Daughters |
|---|---|---|
| Height | 5 ft 9 in ± 2 in | 5 ft 4 in ± 2 in |
| Weight | 165 lbs ± 10 lbs | 135 lbs ± 10 lbs |

**FIGURE 24**

EP 2 288 724 B1

100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007053752 A
- US 20070037182 A, Gaskin
- US 20070099208 A, Drmanac
- US 7368265 B, Brenner
- US 7361468 B, Liu
- US 7332277 B, Dhallan
- US 6576424 B, Fodor
- US 7323305 B, Leamon
- US 7344865 B
- US 7364855 B

**Non-patent literature cited in the description**

- **FRIEDMAN, NAOMI P. ; MIYAKE, AKIRA ; YOUNG, SUSAN E. ; DEFRIES, JOHN C. ; CORLEY, ROBIN P. ; HEWITT, JOHN K.** Individual differences in executive functions are almost entirely genetic in origin. *Journal of Experimental Psychology: General.,* May 2008, vol. 137 (2), 201-225
- **PASCHOU et al.** PCA-correlated SNPs for structure identification in worldwide human populations. *PLoS Genet,* 2007, vol. 3 (9), 1672-86
- **SELDIN et al.** Application of ancestry informative markers to association studies in European Americans. *PLoS Genet,* 2008, vol. 4 (1), e5
- **HALDER et al.** A panel of ancestry informative markers for estimating individual biogeographical ancestry and admixture from four continents: utility and applications. *Hum Mutat,* 2008, vol. 29 (5), 648-58
- **TIAN et al.** Analysis and application of European genetic substructure using 300 K SNP information. *PLoS Genet,* 2008, vol. 4 (1), e4
- **PRICE et al.** Discerning the ancestry of European Americans in genetic association studies. *PLoS Genet,* 2008, vol. 4 (1), e236
- **BAUCHET et al.** Measuring European population stratification with microarray genotype data. *Am J Hum Genet,* 2007, vol. 80 (5), 948-56
- **HYMAN.** A new method of sequencing DNA. *Anal Biochem.,* 1988, vol. 174, 423-36
- **RONAGHI.** Pyrosequencing sheds light on DNA sequencing. *Genome Res.,* 2001, vol. 11, 3-11
- **ALBERT TJ et al.** Direct selection of human genomic loci by microarray hybridization. *Nature Methods,* November 2007, vol. 4 (11), 903-5
- **OKOU DT et al.** Microarray-based genomic selection for high-throughput resequencing. *Nature Methods,* November 2007, vol. 4 (11), 907-9
- **HODGES E et al.** Genome-wide in situ exon capture for selective resequencing. *Nature Genetics,* December 2007, vol. 39 (12), 1522-7